(19) **Europäisches Patentamt / European Patent Office / Office européen des brevets**

(11) **EP 4 220 152 B1**

(12) # EUROPEAN PATENT SPECIFICATION

(45) Date of publication and mention of the grant of the patent:
**28.01.2026  Bulletin 2026/05**

(21) Application number: **21906715.4**

(22) Date of filing: **17.12.2021**

(51) International Patent Classification (IPC):
**G01N 33/94** (2006.01)    **G01N 13/00** (2006.01)

(52) Cooperative Patent Classification (CPC):
**G01N 13/00; G01N 33/94;** B01D 2325/0283;
G01N 33/15; G01N 2013/003

(86) International application number:
**PCT/JP2021/046680**

(87) International publication number:
**WO 2022/131357 (23.06.2022 Gazette 2022/25)**

(54) **METHOD FOR MEASURING RELATIVE FU RATIO BY DYNAMIC ANALYSIS**

VERFAHREN ZUR MESSUNG DES RELATIVEN FU-VERHÄLTNISSES DURCH DYNAMISCHE ANALYSE

PROCÉDÉ DE MESURE DE RAPPORT RELATIF FU PAR ANALYSE DYNAMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2020  JP 2020210495**

(43) Date of publication of application:
**02.08.2023  Bulletin 2023/31**

(60) Divisional application:
**25217277.0 / 4 675 275**

(73) Proprietor: **CHUGAI SEIYAKU KABUSHIKI KAISHA**
**Tokyo 115-8543 (JP)**

(72) Inventors:
• **IGARASHI, Fumihiko**
  **Gotemba-shi, Shizuoka 412-8513 (JP)**
• **TACHIBANA, Tatsuhiko**
  **Gotemba-shi, Shizuoka 412-8513 (JP)**
• **YAMAUCHI, Tsuyoshi**
  **Gotemba-shi, Shizuoka 412-8513 (JP)**
• **KURAMOTO, Shino**
  **Kamakura-shi, Kanagawa 247-8530 (JP)**
• **MATSUNO, Fumiyo**
  **Kamakura-shi, Kanagawa 247-8530 (JP)**

(74) Representative: **Vossius & Partner**
**Patentanwälte Rechtsanwälte mbB**
**Siebertstraße 3**
**81675 München (DE)**

(56) References cited:
**JP-A- 2001 145 821    JP-A- H07 311 200**

• **ERIKSSON M A L ET AL: "Studies of drug binding to plasma proteins using a variant of equilibrium dialysis", JOURNAL OF PHARMACEUTICAL AND BIOMEDICAL ANALYSIS, ELSEVIER B.V, AMSTERDAM, NL, vol. 38, no. 3, 1 July 2005 (2005-07-01), pages 381 - 389, XP004940422, ISSN: 0731-7085, DOI: 10.1016/J.JPBA.2005.01.015**
• **SCHUHMACHER J ET AL: "Determination of the free fraction and relative free fraction of drugs strongly bound to plasma proteins", JOURNAL OF PHARMACEUTICAL SCIENCES, WILEY, UNITED STATES, vol. 89, no. 8, 1 August 2000 (2000-08-01), pages 1008 - 1021, XP002301862, ISSN: 0022-3549, DOI: 10.1002/1520-6017(200008)89:8<1008::AID-JPS5>3.0.CO;2-B**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- JONES ROBERT S, CHANG JAE H, FLORES MAURICIO, BRECHT ELLIOT: "Evaluation of a Competitive Equilibrium Dialysis Approach for Assessing the Impact of Protein Binding on Clearance Predictions", JOURNAL OF PHARMACEUTICAL SCIENCES, vol. 110, no. 1, 1 January 2021 (2021-01-01), United States, pages 536 - 542, XP009537498, ISSN: 0022-3549, DOI: 10.1016/j.xphs.2020.09.012
- DESHMUKH, S.V. ; HARSCH, A.: "Direct determination of the ratio of unbound fraction in plasma to unbound fraction in microsomal system (fu"p/fu"m"i"c) for refined prediction of phase I mediated metabolic hepatic clearance", JOURNAL OF PHARMACOLOGICAL AND TOXICOLOGICAL METHODS, vol. 63, no. 1, 1 January 2011 (2011-01-01), US
, pages 35 - 39, XP027583074, ISSN: 1056-8719

**Description**

[Technical Field]

The present invention relates to a method for measuring a relative fraction unbound ratio (relative $f_u$ ratio) of an analyte between different biological samples by equilibrium dialysis and dynamic analysis.

[Background Art]

It is considered that a drug in biological material exists in a bound form (state of being bound to plasma protein in a non-specific manner) and an unbound form (free drug state), and the bound form cannot act on a target, whereas the unbound form directly acts on the target (a free drug hypothesis). According to the free drug hypothesis, a factor having an effect on drug efficacy/onset of toxicity could be an unbound form concentration. Since the drug concentration in plasma is normally given as a total of the bound form and the unbound form concentration, a fraction unbound ($f_u$) value for calculating the unbound form concentration from the total concentration is considered to be an important parameter. In nonclinical pharmacokinetic studies (pharmacokinetic studies using mice, rats, dogs, monkeys, cell assays or the like), the $f_u$ value has been individually acquired from each sample, and used for free drug concentration information to improve the accuracy of prediction in humans (prediction of clearance/distribution volume showing the disappearance/distribution of a drug, and the correction of the in vitro metabolic intrinsic clearance) and prediction of drug-drug interaction (DDI) risk.

There is an equilibrium dialysis method as a known method for determining the fraction unbound value (absolute value of $f_u$) in plasma. Specifically, in a buffer chamber and a plasma-containing chamber which are separated by a semipermeable membrane, the fraction unbound of a compound of interest is reflected on the basis of a concentration ratio at the time of reaching an equilibrium state (Non Patent Literature 1). The equilibrium dialysis is called a gold standard method because of its speedy and easy operations and various methods have been proposed. For example, in Non Patent Literature 2, flux analysis with both chambers containing plasma of the same species is performed, and $f_u$ is estimated within the range of 0.000013 to 0.22. In Non Patent Literature 3, a method is devised in which bidirectional equilibrium dialysis evaluation is performed, and $f_u$ values obtained in the A-to-B direction and in the B-to-A direction are equivalent to each other, such a numerical value is confidently adopted. Meanwhile, an approach to directly determining a $f_u$ ratio between two species as a method based on equilibrium dialysis has been proposed (Non Patent Literature 4 and Non Patent Literature 5).

[Citation List]

[Non Patent Literature]

[Non Patent Literature 1] Eriksson, M. A., et al. (2005). "Studies of drug binding to plasma proteins using a variant of equilibrium dialysis." J Pharm Biomed Anal 38(3): 381-389.

[Non Patent Literature 2] Kalvass, J. C., et al. (2018). "Mathematical and Experimental Validation of Flux Dialysis Method: An Improved Approach to Measure Unbound Fraction for Compounds with High Protein." Drug Metabolism and Disposition, 46: Pages 458-469, April.

[Non Patent Literature 3] Chen, Y. C., et al. (2019). "Improving Confidence in the Determination of Free Fraction for Highly Bound Drugs Using Bidirectional Equilibrium Dialysis." J Pharm Sci 108(3): 1296-1302.

[Non Patent Literature 4] Deshmukh, S. V. and A. Harsch (2011). "Direct determination of the ratio of unbound fraction in plasma to unbound fraction in microsomal system (fu p/fu mic) for refined prediction of phase I mediated metabolic hepatic clearance." J Pharmacol Toxicol Methods 63(1): 35-39.

[Non Patent Literature 5] Jones, R. S., et al. (2020). "Evaluation of a competitive equilibrium dialysis approach for assessing the impact of protein binding on clearance predictions." J Pharm Sci., Available online 15 September 2020.

[Non Patent Literature 6] Tang, H. and M. Mayersohn (2005). "A novel model for prediction of human drug clearance by allometric scaling." Drug Metab Dispos 33(9): 1297-1303.

[Non Patent Literature 7] Berry, L. M., et al. (2011). "Species differences in distribution and prediction of human V(ss) from preclinical data." Drug Metab Dispos 39(11): 2103-2116.

[Non Patent Literature 8] Riccardi K, Cawley S, Yates PD, et al. Plasma protein binding of challenging compounds. J Pharm Sci. 2015;104:2627-2636.

[Non Patent Literature 9] Kalvass JC, Maurer TS. Influence of nonspecific brain and plasma binding on CNS exposure: implications for rational drug discovery. Biopharm Drug Dispos. 2002;23:327-338.

[Non Patent Literature 10] Vieira, M. L., et al. (2014). "Evaluation of various static in vitro-in vivo extrapolation models for risk assessment of the CYP3A inhibition potential of an investigational drug." Clin Pharmacol Ther 95(2): 189-198.
[Non Patent Literature 11] Hachad, H., et al. (2010). "A useful tool for drug interaction evaluation: the University of Washington Metabolism and Transport Drug Interaction Database." Hum Genomics 5(1): 61-72.
[Non Patent Literature 12] Chu, Q. S., et al. (2008). "A phase I and pharmacokinetic study of lapatinib in combination with letrozole in patients with advanced cancer." Clin Cancer Res 14(14): 4484-4490.
[Non Patent Literature 13] Koch, K. M., et al. (2017). "The effects of lapatinib on CYP3A metabolism of midazolam in patients with advanced cancer." Cancer Chemother Pharmacol 80(6): 1141-1146.
[Non Patent Literature 14] Takano, J., et al. (2016). "The Prediction of the Relative Importance of CYP3A/P-glycoprotein to the Nonlinear Intestinal Absorption of Drugs by Advanced Compartmental Absorption and Transit Model." Drug Metab Dispos 44(11): 1808-1818.
[Non Patent Literature 15] FDA DDI guidance(2020)
[Non Patent Literature 16] Yang, J., et al. (2007). "Prediction of intestinal first-pass drug metabolism." Curr Drug Metab 8(7): 676-684.
[Non Patent Literature 17] Yang, J., et al. (2007). "Misuse of the well-stirred model of hepatic drug clearance." Drug Metab Dispos 35(3): 501-502.
[Non Patent Literature 18] Obach, R. S., et al. (2006). "The utility of in vitro cytochrome P450 inhibition data in the prediction of drug-drug interactions." J Pharmacol Exp Ther 316(1): 336-348.

[Summary of Invention]

[Technical Problem]

If a compound having a low $f_u$ value (a compound having a high plasma protein binding ratio, or a compound having a high protein binding ratio) is treated in these known methods, an increase in time until reaching an equilibrium state and an effect of adsorption to a buffer chamber wall surface may be the problem, and make it difficult to obtain an accurate numerical value. Thus, compounds suitable for use in the method for determining a $f_u$ value using equilibrium dialysis may be limited. In addition, low $f_u$ values are susceptible to error (e.g. numerical values of $f_u = 0.00001$ and $f_u = 0.00002$), and it is considered that a process in which these values are individually obtained and used for comparison among samples of multiple species (comparison among mouse, rat, dog, monkey and human) causes an increase in error, and is disadvantageous in analysis accuracy. In the method of Non Patent Literature 2, an average value of semipermeable membrane permeation rates (Pmem [m/s]) determined from low-molecular-weight compounds in the process of calculating a $f_u$ value is used as a common value in determination of the $f_u$ value of each compound. However, since Pmem may be a coefficient negatively correlated with a molecular weight according to the Stokes-Einstein theory, the error in the method of Non Patent Literature 2 may increase in the case of a compound having a large molecular weight and overestimated Pmem like cyclosporine (molecular weight: 1202.61). In addition, in the case of a compound having a large molecular weight, the time until reaching an equilibrium state increases, so that it may be difficult to obtain an accurate $f_u$ value from an experiment when the compound has a low $f_u$ value. In the method of Non Patent Literature 3, a buffer chamber is still used, and it is not possible to deal with a compound which is strongly adsorbed to a device like cyclosporine. In Non Patent Literatures 4 and 5, a concentration after an equilibrium state is reached is also used for determining a $f_u$ ratio. Thus, it is considered that it is difficult to detect of the $f_u$ ratio of a compound which has not reached an equilibrium state, and the methods of Non Patent Literatures 4 and 5 are not suitable for a compound having a low $f_u$ value (i.e. a compound having a high protein binding ratio).
In view of the above-described problems, an object of the present invention is to provide a method for acquiring a high-accuracy $f_u$ value ratio in a short time for a compound having a high protein binding ratio.

[Solution to Problem]

The present inventors have extensively conducted studies for solving the above-described problems, and resultantly found that by combining equilibrium dialysis with dynamic analysis, a relative fraction unbound ratio (relative $f_u$ ratio) between different biological samples for a compound can be acquired with high accuracy and in a short time even when the compound has a high protein binding ratio. From the data of clearance or distribution volume of a drug in a species from which one biological sample is derived, the data of clearance or distribution volume of a drug in a species derived from another biological sample can be corrected, and human pharmacokinetics can be accurately predicted, by using the relative $f_u$ ratio.
That is, the present invention relates to the following.

[1] A method for determining a relative fraction unbound ratio (relative $f_u$ ratio) of an analyte between different biological samples, the method comprising the following steps of:

(1) providing a chamber system (I) in which adjacent chambers are separated by a semipermeable membrane permeable to the analyte;

(2) adding a donor solution containing a first biological sample (A) and the analyte to one chamber (donor-side chamber) in the chamber system (I);

(3) adding an acceptor solution containing a second biological sample (B) to a chamber different from the donor-side chamber (acceptor-side chamber) in the chamber system (I);

(4) measuring concentrations of the analyte in the donor solution in the step (2) and the acceptor solution in the step (3) over time; and

(5) calculating the relative $f_u$ ratio using data associated with the concentrations of the analyte which are measured in the step (4), wherein the step (5) is a dynamic analysis.

[2] The method according to [1], further comprising the following steps of:

(6) providing a chamber system (II) different from the chamber system (I), in which adjacent chambers are separated by a semipermeable membrane permeable to the analyte;

(7) adding an acceptor solution containing the biological sample (A) to one chamber (acceptor-side chamber) in the chamber system (II);

(8) adding a donor solution containing the biological sample (B) and the analyte to a chamber different from the acceptor-side chamber (donor-side chamber) in the chamber system (II);

(9) measuring concentrations of the analyte in the acceptor solution in the step (7) and the donor solution in the step (8) over time; and

(10) calculating the relative $f_u$ ratio using data associated with the concentrations of the analyte which are measured in the step (9), wherein the step (10) is a dynamic analysis.

[3] The method according to [1] or [2], wherein the biological samples (A) and/or (B) are diluted with a buffer solution.
[4] The method according to any one of [1] to [3], wherein a molecular weight cutoff of the semipermeable membrane is 50 kDa or less.
[5] The method according to any one of [1] to [4], wherein the steps (5) and (10) are dynamic analysis.
[6] The method according to [5], wherein the dynamic analysis is performed using the following expressions 1 to 3:

(Expression 1)

$$V1\frac{dC1}{dt} = fu2 * C2 * PS - fu1 * C1 * PS - fu1 * C1 * CLad + koff * Xad$$

(Expression 2)

$$V2\frac{dC2}{dt} = -fu2 * C2 * PS + fu1 * C1 * PS$$

(Expression 3)

$$\frac{dXad}{dt} = fu1 * C1 * CLad - koff * Xad$$

wherein

expression 1 represents a change in amount per unit time of the analyte in the donor solution, expression 2 represents a change in amount per unit time of the analyte in the acceptor solution, and expression 3 represents a change in amount per unit time of the analyte of an adsorbed fraction in the donor-side chamber; and

V1 represents a volume of the donor solution, V2 represents a volume of the acceptor solution, fu1 represents a $f_u$ value of the analyte with respect to the biological sample contained in the donor solution, fu2 represents a $f_u$ value of the analyte with respect to the biological sample contained in the acceptor solution, C1 is a concentration of the analyte in the donor solution (total concentration: total of bound form and unbound form analytes, except for the analyte adsorbed to an equilibrium dialysis device), C2 represents a concentration of the analyte in the acceptor solution (total concentration: total of bound form and unbound form analytes), PS represents dialysis membrane permeation clearance of the unbound form analyte, CLad represents adsorption clearance of the analyte in the donor-side chamber, koff represents a dissociation rate constant of the analyte of adsorbed fraction in the donor-side chamber, and Xad represents an amount of adsorption of the analyte in the donor-side chamber.

[7] The method according to any one of [1] to [6], wherein the measurement over time is performed within a period of time during denaturalization of the biological sample and/or the analyte.

[8] The method according to any one of [1] to [7], wherein the measurement over time is performed within 24 hours.

[9] The method according to any one of [1] to [8], wherein the chamber system (I) and/or the chamber system (II) comprise two chambers.

[10] The method according to any one of [1] to [9], wherein a protein binding ratio of the analyte in the biological sample (A) is 95% or more.

[11] The method according to any one of [1] to [10], wherein a protein binding ratio of the analyte in the biological sample (B) is 95% or more.

[12] The method according to any one of [1] to [11], wherein the biological sample (A) is one selected from the group consisting of a microsomal fraction, blood, plasma and serum.

[13] The method according to any one of [1] to [12], wherein the biological sample (B) is one selected from the group consisting of a microsomal fraction, blood, plasma and serum.

[14] The method according to any one of [1] to [13], wherein the biological sample is serum.

[15] The method according to any one of [1] to [14], wherein the biological sample (A) is derived from a mammal.

[16] The method according to any one of [1] to [15], wherein the biological sample (B) is derived from a mammal.

[17] The method according to any one of [1] to [16], wherein the biological sample (B) is derived from an animal species different from an animal species from which the biological sample (A) is derived.

[18] The method according to any one of [1] to [17], wherein the biological sample (A) is derived from one species selected from the group consisting of a mouse, a rat, a rabbit, a dog, a monkey and a human.

[19] The method according to any one of [1] to [18], wherein the biological sample (B) is derived from one species selected from the group consisting of a mouse, a rat, a rabbit, a dog, a monkey and a human.

[20] The method according to any one of [1] to [19], wherein CLogP of the analyte is 25 or less.

[21] The method according to any one of [1] to [20], wherein a molecular weight of the analyte is 5000 or less.

[22] The method according to any one of [1] to [21], wherein the analyte is a low-molecular-weight compound or a peptide compound.

[23] The method according to any one of [1] to [22], wherein the analyte is a peptide compound having a cyclic structure.

[24] The method according to any one of [1] to [23], wherein the analyte is a peptide compound containing a non-natural amino acid residue.

[25] The composition according to [24], wherein the non-natural amino acid residue is a non-natural N-substituted amino acid residue.

[26] The method according to any one of [1] to [25], wherein the analyte is a peptide compound having 7 or more and 30 or less amino acid residues.

[27] A method comprising using the relative $f_u$ ratio determined by the method according to any one of [1] to [26] and the data of clearance of a drug in a species from which one of the biological sample (A) or the biological sample (B) is derived, to predict clearance of a drug in a species from which the other biological sample is derived.

[28] A method comprising using the relative $f_u$ ratio determined by the method according to any one of [1] to [26] and the data of distribution volume of a drug in a species from which one of the biological sample (A) or the biological sample (B) is derived, to predict distribution volume of a drug in a species from which the other biological sample is derived.

[Advantageous Effects of Invention]

According to the method of the present invention, the relative $f_u$ ratio between different biological samples pertaining to an analyte can be acquired with high accuracy and in a short time without absolute necessity of reaching an equilibrium state in equilibrium dialysis even when the analyte is a compound having a high protein binding ratio. From the data of clearance or distribution volume of a drug in a species from which one biological sample is derived, the data of clearance or distribution volume of a drug in a species derived from another biological sample can be accurately predicted by using the relative $f_u$ ratio value.

[Brief Description of Drawings]

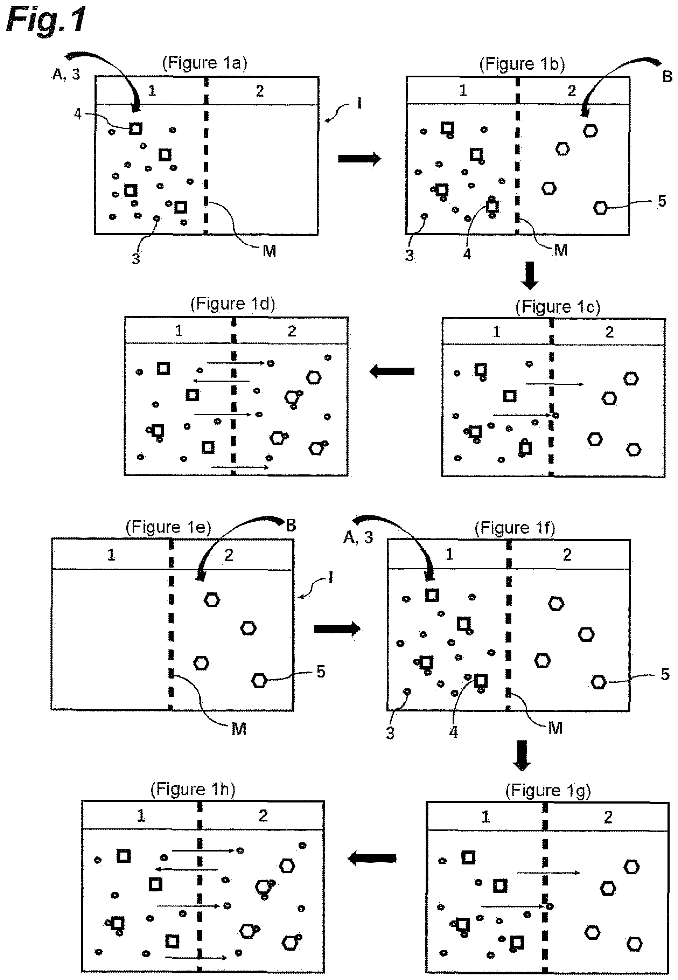

[Figure 1] Figure 1 (1a to 1h) shows an example of a schematic diagram of steps in the method of the present invention.

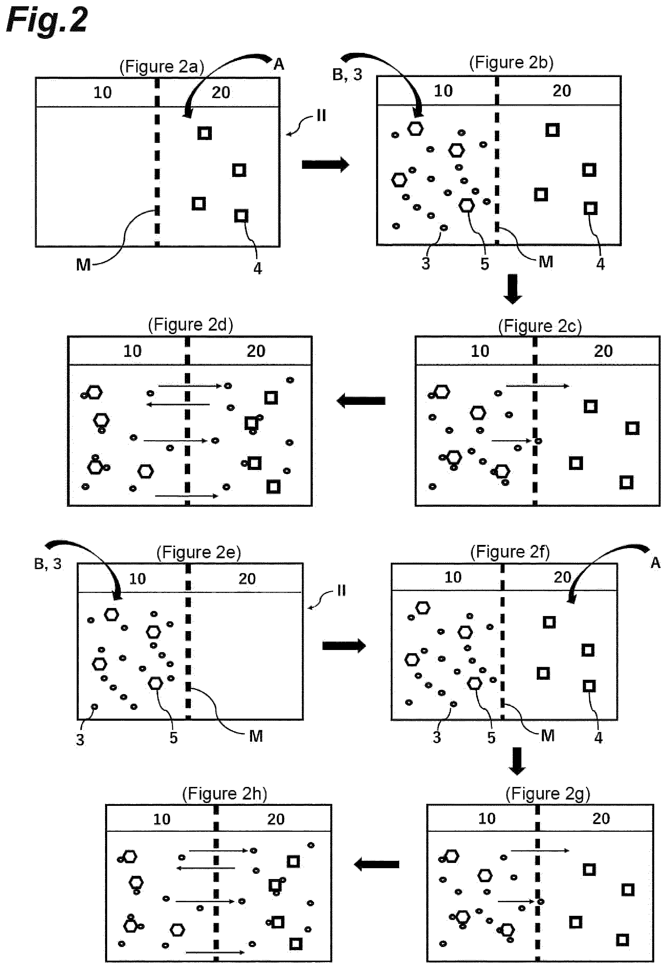

[Figure 2] Figure 2 (2a to 2h) shows an example of a schematic diagram of steps in the method of the present invention. Biological samples (A) and (B) are put in chambers different from those in Figure 1.

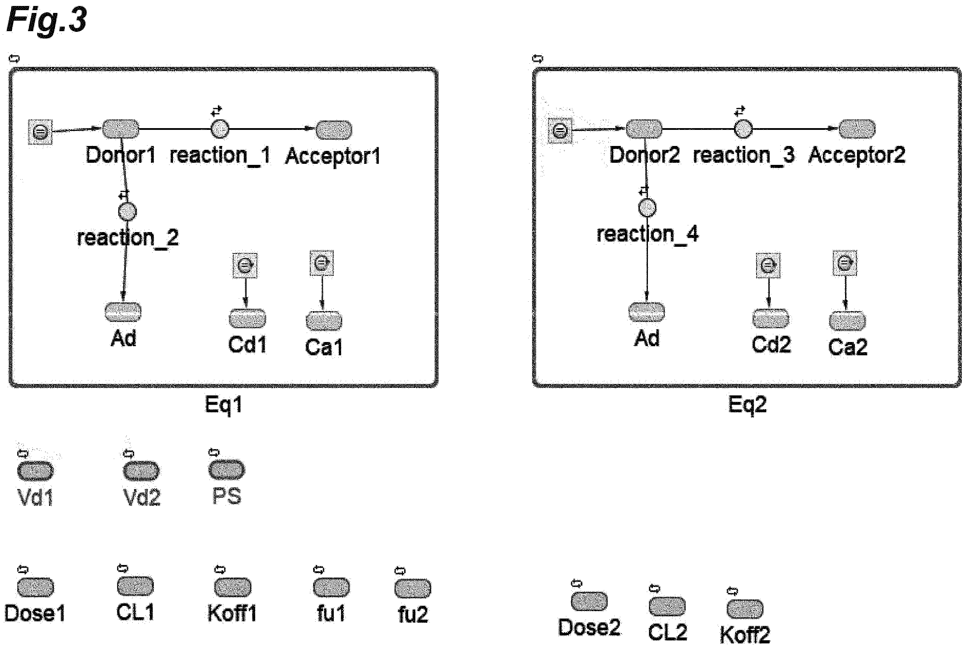

[Figure 3] Figure 3 is a diagram of a mathematical model in analysis performed with dynamic analysis software ("SimBiology (R)/MATLAB, version information: 9.7.0.1190202 (R2019b)", manufactured by The Math Works, Inc.).

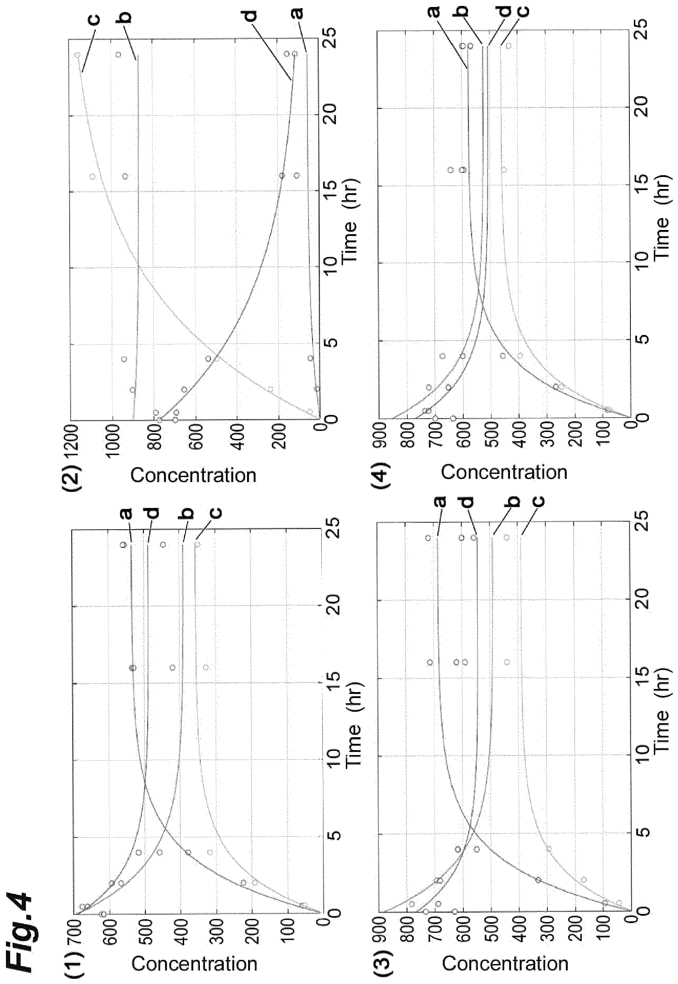

[Figure 4] Figure 4 ((1) to (4)) shows the plotted concentrations of an analyte in each solution (measured value: circle) (ordinate) at the time points of 0.5 hours, 2 hours, 4 hours, 16 hours and 24 hours after the start of dialysis reaction (abscissa) and fitting curves by dynamic analysis on those plots (predicted value: curve) when different biological samples (human serum in one chamber and mouse, rat, dog or monkey serum in the other chamber) are used and nifedipine is used as the analyte in accordance with the method of the present invention.

Figure 4(1) shows the results of using human serum and mouse serum. In Figure 4(1), the fitting curve a represents a time-dependent change of the concentration in the acceptor solution (human serum), and the fitting curve b represents a time-dependent change of the concentration in the donor solution (mouse serum). The fitting curve c represents a time-dependent change of the concentration in the acceptor solution (mouse serum), and the fitting curve d represents a time-dependent change of the concentration in the donor solution (human serum).
Figure 4(2) shows the results of using human serum and rat serum. In Figure 4(2), the fitting curve a represents a time-dependent change of the concentration in the acceptor solution (human serum), and the fitting curve b represents a time-dependent change of the concentration in the donor solution (rat serum). The fitting curve c represents a time-dependent change of the concentration in the acceptor solution (rat serum), and the fitting curve d represents a time-dependent change of the concentration in the donor solution (human serum).
Figure 4(3) shows the results of using human serum and dog serum. In Figure 4(3), the fitting curve a represents a time-dependent change of the concentration in the acceptor solution (human serum), and the fitting curve b represents a time-dependent change of the concentration in the donor solution (dog serum). The fitting curve c represents a time-dependent change of the concentration in the acceptor solution (dog serum), and the fitting curve d represents a time-dependent change of the concentration in the donor solution (human serum).
Figure 4(4) shows the results of using human serum and monkey serum. In Figure 4(4), the fitting curve a represents a time-dependent change of the concentration in the acceptor solution (human serum), and the fitting curve b represents a time-dependent change of the concentration in the donor solution (monkey serum). The fitting curve c represents a time-dependent change of the concentration in the acceptor solution (monkey serum), and the fitting curve d represents a time-dependent change of the concentration in the donor solution (human serum).

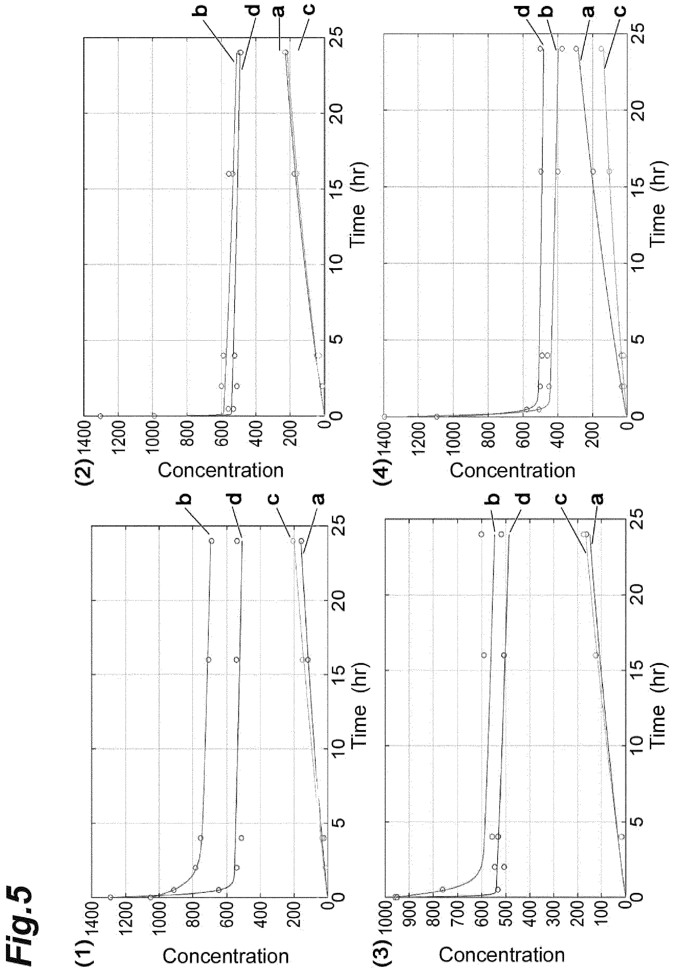

[Figure 5] Figure 5 ((1) to (4)) shows the plotted concentrations of an analyte in each solution (measured value: circle) (ordinate) at the time points of 0.5 hours, 2 hours, 4 hours, 16 hours and 24 hours after the start of dialysis reaction (abscissa) and fitting curves by dynamic analysis on those plots (predicted value: curve) when different biological samples (human serum in one chamber and mouse, rat, dog or monkey serum in the other chamber) are used and cyclosporine is used as the analyte in accordance with the present invention.

Figure 5(1) shows the results of using human serum and mouse serum. In Figure 5(1), the fitting curve a represents a time-dependent change of the concentration in the acceptor solution (human serum), and the fitting curve b represents a time-dependent change of the concentration in the donor solution (mouse serum). The fitting curve c represents a time-dependent change of the concentration in the acceptor solution (mouse serum), and the fitting curve d represents a time-dependent change of the concentration in the donor solution (human serum).
Figure 5(2) shows the results of using human serum and rat serum. In Figure 5(2), the fitting curve a represents a time-dependent change of the concentration in the acceptor solution (human serum), and the fitting curve b represents a time-dependent change of the concentration in the donor solution (rat serum). The fitting curve c represents a time-dependent change of the concentration in the acceptor solution (rat serum), and the fitting curve d represents a time-dependent change of the concentration in the donor solution (human serum).
Figure 5(3) shows the results of using human serum and dog serum. In Figure 5(3), the fitting curve a represents a time-dependent change of the concentration in the acceptor solution (human serum), and the fitting curve b represents a time-dependent change of the concentration in the donor solution (dog serum). The fitting curve c represents a time-dependent change of the concentration in the acceptor solution (dog serum), and the fitting curve d represents a time-dependent change of the concentration in the donor solution (human serum).

Figure 5(4) shows the results of using human serum and monkey serum. **In** Figure 5(4), the fitting curve a represents a time-dependent change of the concentration in the acceptor solution (human serum), and the fitting curve b represents a time-dependent change of the concentration in the donor solution (monkey serum). The fitting curve c represents a time-dependent change of the concentration in the acceptor solution (monkey serum), and the fitting curve d represents a time-dependent change of the concentration in the donor solution (human serum).

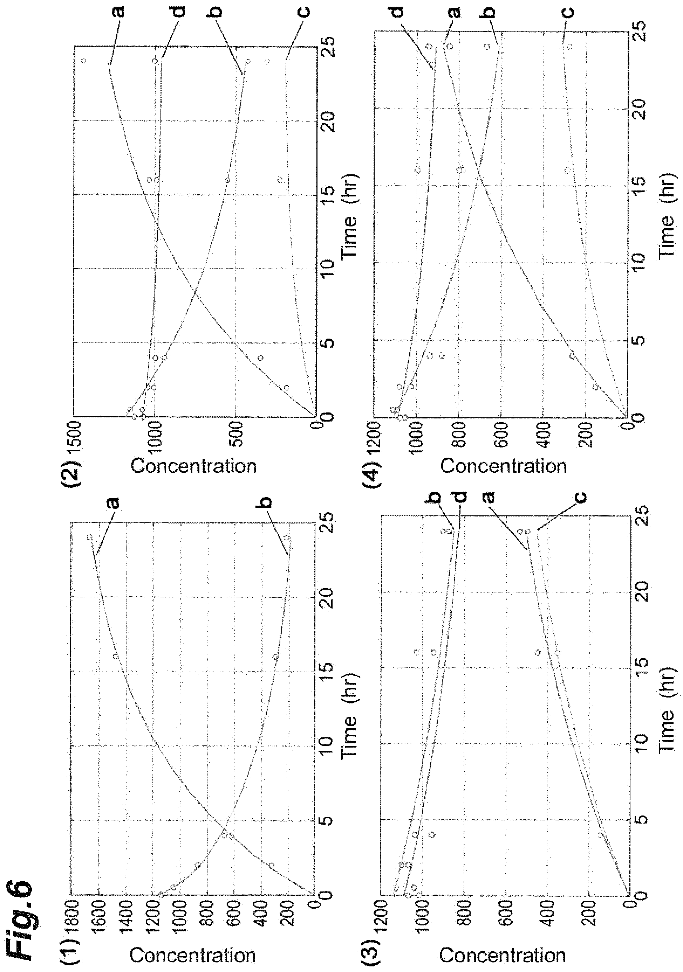

[Figure 6] Figure 6 ((1) to (4)) shows the plotted concentrations of an analyte in each solution (measured value: circle) (ordinate) at the time points of 0.5 hours, 2 hours, 4 hours, 16 hours and 24 hours after the start of dialysis reaction (abscissa) and fitting curves by dynamic analysis on those plots (predicted value: curve) when different biological samples (human serum in one chamber and mouse, rat, dog or monkey serum in the other chamber) are used and ibuprofen is used as the analyte in accordance with the method of the present invention.

Figure 6(1) shows the results of using human serum and mouse serum. **In** Figure 6(1), the fitting curve a represents a time-dependent change of the concentration in the acceptor solution (human serum), and the fitting curve b represents a time-dependent change of the concentration in the donor solution (mouse serum).
Figure 6(2) shows the results of using human serum and rat serum. **In** Figure 6(2), the fitting curve a represents a time-dependent change of the concentration in the acceptor solution (human serum), and the fitting curve b represents a time-dependent change of the concentration in the donor solution (rat serum). The fitting curve c represents a time-dependent change of the concentration in the acceptor solution (rat serum), and the fitting curve d represents a time-dependent change of the concentration in the donor solution (human serum).
Figure 6(3) shows the results of using human serum and dog serum. **In** Figure 6(3), the fitting curve a represents a time-dependent change of the concentration in the acceptor solution (human serum), and the fitting curve b represents a time-dependent change of the concentration in the donor solution (dog serum). The fitting curve c represents a time-dependent change of the concentration in the acceptor solution (dog serum), and the fitting curve d represents a time-dependent change of the concentration in the donor solution (human serum).
Figure 6(4) shows the results of using human serum and monkey serum. **In** Figure 6(4), the fitting curve a represents a time-dependent change of the concentration in the acceptor solution (human serum), and the fitting curve b represents a time-dependent change of the concentration in the donor solution (monkey serum). The fitting curve c represents a time-dependent change of the concentration in the acceptor solution (monkey serum), and the fitting curve d represents a time-dependent change of the concentration in the donor solution (human serum).

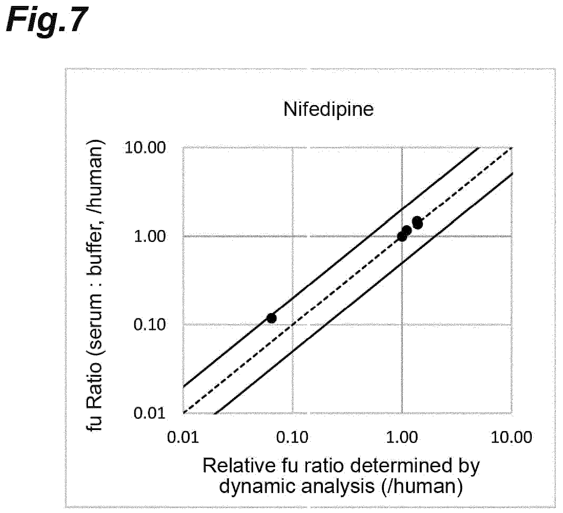

[Figure 7] Figure 7 shows a graph in which a relative $f_u$ ratio obtained in accordance with the method of the present invention [a $f_u$ ratio determined by dynamic analysis using data at the time points of 0.5 hours, 2 hours, 4 hours, 16 hours and 24 hours after the start of dialysis reaction when different biological samples (human serum in one chamber and mouse, rat, dog or monkey serum in the other chamber) are used and nifedipine is used as an analyte] (x axis) is compared with a $f_u$ ratio determined by the method of Reference Example (a $f_u$ ratio determined by static analysis using mouse, rat, dog, monkey or human serum in one chamber and a buffer solution in the other chamber and using data at the time point of 24 hours after the start of dialysis reaction) (y axis). **In** the graph, the area surrounded by two solid oblique lines represents an area where the ratio between the $f_u$ ratio shown on the ordinate and the $f_u$ ratio shown on the abscissa is 0.5 or more and 2.0 or less.

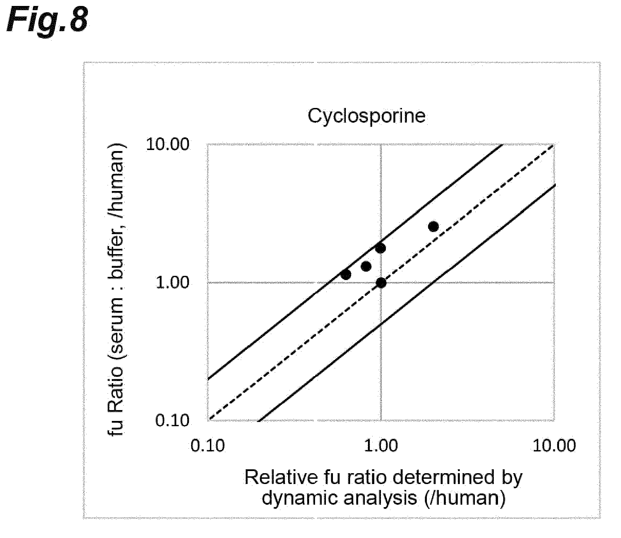

[Figure 8] Figure 8 shows a graph in which a relative $f_u$ ratio obtained in accordance with the method of the present invention [a $f_u$ ratio determined by dynamic analysis using data at the time points of 0.5 hours, 2 hours, 4 hours, 16 hours and 24 hours after the start of dialysis reaction when different biological samples (human serum in one chamber and mouse, rat, dog or monkey serum in the other chamber) are used and cyclosporine is used as an analyte] (x axis) is compared with a $f_u$ ratio determined by the method of Reference Example (a $f_u$ ratio determined by static analysis using mouse, rat, dog, monkey or human serum in one chamber and a buffer solution in the other chamber and using data at the time point of 24 hours after the start of dialysis reaction) (y axis). In the graph, the area surrounded by two solid oblique lines represents an area where the ratio between the $f_u$ ratio shown on the ordinate and the $f_u$ ratio shown on the abscissa is 0.5 or more and 2.0 or less.

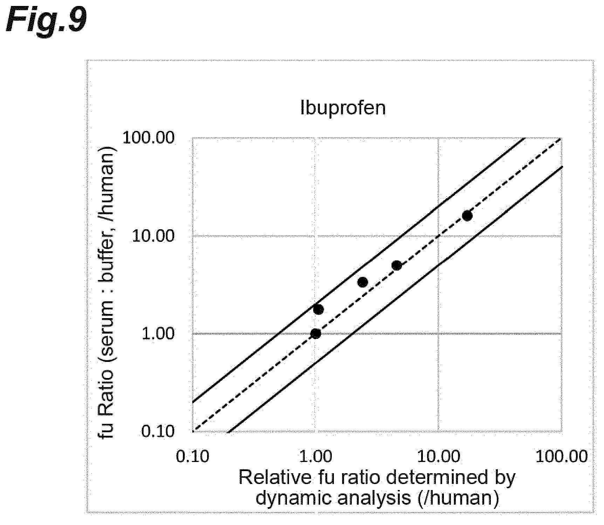

[Figure 9] Figure 9 shows a graph in which a relative $f_u$ ratio obtained in accordance with the method of the present invention [a $f_u$ ratio determined by dynamic analysis using data at the time points of 0.5 hours, 2 hours, 4 hours, 16 hours and 24 hours after the start of dialysis reaction when different biological samples (human serum in one chamber and mouse, rat, dog or monkey serum in the other chamber) are used and ibuprofen is used as an analyte] (x axis) is compared with a $f_u$ ratio determined by the method of Reference Example (a $f_u$ ratio determined by static analysis using mouse, rat, dog, monkey or human serum in one chamber and a buffer solution in the other chamber and using data at the time point of 24 hours after the start of dialysis reaction) (y axis). In the graph, the area surrounded by two solid oblique lines represents an area where the ratio between the $f_u$ ratio shown on the ordinate and the $f_u$ ratio shown on the abscissa is 0.5 or more and 2.0 or less.

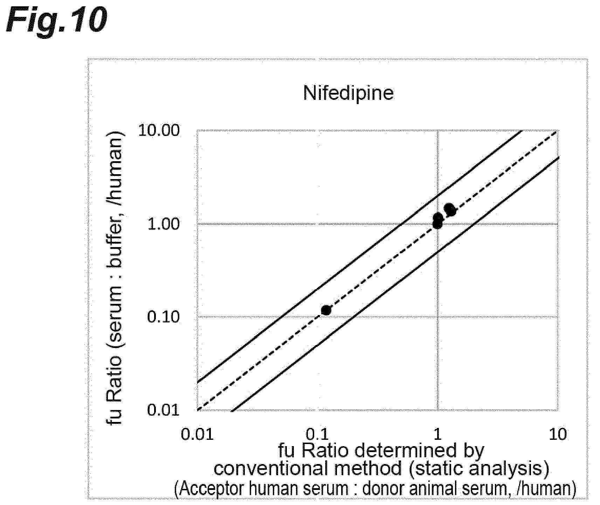

[Figure 10] Figure 10 shows a graph in which a relative $f_u$ ratio determined by the method of the present invention [a $f_u$ ratio determined by static analysis using data at the time points of 24 hours after the start of dialysis reaction when different biological samples (human serum in one chamber and mouse, rat, dog or monkey serum in the other chamber) are used and nifedipine is used as an analyte] (x axis) is compared with a $f_u$ ratio determined by the method of Reference Example (a $f_u$ ratio determined by static analysis using mouse, rat, dog, monkey or human serum in one chamber and a buffer solution in the other chamber and using data at the time point of 24 hours after the start of dialysis reaction) (y axis). In the graph, the area surrounded by two solid oblique lines represents an area where the ratio between the $f_u$ ratio shown on the ordinate and the $f_u$ ratio shown on the abscissa is 0.5 or more and 2.0 or less.

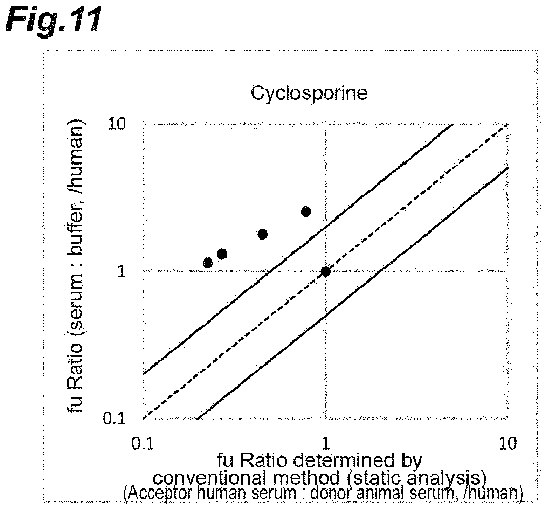

[Figure 11] Figure 11 shows a graph in which a relative $f_u$ ratio determined by the method of the present invention [a $f_u$ ratio determined by static analysis using data at the time points of 24 hours after the start of dialysis reaction when different biological samples (human serum in one chamber and mouse, rat, dog or monkey serum in the other chamber) are used and cyclosporine is used as an analyte] (x axis) is compared with a $f_u$ ratio determined by the method of Reference Example (a $f_u$ ratio determined by static analysis using mouse, rat, dog, monkey or human serum in one chamber and a buffer solution in the other chamber and using data at the time point of 24 hours after the start of dialysis reaction) (y axis). In the graph, the area surrounded by two solid oblique lines represents an area where the ratio between the $f_u$ ratio shown on the ordinate and the $f_u$ ratio shown on the abscissa is 0.5 or more and 2.0 or less.

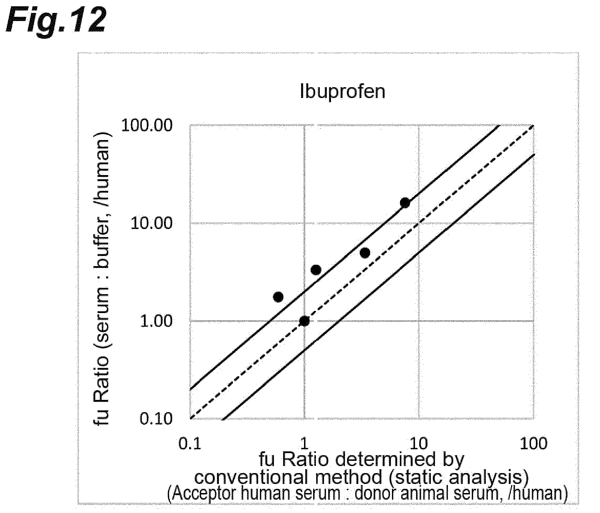

[Figure 12] Figure 12 shows a graph in which a relative $f_u$ ratio determined by the method of the present invention [a $f_u$ ratio determined by static analysis using data at the time points of 24 hours after the start of dialysis reaction when different biological samples (human serum in one chamber and mouse, rat, dog or monkey serum in the other chamber) are used and ibuprofen is used as an analyte] (x axis) is compared with a $f_u$ ratio determined by the method of Reference Example (a $f_u$ ratio determined by static analysis using mouse, rat, dog, monkey or human serum in one chamber and a buffer solution in the other chamber and using data at the time point of 24 hours after the start of dialysis reaction) (y axis). In the graph, the area surrounded by two solid oblique lines represents an area where the ratio between the $f_u$ ratio shown on the ordinate and the $f_u$ ratio shown on the abscissa is 0.5 or more and 2.0 or less.

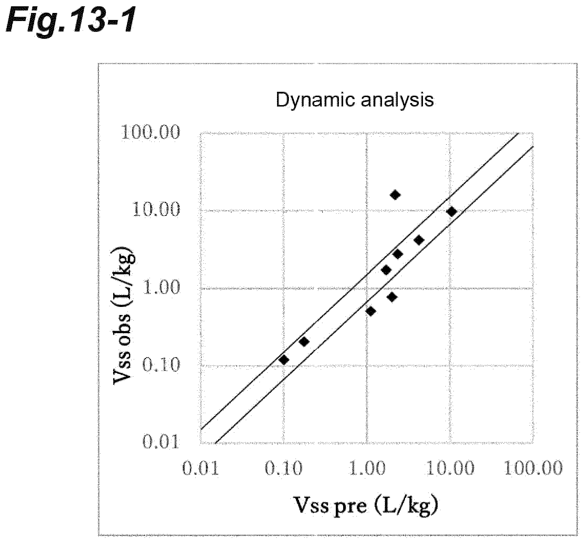

[Figure 13-1] Figure 13-1 is a graph showing a comparison between an observed value of human steady state distribution volume (ordinate: Vss obs (L/kg)) and a predicted value of steady state distribution volume in a human (abscissa: Vss pre (L/kg)) which is determined in accordance with a known method for predicting distribution volume with the use of the relative $f_u$ ratio (rat/human) of each analyte which is determined by the method of the present invention in each of Examples 1, 3, 4, 6 and 8 to 12 (dynamic analysis). The solid oblique line in the graph represents an area where the error of the predicted value to the observed value is 0.67 or more and 1.5 or less.

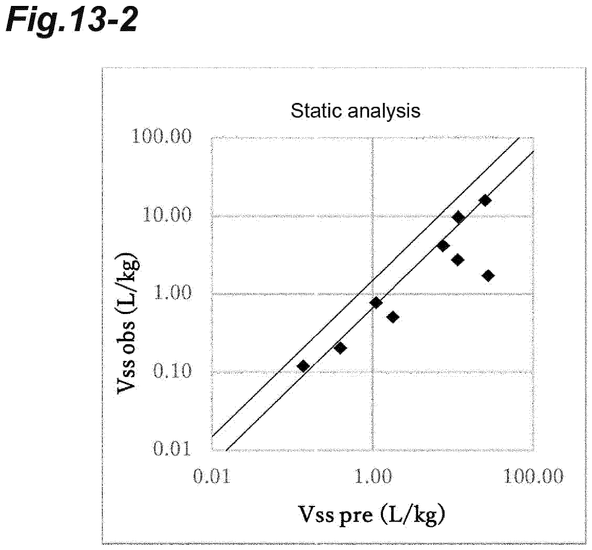

[Figure 13-2] Figure 13-2 is a graph showing a comparison between an observed value of human steady state distribution volume (ordinate: Vss obs (L/kg)) and a predicted value of steady state distribution volume in a human (abscissa: Vss pre (L/kg)) which is determined in accordance with a known method for predicting distribution volume with the use of the $f_u$ ratio (rat/human) of the analyte in each of Comparative Examples 1, 3, 4, 6 and 8 to 12 (static analysis). The solid oblique line in the graph represents an area where the error of the predicted value to the observed value is 0.67 or more and 1.5 or less.

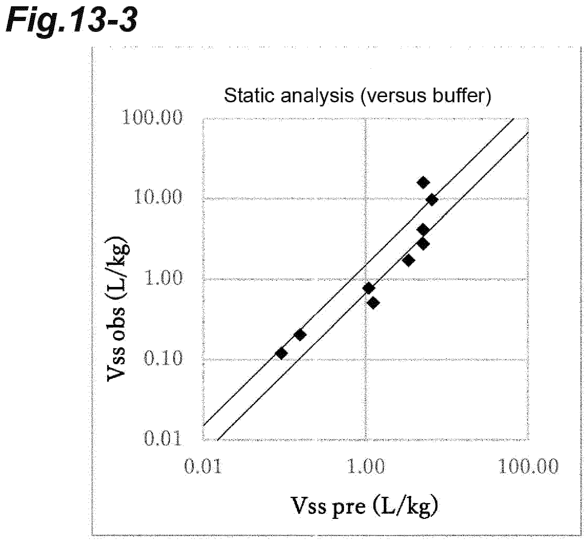

[Figure 13-3] Figure 13-3 is a graph showing a comparison between an observed value of human steady state distribution volume (ordinate: Vss obs (L/kg)) and a predicted value of steady state distribution volume in a human (abscissa: Vss pre (L/kg)) which is determined in accordance with a known method for predicting distribution volume with the use of the $f_u$ ratio (rat/human) of the analyte in each of Reference Examples 1, 3, 4, 6 and 8 to 12 (static analysis (versus buffer)). The solid oblique line in the graph represents an area where the error of the predicted value to the observed value is 0.67 or more and 1.5 or less.

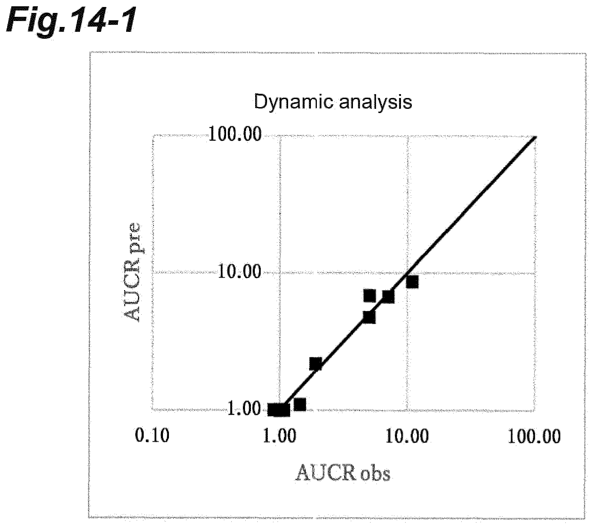

[Figure 14-1] Figure 14-1 is a graph showing a comparison between an observed value of area under the blood concentration-time curve ratio (AUCR) of midazolam (abscissa: AUCR obs) and a predicted value of AUCR of midazolam as CYP3A4 substrate (ordinate: AUCR pre) from the relative $f_u$ ratio determined in each of Examples 14 to 20 and the Ki value used in the inhibitor experiment. The solid oblique line in the graph represents a line where the ratio of the predicted value of AUCR to the observed value of AUCR is 1 (a line where the observed value and the predicted value of AUCR coincide with each other).

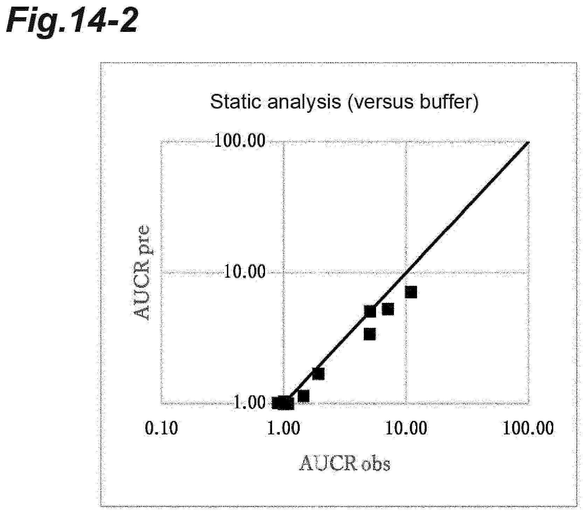

[Figure 14-2] Figure 14-2 is a graph showing a comparison between an observed value of AUCR of midazolam (abscissa: AUCR obs) and a predicted value of AUCR of midazolam as CYP3A4 substrate (ordinate: AUCR pre) from the $f_u$ value determined in each of Reference Examples 14 to 20 and the Ki value used in the inhibitor experiment. The solid oblique line in the graph represents a line where the ratio of the predicted value of AUCR to the observed value of AUCR is 1 (a line where the observed value and the predicted value of AUCR coincide with each other).

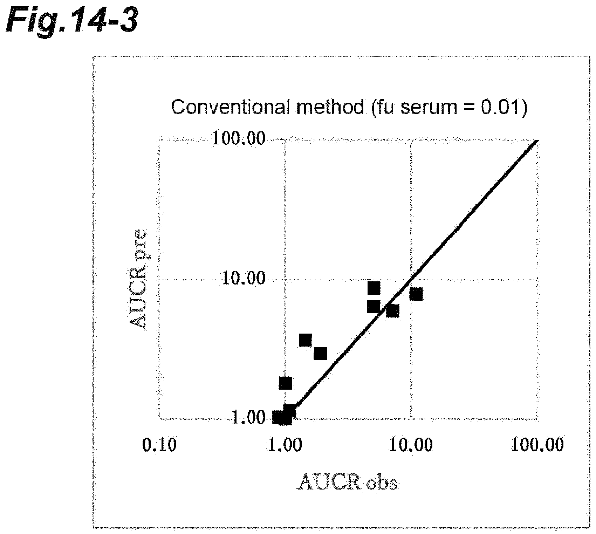

[Figure 14-3] Figure 14-3 is a graph showing a comparison between an observed value of AUCR of midazolam (abscissa: AUCR obs) and a predicted value of AUCR of midazolam as CYP3A4 substrate (ordinate: AUCR pre) from the $f_u$ ratio determined in each of Reference Examples 21 to 27 and the Ki value used in the inhibitor experiment. The solid oblique line in the graph represents a line where the ratio of the predicted value of AUCR to the observed value of AUCR is 1 (a line where the observed value and the predicted value of AUCR coincide with each other).

[Description of Embodiments]

Terms in the present specification

In the present specification, the "analyte" is a compound of which a relative $f_u$ ratio is measured, and a low-molecular-weight compound or a peptide compound is preferable.

The "low-molecular-weight compound" in the present specification refers to a compound other than peptide compounds described later, which has a molecular weight of preferably 2000 or less, and examples thereof include a natural compound and a new molecular entity (NME) produced by a process including chemical synthesis. The molecular weight of the low-molecular-weight compound is more preferably 1000 or less, still more preferably 800 or less, particularly preferably 500 or less. The CLogP of the low-molecular-weight compound is preferably 25 or less, more preferably 16 or less, still more preferably 10 or less, still more preferably 7 or less, still more preferably 6 or less, still more preferably 5 or less, still more preferably 4 or less. The CLogP is a distribution coefficient calculated by a computer with a compound divided into partial structures (calculation software is known; and the calculation can be performed using, for example, software from Daylight Chemical Information Systems, Inc.).

The "peptide compound" in the present specification is not particularly limited as long as it is a peptide compound in which natural amino acids and/or non-natural amino acids are linked through an amide bond or an ester bond, and a peptide compound having preferably 5 or more residues, more preferably 7 or more residues, still more preferably 8 or more residues, particularly preferably 9 or more residues, and preferably 30 or less residues, more preferably 25 or less residues, still more preferably 15 or less residues, particularly preferably 13 or less residues is desirable. For example, the peptide compound may be a peptide compound having preferably 5 or more and 30 or less residues, more preferably 7 or more and 25 or less residues, still more preferably 8 or more and 15 or less residues, particularly preferably 9 or more and 13 or less residues.

The peptide compound that can be used in the present invention contains preferably at least 3 N-substituted amino acids, more preferably at least 5 or more N-substituted amino acids, in one peptide. These N-substituted amino acids may be present continuously or discontinuously in the peptide compound. The peptide compound in the present invention may be linear or cyclic, and is preferably a cyclic peptide compound. The peptide compound may have a branched structure. Further, among the peptide compounds having a non-natural amino acid residue, peptide compounds having a N-methylated non-natural amino acid residue are preferable.

The molecular weight of the peptide compound is preferably 5000 or less, more preferably 3000 or less, still more preferably 2000 or less, and preferably 500 or more, more preferably 800 or more, still more preferably 1000 or more. For example, the molecular weight of the peptide compound is preferably 500 or more and 5000 or less, more preferably 800 or more and 3000 or less, still more preferably 1000 or more and 2000 or less.

The CLogP of the peptide compound is preferably 25 or less, more preferably 22 or less, still more preferably 20 or less, still more preferably 18 or less, still more preferably 16 or less, still more preferably 15 or less, and preferably 5 or more, more preferably 10 or more, still more preferably 12 or more. For example, the CLogP of the peptide compound is preferably 5 or more and 25 or less, more preferably 10 or more and 20 or less, still more preferably 12 or more and 18 or less.

The "cyclic peptide compound" in the present specification is not particularly limited as long as it is a peptide compound having a cyclic portion composed of 5 or more amino acid residues, and the method for cyclization of the peptide compound is also not limited. The cyclic peptide compound is a cyclic peptide compound which can be obtained by cyclizing a N-terminal-side group and a C-terminal-side group of a linear peptide compound. The cyclization may take any form, such as cyclization with a carbon-nitrogen bond such as an amide bond, cyclization with a carbon-oxygen bond such as an ester bond or ether bond, cyclization with a carbon-sulfur bond such as a thioether bond, cyclization with a carbon-carbon bond, or cyclization by heterocyclic construction. Among these, cyclization through covalent bonds such as amide bonds and carbon-carbon bonds is preferred, and cyclization through an amide bond by a carboxy group of the side chain and a N-terminal amino group of the main chain is more preferable. The positions of the carboxy group, the amino group, and the like used for cyclization may be on the main chain or the side chain, and are not particularly limited as long as the positions allow the groups to be cyclized.

The "amino acid" in the present specification includes a natural amino acid and a non-natural amino acid (sometimes also referred to as an amino acid derivative). In the present specification, the "natural amino acid" refers to Gly, Ala, Ser, Thr, Val, Leu, Ile, Phe, Tyr, Trp, His, Glu, Asp, Gln, Asn, Cys, Met, Lys, Arg, or Pro. Non-natural amino acids (amino acid derivatives) are not particularly limited, and examples thereof include a $\beta$-amino acid, a D-type amino acid, an N-substituted amino acid (excluding Pro), an $\alpha,\alpha$-disubstituted amino acid, an amino acid having a side chain different from that of natural amino acids, and a hydroxycarboxylic acid. As the amino acids herein, amino acids having any conformation are acceptable. The selection of a side chain of an amino acid is not particularly limited, and the side chain is freely selected from, in addition to a hydrogen atom, an alkyl group, an alkenyl group, an alkynyl group, an aryl group, a heteroaryl group, an aralkyl group, a heteroaralkyl group, a cycloalkyl group, a spiro-bonded cycloalkyl group, and the like. Each of the side chains may have a substituent. The substituent is also not limited, and one or two or more substituents may be freely selected independently from any substituents including, for example, a halogen atom, an O atom, a S atom, a N atom, a B atom, a Si atom, or a P atom. That is, examples of the side chain include an alkyl group, an alkoxy group, an alkenyl group, an alkynyl group, an aryl group, a heteroaryl group, an aralkyl group, or a cycloalkyl group which may be substituted, oxo, aminocarbonyl, and a halogen atom. In a non-limiting aspect, the amino acid in the present specification may be a compound having a carboxy group and an amino group in the same molecule (even in this case, the amino acid also includes imino acids such as proline and hydroxyproline).

Examples of halogen-derived substituents include fluoro (-F), chloro (-Cl), bromo (-Br), and iodo (-I).

Examples of O-atom-derived substituents include hydroxy (-OH), oxy (-OR), carbonyl (-C=O-R), carboxy (-CO$_2$H), oxycarbonyl (-C=O-OR), carbonyloxy (-O-C=O-R), thiocarbonyl (-C=O-SR), a carbonylthio group (-S-C=O-R), aminocarbonyl (-C=O-NHR), carbonylamino (-NH-C=O-R), oxycarbonylamino (-NH-C=O-OR), sulfonylamino (-NH-SO$_2$-R), aminosulfonyl (-SO$_2$-NHR), sulfamoylamino (-NH-SO$_2$-NHR), thiocarboxy (-C(=O)-SH), and carboxylcarbonyl (-C(=O)-CO$_2$H).

Examples of the oxy (-OR) include alkoxy, cycloalkoxy, alkenyloxy, alkynyloxy, aryloxy, heteroaryloxy, and aralkyloxy.

Examples of the carbonyl (-C=O-R) include formyl (-C=O-H), alkylcarbonyl, cycloalkylcarbonyl, alkenylcarbonyl, alkynylcarbonyl, arylcarbonyl, heteroarylcarbonyl, and aralkylcarbonyl.

Examples of the oxycarbonyl (-C=O-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of the carbonyloxy (-O-C=O-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of the thiocarbonyl (-C=O-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of the carbonylthio (-S-C=O-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C=O-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. Examples thereof additionally include compounds in which the H atom bonded to the N atom in -C=O-NHR is further replaced with alkyl, cycloalkyl, alkenyl, alkynyl, aryl, heteroaryl, or aralkyl.

Examples of the carbonylamino (-NH-C=O-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Examples thereof additionally include compounds in which the H atom bonded to the N atom in -NH-C=O-R is further replaced with alkyl, cycloalkyl, alkenyl, alkynyl, aryl, heteroaryl, or aralkyl.

Examples of the oxycarbonylamino (-NH-C=O-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. Examples thereof additionally include compounds in which the H atom bonded to the N atom in -NH-C=O-OR is further replaced with alkyl, cycloalkyl, alkenyl, alkynyl, aryl, heteroaryl, or aralkyl.

Examples of the sulfonylamino (-NH-SO$_2$-R) include alkylsulfonylamino, cycloalkylsulfonylamino, alkenylsulfonylamino, alkynylsulfonylamino, arylsulfonylamino, heteroarylsulfonylamino, and aralkylsulfonylamino. Examples thereof additionally include compounds in which the H atom bonded to the N atom in -NH-SO$_2$-R is further replaced with alkyl, cycloalkyl, alkenyl, alkynyl, aryl, heteroaryl, or aralkyl.

Examples of the aminosulfonyl (-SO$_2$-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Examples thereof additionally include compounds in which the H atom bonded to the N atom in -SO$_2$-NHR is further replaced with alkyl, cycloalkyl, alkenyl, alkynyl, aryl, heteroaryl, or aralkyl.

Examples of the sulfamoylamino (-NH-SO$_2$-NHR) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. The two H atoms bonded to the N atoms in -NH-SO$_2$-NHR may be substituted by substituents each independently selected from the group consisting of alkyl, cycloalkyl, alkenyl, alkynyl, aryl, heteroaryl, and aralkyl, and these two substituents may form a ring.

Examples of S-atom-derived substituents include thiol (-SH), thio (-S-R), sulfinyl (-S=OR), sulfonyl (-S(O)$_2$-R), sulfo (-SO$_3$H), and pentafluorosulfanyl (-SF$_5$).

Examples of the thio (-S-R) that can be selected include alkylthio, cycloalkylthio, alkenylthio, alkynylthio, arylthio, heteroarylthio, and aralkylthio.

Examples of sulfinyl (-S=O-R) include alkylsulfinyl, cycloalkylsulfinyl, alkenylsulfinyl, alkynylsulfinyl, arylsulfinyl, heteroarylsulfinyl, and aralkylsulfinyl.

Examples of the sulfonyl (-S(O)$_2$-R) include alkylsulfonyl, cycloalkylsulfonyl, alkenylsulfonyl, alkynylsulfonyl, arylsulfonyl, heteroarylsulfonyl, and aralkylsulfonyl.

Examples of N-atom-derived substituents include azido (-N$_3$, also referred to as "azide group"), cyano (-CN), primary amino (-NH$_2$), secondary amino (-NH-R), tertiary amino (-NR(R')), amidino (-C(=NH)-NH$_2$), substituted amidino (-C(=NR)-NR'R"), guanidino (-NH-C(=NH)-NH$_2$), substituted guanidino (-NR-C(=NR''')-NR'R"), and aminocarbonylamino (-NR-CO-NR'R").

Examples of secondary amino (-NH-R) include alkylamino, cycloalkylamino, alkenylamino, alkynylamino, arylamino, heteroarylamino, and aralkylamino.

Examples of tertiary amino (-NR(R')) include an amino group having any two substituents each independently selected from alkyl, cycloalkyl, alkenyl, alkynyl, aryl, heteroaryl, aralkyl, and the like, such as alkyl(aralkyl)amino, and the any two substituents may form a ring.

Examples of the substituted amidino (-C(=NR)-NR'R") include groups in which three substituents R, R', and R" on the N atoms are each independently selected from alkyl, cycloalkyl, alkenyl, alkynyl, aryl, heteroaryl, and aralkyl, for example, alkyl(aralkyl)(aryl)amidino.

Examples of the substituted guanidino (-NR-C(=NR''')-NR'R") include groups in which R,R', R", and R''' are each independently selected from alkyl, cycloalkyl, alkenyl, alkynyl, aryl, heteroaryl, and aralkyl, and groups in which these substituents form a ring.

Examples of the aminocarbonylamino (-NR-CO-NR'R") include groups in which R, R', and R" are each independently selected from a hydrogen atom, alkyl, cycloalkyl, alkenyl, alkynyl, aryl, heteroaryl, and aralkyl, and groups in which these substituents form a ring.

Examples of B-atom-derived substituents include boryl (-BR(R')), and dioxyboryl (-B(OR)(OR')). The two substituents R and R' may be each independently selected from alkyl, cycloalkyl, alkenyl, alkynyl, aryl, heteroaryl, aralkyl, or the like, or may form a ring. Specific examples of the substituent include a cyclic boryl group, and more specific examples thereof include a pinacolatoboryl group, a neopentanediolatoboryl group, and a catecholatoboryl group.

Specific examples of the substituent on the nitrogen atom of the N-substituted amino acid in the present specification include alkyl, C$_1$-C$_6$ alkyl, C$_1$-C$_4$ alkyl, methyl, C$_7$-C$_{14}$ aralkyl, benzyl, and phenethyl.

The amino group of the main chain of the amino acid may be unsubstituted (-NH$_2$) or substituted (i.e., -NHR, wherein R represents, for example, alkyl, alkenyl, alkynyl, aryl, heteroaryl, aralkyl or cycloalkyl optionally having a substituent, or a carbon chain bonded to the N atom and a carbon atom at position $\alpha$ may form a ring as in proline). In the present specification, such an amino acid in which an amino group on the main chain is substituted is sometimes referred to as "N-substituted amino acid". Examples of the "N-substituted amino acid" as used herein include preferably, but are not limited to, N-alkylamino acid, N-C$_1$-C$_6$ alkylamino acid, N-C$_1$-C$_4$ alkylamino acid, N-methylamino acid, N-C$_7$-C$_{14}$ aralkyl amino acid, N-benzylamino acid, and N-phenethylamino acid.

The "amino acid" as used herein includes all corresponding isotopes to each. The isotope of "amino acid" is one in which at least one atom is substituted with an atom having the same atomic number (the same number of protons) and a different mass number (different sum of numbers of protons and neutrons). Examples of the isotope included in the "amino acid" as used herein include a hydrogen atom, a carbon atom, a nitrogen atom, an oxygen atom, a phosphorus atom, a sulfur atom, a fluorine atom, and a chlorine atom, and they include $^2$H, $^3$H; $^{13}$C, $^{14}$C; $^{15}$ $^{17}$O, $^{18}$O; $^{32}$P; $^{35}$S; $^{18}$F; $^{36}$Cl; and the like, respectively.

The "biological sample" for use in the present invention means a biotic sample derived from an organism, preferably a biotic sample derived from a mammal. Example of the mammal include preferably a mouse, a rat, a rabbit, a dog, a monkey, and a human. Example of the biotic sample include preferably a body fluid, a tissue, a cell, and a portion thereof, more preferably a biotic sample selected from the group consisting of a microsomal fraction, blood, plasma and serum.

In the present invention, the term "different biological samples" means two or more different biological samples. As different biological samples, biotic samples derived from organisms of different species (e.g. mouse and human, or rat and human), or biotic samples derived from organisms of the same species can be used. Further, different biological samples include biotic samples from the same biological portion or different biological portions derived from organisms of different species, and biotic samples from the same biological portion or different biological portions derived from organisms of the same species. For example, different biological samples in the present invention include biotic sample portions of the same type such as a microsomal fraction and a microsomal fraction, blood and blood, plasma and plasma, or serum and serum, and different biotic sample portions such as, for example, blood and plasma, a microsomal fraction and plasma, or plasma and serum, which are derived from mammals of the same species or different species. The different biotic samples in the present invention may include even different biotic samples derived from the same biological portion of organisms of the same species when the biotic samples are derived from different individuals.

In the present specification, the "protein binding ratio" refers to a ratio (mass ratio or molar ratio, or percentage thereof) of an analyte binding to protein (e.g. "non-specific protein" such as albumin) in a biological sample to the total amount of the analyte. That is, protein binding ratio = 1 - fraction unbound ($f_u$). In the present invention, it is possible to use an analyte having a protein binding ratio of preferably 95% or more, more preferably 97% or more, still more preferably 98% or more, most preferably 99% or more.

In the present specification, the "fraction unbound ($f_u$)" refers to a ratio of an analyte, which is not bound to protein in a biological sample, to the total amount of the analyte in the presence of protein (e.g. albumin) (sometimes referred to as a "unbound analyte" in the present specification). That is, fraction unbound ($f_u$) = 1 - protein binding ratio. The "relative fraction unbound ratio (relative $f_u$ ratio)" means a ratio of the fractions unbound of analytes in different biological samples, respectively. For example, the relative $f_u$ ratio is a ratio of the $f_u$ value of an analyte in a biological sample (A) (fu1) to the $f_u$ value of an analyte in a biological sample (B) different from the biological sample (A) (fu2) (fu1/fu2).

In the method of the present invention, the "semipermeable membrane" divides the inside of the "chamber" into two or more parts. The "semipermeable membrane" is a membrane through which selected molecular species can pass by diffusion. The semipermeable membrane is selected such that the semipermeable membrane for equilibrium dialysis is permeable to an analyte (and a solvent such as a buffer solution if present), and is not permeable to a biological sample. Preferably, the semipermeable membrane is a size-selective membrane, and for example, the semipermeable membrane can be characterized by a molecular cutoff (MWCO). In this case, the MWCO of the semipermeable membrane is preferably 50 kDa or less, more preferably 14 kDa or less, still more preferably 8 kDa or less, and preferably 3.5 kDa or more, more preferably 4 kDa or more, still more preferably 6 kDa or more. For example, the MWCO of the semipermeable membrane may be preferably 3.5 kDa or more and 50 kDa or less, more preferably 4 kDa or more and 14 kDa or less, still more preferably 6 kDa or more and 8 kDa or less. The semipermeable membrane that can be used in the method of the present invention may be a known semipermeable membrane, for example, a semipermeable membrane formed of regenerated cellulose (Rapid Equilibrium Dialysis (RED) Device Inserts, 8K MWCO, manufactured by Thermo Fisher Scientific).

The chamber used may be a known chamber for use in the equilibrium dialysis method. For example, the chamber used may one made of Teflon or polypropylene.

In the present specification, the "donor side", "donor" or "Donor" means a side on which a donor solution containing an analyte is added in an early stage of measurement, and for example, the "donor-side chamber" or "donor chamber" means a chamber in which a donor solution containing an analyte is added in an early stage of measurement. The "acceptor side", "acceptor" or "Acceptor" means a side on which an acceptor solution free of an analyte is added in an early stage of measurement, and for example, the "acceptor-side chamber" or "acceptor chamber" means a chamber in which an acceptor solution free of an analyte is added in an early stage of measurement.

In the present specification, the "donor solution" means a solution which is added to the donor-side chamber (or donor chamber) and thereafter exists in the donor-side chamber (or donor chamber). The "acceptor solution" means a solution which is added to the acceptor-side chamber (or acceptor chamber) and thereafter exists in the acceptor-side chamber (or acceptor chamber).

In the present specification, the "early stage" is the time when the donor solution and the acceptor solution are added to the chambers and transfer of the analyte between the solutions is started. For example, the time point at which the donor solution and the acceptor solution are added into the chamber for the first time and stirring is immediately started can be taken as an "early stage". However, if the analyte has a property of being adsorbed to, for example, a wall surface of the chamber, it is difficult to accurately measure the concentration of the analyte in the donor solution in the early stage, and therefore it is preferable that the donor solution immediately before addition to the chamber be samples, and the concentration be measured, and used for analysis as a concentration of the analyte in the donor solution in the early stage (i.e. at time point 0).

In the present specification, the "equilibrium state" means a state in which between the solutions added to chambers partitioned by a semipermeable membrane, a change in concentration of the analyte reaches a steady state, so that there is substantially no change in concentration.

In the present invention, "measuring over time" means performing measurement while keeping track of time, and includes temporally continuously performing measurement, and non-continuously performing measurement two or more times on an every-fixed-time basis or an every-key-time basis.

In the present specification, the "dynamic analysis" refers to an analysis method in which the concentration or the like of an analyte in a solution in a chamber is measured over time, and convergence calculation (fitting) etc. is applied to the measured values with analysis software or the like to calculate a $f_u$ ratio ($f_u$ value). On the other hand, the "static analysis" refers to an analysis method in which a $f_u$ ratio ($f_u$ value) is calculated using measured values or a ratio of the measured values of the concentrations or the like of an analyte in solutions in chambers only at a specific time point. The specific time point is preferably a time point at the end of the operation of equilibrium dialysis reaction, still more preferably a time point at the end of equilibrium dialysis reaction after an equilibrium state is reached.

Method for measuring relative $f_u$ ratio of analyte between different biological samples (a)

The method for determining a relative fraction unbound ratio (relative $f_u$ ratio) of an analyte between different biological samples according to the present invention comprises the following steps (1) to (5):

(1) providing a chamber system (I) in which adjacent chambers are separated by a semipermeable membrane permeable to the analyte;
(2) adding a donor solution containing a first biological sample (A) and the analyte to one chamber (donor-side chamber) in the chamber system (I) (e.g. Figures 1a and 1f);
(3) adding an acceptor solution containing a second biological sample (B) to a chamber different from the donor-side chamber (acceptor-side chamber) in the chamber system (I) (e.g. Figures 1b and 1e);
(4) measuring concentrations of the analyte in the donor solution in the step (2) and the acceptor solution in the step (3) over time (e.g. Figures 1c, 1d, 1g and 1h); and
(5) calculating the relative $f_u$ ratio using data associated with the concentrations of the analyte which are measured in the step (4).

In the method of the present invention, the order in which the steps (2) and (3) are carried out is not particularly limited, and for example, step (3) may be carried out after step (2), step (2) may be carried out after step (3), or step (2) may be carried out simultaneously or in parallel with step (3) (e.g. Figures 1a, 1b, 1e and 1f).

Hereinafter, an example of the present invention will be described with reference to Figure 1. In the figure, the chamber system comprises two adjacent chambers (donor-side chamber and acceptor side-chamber) separated by a membrane M. The size-selective membrane M separates the two chambers, is permeable to an analyte, and is not permeable to non-specific protein derived from the first biological sample (A) and non-specific protein derived from the second biological sample (B).

Figure 1 (Figures 1a to 1d and 1e to 1h) show a dialysis chamber system I between assays. Figure 1a shows a state when a donor solution containing the first biological sample (A) containing non-specific protein 4, and an analyte 3 is added to the donor-side chamber 1 (black arrow), where the analyte 3 and the non-specific protein 4 exist in the donor solution in the donor-side chamber 1. Figure 1b shows a state when an acceptor solution containing the second biological sample (B) containing non-specific protein 5 is added to the acceptor-side chamber 2 (black arrow), where the non-specific protein 5 exists in the acceptor solution in the acceptor-side chamber 2. Figure 1c shows the situation of an early stage in which transfer of the analyte from the donor-side chamber 1 to the acceptor-side chamber 2 starts. Figure 1d shows the situation of a stage in which the analyte is transferred from the donor-side chamber 1 to the acceptor-side chamber 2, or optionally transferred from the acceptor-side chamber 2 to the donor-side chamber 1 conversely, so that dialysis proceeds to equilibrium. The concentration of the analyte in the solution added to each chamber is measured two or more times over time from the early stage.

Figures 1e to 1h show an example of a schematic diagram of a situation in which the order of the operations of Figures 1a and 1b is reversed.

Of the analyte 3 added to the donor-side chamber 1, only the analyte which has not been bound to non-specific protein 4 or a chamber wall passes through the semipermeable membrane. A part of the analyte passing through the semipermeable membrane binds to non-specific protein 5 in the acceptor-side chamber 2. The concentration of the analyte in the solution is directed to equilibrium between both chambers, but since the binding properties of the analyte to the non-specific proteins are different, the binding properties to each of the non-specific proteins have an impact on the total analyte distribution.

In the method of the present invention, the biological sample and the analyte added to the chamber may be diluted with a solvent. The solvent is not particularly limited as long as it is a solvent that does not have no substantial impact on the properties of the biological sample and the analyte, and dissolves the biological sample or the analyte, or is adequately mixed with the biological sample or the analyte. As such a solvent, for example, a buffer solution can be preferably used. Examples of the buffer solution include phosphate-based buffer solutions, Good's buffers such as tris buffer solution/-HEPES buffer solutions, acetate-based buffer solutions, citrate-based buffer solutions, citrate phosphate-based buffer solutions, borate-based buffer solutions, tartrate-based buffer solutions, carbonate-based buffer solutions, phthalate-based buffer solutions, and oxalate-based buffer solutions.

It is desirable that the osmotic pressure of the buffer solution be preferably 0.5 times or more, more preferably 0.9 times or more that of physiological saline, and preferably 2 times or less, more preferably 1.1 times or less that of physiological saline. For example, the osmotic pressure of the buffer solution may be in the range of preferably 0.5 times or more and 2 times or less, still more preferably 0.9 times or more and 1.1 times or less that of physiological saline. It is desirable that the pH of the buffer solution be preferably 6 or more, more preferably 7.0 or more, and preferably 8 or less, still more preferably 7.8 or less. For example, the pH of the buffer solution may be preferably 6 or more and 8 or less, still more preferably 7.0 or more and 7.8 or less.

Further, for improving the solubility of the analyte, the analyte may be dissolved in a solvent such as dimethylsulfoxide (DMSO) or ethanol, followed by mixing the solution with a buffer solution together with the biological sample.

As the biological sample in the method of the present invention, a microsomal fraction, blood, plasma and serum can be preferably used, and among them, plasma or serum can be more preferably used, and serum can be still more preferably used. When serum is used as the biological sample, adsorption of the analyte to a device or the like can be reduced, and a high recovery ratio of the analyte can be achieved to suppress a decrease in concentration of the analyte.

Further, in the method of the present invention, it is preferable to use diluted serum when the biological sample is diluted with a buffer solution or the like and used. By using diluted serum, an initial concentration change can be easily known in measurement of the concentration in dynamic analysis. This enables further accuracy improvement and time shortening in measurement of the relative $f_u$ ratio on a compound having a high protein binding ratio, for which it is difficult to obtain a steady state in dialysis.

In the present invention, the initial concentration of the biological sample in the donor solution or the acceptor solution is not particularly limited, and it is desirable that the initial concentration be, for example, in the range of preferably 1% or more and 50% or less, more preferably 1% or more and 30% or less, still more preferably about 1% or more and 10% or less (volume ratio in the solution).

In the present invention, the initial concentration of the analyte in the donor solution or the acceptor solution is not particularly limited, and it is desirable that the initial concentration be, for example, in the range of preferably 0.1 ($\mu$mol/L) or more and 10 ($\mu$mol/L) or less, still more preferably 0.1 ($\mu$mol/L) or more and 1 ($\mu$mol/L) or less, more preferably 0.1 ($\mu$mol/L) or more and 0.5 ($\mu$mol/L) or less.

After the donor solution and the acceptor solution are added into the chambers, the added sample and substance may be diffused by stirring or allowed to freely diffuse in the chambers. Preferably, dialysis can be performed while the solution in the chambers is stirred. The stirring can be performed at a rotation speed of preferably 20 rpm or more and 800 rpm or less, more preferably 250 rpm or more and 800 rpm or less.

The temperature of the solution or the like in the chambers in dialysis reaction is not particularly limited as long as the properties of the biological sample and the analyte are not adversely affected, and for example the temperature may be in the range of preferably 0°C or higher and 40°C or lower, more preferably 20°C or higher and 40°C or lower, still more preferably in the range of 35°C or higher and 40°C or lower.

In the present invention, the equilibrium dialysis can be performed using a known apparatus. For example, the equilibrium dialysis can be performed using a rapid equilibrium dialysis device or the like.

In the present invention, the concentrations of the analyte in the donor solution and the acceptor solution are measured over time. The concentration can be measured continuously, or on an every-fixed-time basis or an every-key-time basis. In measurement on an every-fixed-time basis, for example, the solution in each chamber can be injected to obtain samples, followed by measurement by a known method. For example, the concentration of the analyte in the obtained sample can be measured by a method such as liquid chromatography mass spectrometry (LC-MS/MS), or liquid chromatography using a UV detector.

In the present invention, the measurement period in measurement over time depends on a biological sample and an analyte to be used, and is not particularly limited, and for example, appropriate measurement can be performed for a period of preferably 72 hours, more preferably 36 hours, still more preferably about 24 hours from the initial stage. When measurement is performed on an every-fixed-time basis or an every-key-time basis, for example, the number of the measurements may be preferably 20 or less, more preferably 10 or less, still more preferably 5 or less. By measuring the concentration over time over such a time range to perform dynamic analysis, the relative $f_u$ ratio can be accurately determined even if an equilibrium state is not reached.

In the present invention, the relative $f_u$ ratio can be calculated using the time-dependent data of the measured concentrations of the analyte in the donor solution and the acceptor solution. The relative $f_u$ ratio can be determined using known software for performing dynamic analysis. As such dynamic analysis software, for example, Simbiology/MATLAB R2019b (manufactured by The Math Works, Inc.), Microsoft Excel 2019(manufactured by Microsoft Corporation), or the like can be used.

Hereinafter, the dynamic analysis method in the present invention will be described. The present invention provides a novel assay technique for directly detecting a relative fraction unbound-to-protein ratio (relative $f_u$ ratio) of an analyte between different biological samples, where best conditions are set for detecting a relative $f_u$ ratio for a compound having a high protein binding ratio by performing (i) evaluation on a change in concentration of the analyte by rapid equilibrium dialysis between different biological samples and (ii) dynamic (kinetics) analysis.

In the dynamic analysis according to the present invention, the measured values of the concentrations of the analyte in the solutions (e.g. 0.5-hour value, 1-hour value, 2-hour value, 4-hour value, 16-hour value and 24-hour value) which are obtained by equilibrium dialysis, and intrinsic parameters are applied to the following expression 1 (change in amount of the analyte in the donor solution), the following expression 2 (change in amount of the analyte in the acceptor solution) and the following expression 3 (change in amount per unit time of the analyte of an adsorbed fraction in the donor-side chamber), convergence calculation (fitting) is performed to determine optimum solutions of $f_u$1 and $f_u$2, and the ratio of

$f_u1/f_u2$ obtained is taken as a relative $f_u$ ratio.

For an analyte which is adsorbed to the device, adsorption clearance is set as a parameter in mass balance equation and fitting accuracy is improved by a mechanism-based model in (ii) dynamic analysis. When the analyte is a compound having a high protein binding ratio (i.e. a compound having a low $f_u$ value), the accuracy of a $f_u$ value obtained by analysis is often viewed with suspicion, but in the present invention, by performing (iii) bidirectional evaluation in which the measurement method (a) is combined with the following measurement method (b), i.e. comparing the relative $f_u$ ratio determined by the above-described method with a relative $f_u$ ratio determined by the same method except that the biological samples added to the donor solution and the acceptor solution are reversed, if necessary, mathematical validation is made possible, so that the accuracy of the resulting relative $f_u$ ratio can be further improved. Further, by using (iv) diluted serum if necessary, conditions for detection of an initial concentration change can be modified to further improve the accuracy of the resulting relative $f_u$ ratio.

Specifically, the software for performing dynamic analysis can be used, where data of a change in concentration, which is obtained by the following method, is input, and the parameters and the $f_u$ ratio are calculated under the following conditions and by the non-linear least-squares method (lsqnonlin).

(Expression 1):

$$V1\frac{dC1}{dt} = fu2 * C2 * PS - fu1 * C1 * PS - fu1 * C1 * CLad + koff * Xad$$

(Expression 2):

$$V2\frac{dC2}{dt} = -fu2 * C2 * PS + fu1 * C1 * PS$$

(Expression 3):

$$\frac{dXad}{dt} = fu1 * C1 * CLad - koff * Xad$$

Expression 1 represents a change in amount (change in amount per unit time) of the analyte in the donor solution. That is, expression 1 represents a change in amount of the analyte on the donor side, which includes reversed transfer of the analyte from the acceptor side to the donor side (fu2*C2*PS), transfer of the analyte from the donor side to the acceptor side (fu1*C1*PS), and effects of adsorption/ desorption to/from a device wall on the donor side (adsorption of the analyte to the device wall (fu1*C1*CLad) and desorption of the adsorbed analyte from the device wall (koff*Xad)).

Expression 2 represents a change in amount (change in amount per unit time) of the analyte in the acceptor solution. That is, expression 2 represents a change in amount of the analyte on the acceptor side, which includes reversed transfer of the analyte from the acceptor side to the donor side (fu2*C2*PS), transfer of the analyte from the donor side to the acceptor side (fu1*C1*PS) (the effect of adsorption is not included).

Expression 3 represents a change in amount per unit time of the analyte of the adsorbed fraction in the donor-side chamber (adsorption/desorption of the analyte to/from the device wall per unit time). That is, expression 3 represents a change in amount of adsorption of the analyte in the donor-side, which includes adsorption of the analyte to the device wall on the donor side (fu1*C1*CLad) and desorption of the adsorbed analyte from the device wall (koff*Xad).

The concentrations of the analyte in the solutions measured over time (measured value), and the intrinsic parameters are applied to these expressions to perform convergence calculation (fitting) (parameters related to adsorption/desorption to/from the device wall (CLad, Koff and Xad) are calculated from the concentration by convergence calculation using a computer, and incorporated), whereby the relative $f_u$ ratio ($f_u1/f_u2$) can be obtained.

Here, the parameters have the following meanings.

V1: Volume of donor solution (fixed value, $3.5 \times 10^{-4}$ L)
V2: Volume of acceptor solution (fixed value, $2.0 \times 10^{-4}$ L)
fu1: $f_u$ value of analyte with respect to biological sample contained in donor solution (calculated value)
fu2: $f_u$ value of analyte with respect to biological sample contained in acceptor solution (calculated value)
C1: Concentration of analyte in donor solution (total concentration: total of bound form analyte and unbound form analyte, except for analyte adsorbed to equilibrium dialysis device) (measured value, nmol/L)
C2: Concentration of analyte in acceptor solution (total concentration: total of bound form and unbound form analytes) (measured value, nmol/L)
PS: Dialysis membrane permeation clearance of analyte (fixed value, $1.0 \times 10^{-4}$ L/h or $1.0 \times 10^{-5}$ L/h)
CLad: Adsorption clearance of analyte in donor-side chamber (variable value, L/h)
koff: Dissociation rate constant of analyte in donor-side chamber (variable value, $h^{-1}$)
Xad: Amount of adsorption of analyte in donor-side chamber (variable value, nmol)

Of the numbers written in the symbols, "1" means being obtained on the donor side, and "2" means being obtained on the acceptor side.
In the equilibrium dialysis device used in Examples of the present application, the wall surface area on the donor side is much larger than that on the acceptor side, and therefore the analyte adsorbed to the equilibrium dialysis device is considered only in C1. Even in CLad, Koff and Xad, the analyte in the donor-side chamber is considered, and when an equilibrium dialysis device is used in which the wall surface area on the donor side is close to the wall surface area on the acceptor side to some extent, a change in amount per unit time of the analyte of an adsorbed fraction in the acceptor-side chamber can be additionally established as the following expression 3' to perform dynamic analysis.

(Expression 3')

$$\frac{dXad'}{dt} = fu2 * C2 * \mathrm{CLad}' - \mathrm{koff}' * \mathrm{Xad}'$$

CLad': Adsorption clearance of analyte in acceptor-side chamber (variable value, L/h)
koff': Dissociation rate constant of analyte in acceptor-side chamber (variable value, $h^{-1}$)
Xad': Amount of adsorption of analyte in acceptor-side chamber (variable value, nmol)

In this case, the following expression 2' is used in place of expression 2.

(Expression 2')

$$V2\frac{dC2}{dt} = -fu2 * C2 * PS + fu1 * C1 * PS - fu2 * C2 * \mathrm{CLad}' + \mathrm{koff}' * \mathrm{Xad}'$$

In addition to the above-described analysis method, a method can be used in which PS is not fixed to a specific value, convergence calculation (fitting) is performed with each of (fu1*PS) and (fu2*PS) taken as a single variation parameter, and the relative $f_u$ ratio ($f_u1/f_u2$) is obtained from a ratio of (fu1*PS) to (fu2*PS).
In the convergence calculation (fitting), the relative $f_u$ ratio can be calculated using a set of measurement results obtained while the biological samples contained in the donor solution and the acceptor solution are reversed. Default settings for Fit Data (SimBiology Model Analyzer Program) can be appropriately changed, and for example, the calculation can be performed with the following conditions (model, reaction, rules, definition, estimated parameters) described in the default settings. The model in performing analysis is shown in Figure 3.

Reaction:

Eq1.Donor1<-> Eq1.Acceptor1: +fu1*PS*Eq1.Donorl/Vd1-fu2*PS*Eq1.Acceptor1/Vd2 Eq1.Donor1-> Eq1.Ad: +CL1*fu1*Eq1.Donor1/Vd1-(Koff1*Eq1.Ad)
Eq2.Donor2<-> Eq2.Acceptor2: +fu2*PS*Eq2.Donor2/Vd1-fu1*PS*Eq2.Acceptor2/Vd2 Eq2.Donor2-> Eq2.Ad: +CL2*fu2*Eq2.Donor2/Vd1-(Koff2*Eq2.Ad)

Rules:

rule_1 Eq1.Ca1 = Eq1.Acceptor1/Vd2 repeatedAssignment
rule_2 Eq1.Cd1 = Eq1.Donor1/Vd1 repeatedAssignment

rule_3 Eq2.Ca2 = Eq2.Acceptor2/Vd2 repeatedAssignment
rule_4 Eq2.Cd2 = Eq2.Donor2/Vd1 repeatedAssignment
rule_5 Eq1.Donor1 = Dose1 initialAssignment
rule_6 Eq2.Donor2 = Dose2 initialAssignment

Definition:

When the first biological sample is from a mouse, and the second biological sample is from a human, a set of the following four pieces of data is used for analysis.

Data 1 (Eq1.Ca1): Concentration of analyte in acceptor solution in equilibrium dialysis system with donor solution containing mouse serum and acceptor solution containing human serum
Data 2 (Eq1.Cd1): Concentration of analyte in donor solution in equilibrium dialysis system with donor solution containing mouse serum and acceptor solution containing human serum
Data 3 (Eq2.Ca2): Concentration of analyte in acceptor solution in equilibrium dialysis system with donor solution containing human serum and acceptor solution containing mouse serum
Data 4 (Eq2.Cd2): Concentration of analyte in donor solution in equilibrium dialysis system with donor solution containing human serum and acceptor solution containing mouse serum

Estimated Parameters:

exp(fu1), InitialValue = 1, Bounds = [0.01 100];
exp(fu2), InitialValue = 1, Bounds = [0.01 100];
exp(Koff1), InitialValue = 1, Bounds = [0.01 1000000];
exp(Koff2), InitialValue = 1, Bounds = [0.01 1000000];
exp(CL1), InitialValue = 0.001, Bounds = [1e-07 0.1];
exp(CL2), InitialValue = 0.001, Bounds = [1e-07 0.1];
exp(Dose1), InitialValue = 0.2115, Bounds = [0.1, 0.4 or 0.5];
exp(Dose2), InitialValue = 0.2114, Bounds = [0.1, 0.4 or 0.5];

Pooled fit, true

Method for measuring relative $f_u$ ratio of analyte between different biological samples (b)

The method for determining a relative fraction unbound ratio (relative $f_u$ ratio) of an analyte between different biological samples according to the present invention may further comprise a method comprising reversing the biological sample (A) and the biological sample (B) in the measurement method (a) comprising the steps (1) to (5). That is, the method for determining a relative fraction unbound ratio (relative $f_u$ ratio) of an analyte between different biological samples according to the present invention may further comprise the following steps (6) to (10):

(6) providing a chamber system (II) different from the chamber system (I), in which adjacent chambers are separated by a semipermeable membrane permeable to the analyte;
(7) adding an acceptor solution containing the biological sample (A) to one chamber (acceptor-side chamber) in the chamber system (II) (e.g. Figures 2a and 2f);
(8) adding a donor solution containing the biological sample (B) and the analyte to a chamber different from the acceptor-side chamber (donor-side chamber) in the chamber system (II) (e.g. Figures 2b and 2e);
(9) measuring concentrations of the analyte in the acceptor solution in the step (7) and the donor solution in the step (8) over time (e.g. Figures 2c, 2d, 2g and 2h); and
(10) calculating the relative $f_u$ ratio using data associated with the concentrations of the analyte which are measured in the step (9).

In the method, the order in which steps (7) and (8) are carried out is not particularly limited, and for example, step (8) may be carried out after step (7), step (7) may be carried out after step (8), or step (7) may be carried out simultaneously or in parallel with step (8) (e.g. Figures 2a, 2b, 2e and 2f).
Hereinafter, an example of the present invention will be described with reference to the drawings. Figure 2 (Figures 2a to 2d and 2e to 2h) show a dialysis chamber system II between assays. Figure 2a shows a state when an acceptor solution containing the first biological sample (A) containing non-specific protein 4 is added to the acceptor-side chamber 20 (black arrow), where the non-specific protein 4 exists in the acceptor solution in the acceptor-side chamber 20. Figure 2b shows a state when a donor solution containing the second biological sample (B) containing non-specific protein 5, and an analyte 3 is added to the donor-side chamber 10 (black arrow), where the analyte 3 and the non-specific protein 5 exist in the donor solution in the donor-side chamber 10. Figure 2c shows the situation of an initial stage in which transfer of the analyte from the donor-side chamber 10 to the acceptor-side chamber 20 starts. Figure 2d shows the situation of a stage in which the analyte is transferred from the donor-side chamber 10 to the acceptor-side chamber 20, or optionally transferred from the acceptor-side chamber 20 to the donor-side chamber 10 reversely, so that dialysis proceeds to equilibrium. The concentration of the analyte in the solution added to each chamber is measured over time from the initial stage.

Figures 2e to 2h show an example of a schematic diagram of a situation in which the order of the operations of Figures 2a and 2b is reversed.

Steps (6) to (10) can be carried out in the same method as in the steps (1) to (5). That is, the relative $f_u$ ratio can be calculated using the time-dependent data of the concentrations of the analyte in the donor solution and the acceptor solution, which are measured in step (9) by way of steps (6) to (8) (step (10)).

In the present invention, the time-dependent change in concentration of the analyte in each of the solutions added to the two chambers in steps (1) to (4) can be introduced at a time into calculation expressions in dynamic analysis software incorporating predetermined parameters, and subjected to fitting processing to determine a relative $f_u$ ratio between different species. The time-dependent change in concentration of the analyte in each of the solutions added to the two chambers in steps (6) to (9) can be introduced at a time into calculation expressions in dynamic analysis software incorporating predetermined parameters, and subjected to fitting processing to determine a relative $f_u$ ratio between different species. The relative $f_u$ ratio obtained from steps (1) to (5) (hereinafter, sometimes referred to as a "relative $f_u$ ratio (a)") and the relative $f_u$ ratio obtained from steps (6) to (10) (hereinafter, sometimes referred to as a "relative $f_u$ ratio (b)") are theoretically the same value because the same biological sample and the same analyte are used.

Therefore, by comparing the relative $f_u$ ratios obtained each independently from steps (1) to (5) and steps (6) to (10) (hereinafter, sometimes referred to as a "relative $f_u$ ratio (a)" and a "relative $f_u$ ratio (b)", respectively), each of the relative $f_u$ values can be mathematically validated. For example, when the relative $f_u$ ratio (a)/relative $f_u$ ratio (b) is preferably 0.8 or more and 1.2 or less, more preferably 0.9 or more and 1.1 or less, it can be evaluated that the accuracy of each relative $f_u$ ratio is high.

Further, the time-dependent change in concentration of the analyte in each of the solutions added to the four chambers in steps (1) to (4) and steps (6) to (9), i.e. data associated with the concentrations of the analyte which are measured in step (4) and step (9), can be introduced at a time into calculation expressions in dynamic analysis software incorporating predetermined parameters, and subjected to fitting processing to determine a relative $f_u$ ratio between different species. By such a method, the accuracy of the relative $f_u$ ratio between different species can be further improved. As described in Examples, the relative $f_u$ value determined by the method of the present invention is well consistent with the ratio of $f_u$ values (absolute values) determined by the method in Reference Example.

According to the present invention, relative $f_u$ ratios between biological samples of a plurality of species, e.g. 2 to 5 species can be evaluated at once, so that comparison of the obtained relative $f_u$ ratios between species can be made easier as compared to, for example, a method in which a $f_u$ value (absolute value) is calculated from a biological sample of one species. For example, when the relative $f_u$ ratio between two biological samples is determined, two chambers separated by an equilibrium dialysis membrane are provided, solutions containing biological samples of interest are added to both chambers, respectively, and measurement is performed. When relative $f_u$ value ratios between three or more biological samples are evaluated at once, the same number of chambers as the number of biological samples are provided, and separated by an equilibrium dialysis membrane permeable to an analyte so that the analyte can freely diffuse, solutions containing biological samples of interest are added to the chambers, respectively, and measurement may be performed, or two chambers separated by an equilibrium dialysis membrane are provided, and solutions containing one or more biological samples are added to both chambers, respectively, and measurement may be performed.

In the present invention, a value is obtained as a relative $f_u$ ratio to two or more biological samples, and therefore as compared to a method in which small $f_u$ values susceptible to error are individually obtained, error can be minimized, so that it is possible to perform more accurate comparison $f_u$ ratios between species. Further, by setting a reference biological sample, e.g. reference serum, subsequent experiment-specific validation can be easily performed.

Further, in the present invention, the employment of dynamic analysis enables the relative $f_u$ ratio to be measured in a short time as a matter of course, and consequently, the impact of degradation of the biological sample over time (time-dependent change in pH, variation in solution amount and the like) is minimized, so that it is possible to measure the relative $f_u$ ratio more accurately. Further, even if the concentration of the analyte in the solution hardly reaches an equilibrium state, a relative $f_u$ ratio having a good correlation with a $f_u$ ratio measured after the concentration reaches an equilibrium state can be obtained without reaching an equilibrium state when dynamic analysis is applied. Therefore, the dynamic analysis is particularly effective when the analyte has a high protein binding ratio, etc.

When a $f_u$ value is provided from a biological sample of one species by dynamic analysis, an average value of Pmem in a group of compounds having known $f_u$ is used, and the present invention enables the analysis to be performed even without obtaining an experimental numerical value for the semipermeable membrane permeation rate (Pmem [m/s]). For example, even if the compound has a large molecular weight like cyclosporine (molecular weight: 1202.61), a relative $f_u$ ratio can be acquired by setting Pmem as an arbitrary characteristic value in analysis.

<u>Methods for predicting clearance and distribution volume of drug</u>

The method for predicting clearance of a drug according to the present invention includes a method comprising using the relative $f_u$ ratio and data of clearance of the drug in a species from which one biological sample is derived, to predict clearance of the drug in a species from which another biological sample is derived.

The "clearance" (CL) is defined as a drug disappearance rate (v) divided by a concentration (C) at this time (CL = v/C), and is expressed as a removable volume of the drug per unit time (mL/min).

As the method for predicting clearance using the $f_u$ ratio obtained by the method of the present invention, a known method can be used. For example, human clearance can be predicted by applying a $f_u$ ratio and measured clearance data (e.g. mouse, rat, dog or monkey) of a drug to a known method for estimating human clearance (FCIM:fu-corrected intercept method (Non Patent Literature 6: Tang, H. and M. Mayersohn (2005). "A novel model for prediction of human drug clearance by allometric scaling." Drug Metab Dispos 33(9): 1297-1303.)).

For example, the prediction method in the FCIM method is as follows.

- Clearance of a plurality of animal species are applied to the following expression of SA (Simple Allometry) to optimize a and b;

$$CL = a(BodyWeight)^b$$

(a: scaling coefficient, b: scaling index)
- Predicted human clearance is calculated by the following expression from a and $Rf_u$ (relative $f_u$ ratio (rat/human)) obtained as described above;

$$CL_{human} = 33.35 \times (a/Rf_u)^{0.77}$$

The method for predicting distribution volume of a drug according to the present invention includes a method comprising using the relative $f_u$ ratio and data of distribution volume of the drug in a species from which one biological sample is derived, to predict distribution volume of the drug in a species from which another biological sample is derived.

The "distribution volume" (V) means a theoretical amount of liquid required for the total amount of an administered drug to be contained in the body at a concentration equal to that in the blood or plasma, and is expressed by distribution volume = amount of drug in the body/blood concentration (e.g. L).

As the method for predicting distribution volume using the $f_u$ ratio obtained by the method described above, a known method can be used with modification applied if necessary. For example, desired distribution volume (e.g. human) can be predicted by applying a $f_u$ ratio and steady state distribution volume of the drug (e.g. mouse, rat, dog or monkey) to a known method for estimating a difference in distribution volume between species (Non Patent Literature 7: Berry, L. M., et al. (2011). "Species differences in distribution and prediction of human V(ss) from preclinical data." Drug Metab Dispos 39(11): 2103-2116.).

Examples of the method for predicting distribution volume include the following method for estimating distribution volume corrected with $f_u$ (Non Patent Literature 7).

Steady state distribution volume (Vss, [L])
Steady state distribution volume corrected with $f_u$ (Vss u, [L] = Vss/fu, [L])
SA (Simple Allometry):

$$Vss\ u = a(BodyWeight)^b$$

(a: scaling coefficient, b: scaling index)
Here, the expression of SA can be altered to the following expression, where fu,ref is $f_u$ for an animal species as a reference, and Rfu (= fu/fu,ref) is a $f_u$ ratio with respect to the animal species as a reference.

$$Vss/Rfu = fu,ref \times a(BodyWeight)^b$$

By optimizing fu,ref x a and b using measured Vss and Rfu for a plurality of animal species, Vss/Rfu for an arbitrary species can be predicted, and Vss can be predicted using Rfu. In the modified prediction method, it is not necessary to measure the absolute value of $f_u$ for any of the species.

All references cited herein are incorporated herein by reference.

[Examples]

Hereinafter, suitable specific aspects of the present invention are described by way of Examples, but the present invention is not limited thereto.

[Example 1]

Acquisition of relative $f_u$ ratio between different species by dynamic analysis (analyte: nifedipine)

[Preparation of donor solution]

First, solutions of nifedipine as an analyte at 10 mmol/L and 1 mmol/L were prepared. Specifically, 0.482 mL of dimethylsulfoxide (DMSO) was added to 1.67 mg of powdered nifedipine to obtain a solution of nifedipine at 10 mmol/L. Subsequently, 180 µL of DMSO was added to 20 µL of a solution of nifedipine at 10 mmol/L to obtain a solution of nifedipine at 1 mmol/L.

Next, serums of various species (mouse serum, rat serum, dog serum, monkey serum and human serum) were fused. The serums of various species which had been fused were collected, and to each of the serums were added 9 times its volume of phosphate buffer physiological saline and 1/1000 times its volume of the solution of nifedipine at 1 mmol/L to prepare a donor solution having a serum concentration of 10% (volume ratio), a nifedipine concentration of 1 µmol/L and a DMSO concentration of 0.1% (volume ratio). Table 1 below shows the specific amounts of the samples used.

The samples used are as follows:

Nifedipine: "Nifedipine" manufactured by FUJIFILM Wako Pure Chemical Corporation, molecular weight: 346.335, CLogP: 3.12
DMSO: "CultureSure (R) DMSO" manufactured by FUJIFILM Wako Pure Chemical Corporation
Mouse serum: "Mouse (CD-1 (ICR)) Serum" manufactured by Valley Biomedical Products & Services, Inc.
Rat serum: "Rat (SD (Crl:CD)) Serum" manufactured by Valley Biomedical Products & Services, Inc.
Dog serum: "Beagle Pool Serum" manufactured by Japan SLC, Inc.
Monkey serum: "Monkey (Cynomolgus) Serum" manufactured by Valley Biomedical Products & Services, Inc.
Human serum: "Human True A serum, pool of donors" manufactured by BIOPREDIC International
Phosphate buffer physiological saline (PBS): "Dulbecco Phosphate Buffer Physiological Saline (free of Ca and Mg)" manufactured by nacalai tesque

[Table 1]

| Species from which serum is derived | Serum (mL) | Phosphate buffer physiological saline (mL) | Solution of nifedipine at 1 mmol/L (µL) |
|---|---|---|---|
| Mouse, rat, dog, monkey | 1 | 9 | 10 |
| Human | 4 | 36 | 40 |

[Preparation of acceptor solution]

Except that a DMSO solution was added instead of a solution of nifedipine at 1 mmol/L, the same procedure as that for the donor solution was carried out to prepare an acceptor solution having a serum concentration of 10% (volume ratio) and a DMSO concentration of 0.1% (volume ratio).

[Dialysis reaction]

First, a rapid equilibrium dialysis device ("RED (Rapid Equilibrium Dialysis) Device", Dialysis Membrane MWCO: 8 kDa manufactured by Thermo Fisher Scientific) was provided. 350 µL of a relevant donor solution was injected into each white chamber, and 200 µL of a relevant acceptor solution was injected into each red chamber. Table 2 below shows the donor solutions or acceptor solutions injected into the chambers.

In Table 2, Table 2-1 shows a case where serums of a human and a mouse were used. Measurement was performed at the time points of 0.5 hours, 2 hours, 4 hours, 16 hours and 24 hours for each of a total of six patterns where human serum was set on the donor side and mouse serum was set on the acceptor side (tests A to C: the same conditions) and where the mouse serum was set on the donor side and the human serum was set on the acceptor side (tests D to F: the same conditions). Table 2-2 shows a case where serums of a human and a rat were used, Table 2-3 shows a case where serums of a human and a dog were used, and Table 2-4 shows a case where serums of a human and a monkey were used, where measurement was performed at the time points 0.5 hours, 2 hours, 4 hours, 16 hours and 24 hours for the same six patterns as in the case of the above-described combination of a human and a mouse.

Next, the upper part of the RED device was hermetically sealed with an aluminum seal ("3M (R) Microplate Sealing Tape" manufactured by 3M Japan Limited), and using Shaker ("MB100-4AThermo-shaker", manufactured by Hangzhou Allsheng Instruments Co., Ltd.), a dialysis reaction was started (set value: stirring speed rotation 800 rpm, 37°C).

(Table 2) Chamber pattern using serums of human/mouse, rat, dog and monkey

(Table 2-1) Human/mouse

| Red device | 1 | 2 | 3 | 4 | 5 | 6 | 7 | 8 | 9 | 10 |
|---|---|---|---|---|---|---|---|---|---|---|
| Elapsed time | 0.5h | | 2h | | 4h | | 16h | | 24h | |
| Chamber | **Donor** | **Acceptor** | **Donor** | **Acceptor** | **Donor** | **Acceptor** | **Donor** | **Acceptor** | **Donor** | **Acceptor** |
| A | Human | Mouse | Human | Mouse | Human | Mouse | Human | Mouse | Human | Mouse |
| | 1 | 1 | 1 | 1 | 1 | 1 | 1 | 1 | 1 | 1 |
| B | Human | Mouse | Human | Mouse | Human | Mouse | Human | Mouse | Human | Mouse |
| | 2 | 2 | 2 | 2 | 2 | 2 | 2 | 2 | 2 | 2 |
| C | Human | Mouse | Human | Mouse | Human | Mouse | Human | Mouse | Human | Mouse |
| | 3 | 3 | 3 | 3 | 3 | 3 | 3 | 3 | 3 | 3 |
| D | Mouse | Human | Mouse | Human | Mouse | Human | Mouse | Human | Mouse | Human |
| | 1 | 1 | 1 | 1 | 1 | 1 | 1 | 1 | 1 | 1 |
| E | Mouse | Human | Mouse | Human | Mouse | Human | Mouse | Human | Mouse | Human |
| | 2 | 2 | 2 | 2 | 2 | 2 | 2 | 2 | 2 | 2 |
| F | Mouse | Human | Mouse | Human | Mouse | Human | Mouse | Human | Mouse | Human |
| | 3 | 3 | 3 | 3 | 3 | 3 | 3 | 3 | 3 | 3 |

(Table 2-2) Human/rat

| Red device | 1 | 2 | 3 | 4 | 5 | 6 | 7 | 8 | 9 | 10 |
|---|---|---|---|---|---|---|---|---|---|---|
| Elapsed time | 0.5h | | 2h | | 4h | | 16h | | 24h | |
| Chamber | **Donor** | **Acceptor** | **Donor** | **Acceptor** | **Donor** | **Acceptor** | **Donor** | **Acceptor** | **Donor** | **Acceptor** |
| A | Human | Rat | Human | Rat | Human | Rat | Human | Rat | Human | Rat |
| | 1 | 1 | 1 | 1 | 1 | 1 | 1 | 1 | 1 | 1 |
| B | Human | Rat | Human | Rat | Human | Rat | Human | Rat | Human | Rat |
| | 2 | 2 | 2 | 2 | 2 | 2 | 2 | 2 | 2 | 2 |
| C | Human | Rat | Human | Rat | Human | Rat | Human | Rat | Human | Rat |
| | 3 | 3 | 3 | 3 | 3 | 3 | 3 | 3 | 3 | 3 |
| D | Rat | Human | Rat | Human | Rat | Human | Rat | Human | Rat | Human |
| | 1 | 1 | 1 | 1 | 1 | 1 | 1 | 1 | 1 | 1 |
| E | Rat | Human | Rat | Human | Rat | Human | Rat | Human | Rat | Human |
| | 2 | 2 | 2 | 2 | 2 | 2 | 2 | 2 | 2 | 2 |
| F | Rat | Human | Rat | Human | Rat | Human | Rat | Human | Rat | Human |
| | 3 | 3 | 3 | 3 | 3 | 3 | 3 | 3 | 3 | 3 |

(Table 2-3) Human/dog

| Red device | 1 | 1 2 | 3 | 4 | 5 | 6 | 7 | 8 | 9 | 10 |
|---|---|---|---|---|---|---|---|---|---|---|
| Elapsed time | 0.5h | | 2h | | 4h | | 16h | | 24h | |
| Chamber | **Donor** | **Acceptor** | **Donor** | **Acceptor** | **Donor** | **Acceptor** | **Donor** | **Acceptor** | **Donor** | **Acceptor** |
| A | Human | Dog | Human | Dog | Human | Dog | Human | Dog | Human | Dog |
| | 1 | 1 | 1 | 1 | 1 | 1 | 1 | 1 | 1 | 1 |
| B | Human | Dog | Human | Dog | Human | Dog | Human | Dog | Human | Dog |
| | 2 | 2 | 2 | 2 | 2 | 2 | 2 | 2 | 2 | 2 |
| C | Human | Dog | Human | Dog | Human | Dog | Human | Dog | Human | Dog |
| | 3 | 3 | 3 | 3 | 3 | 3 | 3 | 3 | 3 | 3 |
| D | Dog | Human | Dog | Human | Dog | Human | Dog | Human | Dog | Human |
| | 1 | 1 | 1 | 1 | 1 | 1 | 1 | 1 | 1 | 1 |
| E | Dog | Human | Dog | Human | Dog | Human | Dog | Human | Dog | Human |
| | 2 | 2 | 2 | 2 | 2 | 2 | 2 | 2 | 2 | 2 |
| F | Dog | Human | Dog | Human | Dog | Human | Dog | Human | Dog | Human |
| | 3 | 3 | 3 | 3 | 3 | 3 | 3 | 3 | 3 | 3 |

(Table 2-4) Human/monkey

| Red device | 1 | 2 | 3 | 4 | 5 | 6 | 7 | 8 | 9 | 10 |
|---|---|---|---|---|---|---|---|---|---|---|
| Elapsed time | 0.5h | | 2h | | 4h | | 16h | | 24h | |
| Chamber | **Donor** | **Acceptor** | **Donor** | **Acceptor** | **Donor** | **Acceptor** | **Donor** | **Acceptor** | **Donor** | **Acceptor** |
| A | Human | Monkey | Human | Monkey | Human | Monkey | Human | Monkey | Human | Monkey |
| | 1 | 1 | 1 | 1 | 1 | 1 | 1 | 1 | 1 | 1 |
| B | Human | Monkey | Human | Monkey | Human | Monkey | Human | Monkey | Human | Monkey |
| | 2 | 2 | 2 | 2 | 2 | 2 | 2 | 2 | 2 | 2 |
| C | Human | Monkey | Human | Monkey | Human | Monkey | Human | Monkey | Human | Monkey |
| | 3 | 3 | 3 | 3 | 3 | 3 | 3 | 3 | 3 | 3 |
| D | Monkey | Human | Monkey | Human | Monkey | Human | Monkey | Human | Monkey | Human |
| | 1 | 1 | 1 | 1 | 1 | 1 | 1 | 1 | 1 | 1 |
| E | Monkey | Human | Monkey | Human | Monkey | Human | Monkey | Human | Monkey | Human |
| | 2 | 2 | 2 | 2 | 2 | 2 | 2 | 2 | 2 | 2 |
| F | Monkey | Human | Monkey | Human | Monkey | Human | Monkey | Human | Monkey | Human |
| | 3 | 3 | 3 | 3 | 3 | 3 | 3 | 3 | 3 | 3 |

[Sampling after dialysis reaction]

At the time points 0.5 hours, 2 hours, 4 hours, 16 hours and 24 hours after the start of the dialysis reaction, 20 μL of each of the chambers injected with the donor solutions and acceptor solutions were collected, and mixed with 180 μL of a quench solution 1 (methanol/acetonitrile/DMSO = 85/85/10 in terms of volume ratio), and the mixture was hermetically sealed. The method for preparing the quench solution 1 is as follows [80 μL of an indomethacin solution (concentration: 200 μg/mL) was added to 80 mL of a solution of methanol/acetonitrile = 1/1; and 3 mL of DMSO was added to 51 mL of the prepared mixed solution to obtain the quench solution 1]. The donor solution before injection into the RED device was taken as a 0-hour value sample, and collected in the same amount as described above, and was mixed with the quench solution 1 in the same method as described above, and the mixture was hermetically sealed. Each sample was stored at 4°C before being subjected to pretreatment.

[Pretreatment of sample from sampling]

The sample after sampling was each transferred in a total amount to a filter plate ("Multi Screen (R) Solvinert, Filter Plates 0.45μm, Low-Binding Hydrophilic PTFE" manufactured by Millipore Corporation), 96-well PP microplates were superposed on the lower side, and the sample was filtered by a centrifuge ("CF10RXII" manufactured by Hitachi, Ltd.) (3000 rpm, 5 min). The sample after the filtration was hermetically sealed by seal bonding to obtain a LC-MS/MS measurement sample.

[Measurement of concentration of analyte by LC-MS/MS]

For each of the LC-MS/MS measurement samples, a nifedipine concentration in the solution added to each chamber was measured using the following measuring equipment and measurement conditions. When the concentration of the analyte was measured in biotic samples of the same type (e.g. serum) in a plurality of wells in Examples, Comparative Examples or Reference Examples in the present specification, an average value of concentrations measured in a group of biotic samples of the same type (e.g. serum) in the wells was used for calculation of the $f_u$ ratio. For example, in this Example, an average value of concentrations of the analyte in the same serum group in tests A to C, and an average value of concentrations of the analyte in the same serum group in tests D to F were used for calculation of the $f_u$ ratio.

(Measuring equipment)

LC: Waters Acquity UPLC
MS: Xevo TQ-S (Waters Corporation, Milford, Massachusetts)
Column: YMC-Triart C18 column, column size: 2.0 x 30 mm, particle size: 1.9 μm, pore size: 12 nm

(Measurement conditions)

Column temperature: 50°C (set value)
Solvent:

A) 0.1% aqueous formic acid solution
B) 0.1% acetonitrile formate solution

Gradient conditions:

[Table 3]

| Time (min) | A% | B % |
|------------|-----|-----|
| 0.0 | 99 | 1 |
| 0.8 | 1 | 99 |
| 1.6 | 1 | 99 |
| 1.61 | 99 | 1 |

(continued)

| Time (min) | A% | B % |
|------------|-----|-----|
| 2.0 | 99 | 1 |

Flow rate: 0.6 mL/min
Autosampler temperature: 10°C (set value)
Injection volume: 1 μL

Preparation of calibration curve sample: 90 μL of DMSO was added to 10 μL of a solution of nifedipine at 1 mmol/L dissolved in DMSO to obtain a solution of nifedipine at 100 μmol/L, and the following series of dilutions (1) to (8) and BLANK solution were prepared from the solution of nifedipine at 100 μmol/L.

[(1) 376 μL of DMSO was added to 24 μL of a solution of nifedipine at 100 μmol/L to obtain a solution of nifedipine at 6000 nmol/L.
(2) 240 μL of DMSO was added to 120 μL of a solution of nifedipine at 6000 nmol/L to obtain a solution of nifedipine at 2000 nmol/L.
(3) 280 μL of DMSO was added to 120 μL of a solution of nifedipine at 2000 nmol/L to obtain a solution of nifedipine at 600 nmol/L.
(4) 240 μL of DMSO was added to 120 μL of a solution of nifedipine at 600 nmol/L to obtain a solution of nifedipine at 200 nmol/L.
(5) 280 μL of DMSO was added to 120 μL of a solution of nifedipine at 200 nmol/L to obtain a solution of nifedipine at 60 nmol/L.
(6) 240 μL of DMSO was added to 120 μL of a solution of nifedipine at 60 nmol/L to obtain a solution of nifedipine at 20 nmol/L.
(7) 280 μL of DMSO was added to 120 μL of a solution of nifedipine at 20 nmol/L to obtain a solution of nifedipine at 6 nmol/L.
(8) 240 μL of DMSO was added to 120 μL of a solution of nifedipine at 6 nmol/L to obtain a solution of nifedipine at 2 nmol/L. 200 μL of DMSO was used as a BLANK solution.]

To 10 μL of each of the series of dilutions (1) to (8) and the BLANK solution, 20 μL of a matrix solution (animal or human serum/PBS/DMSO = 10/90/0.1, volume ratio) and 170 μL of a quench solution 2 [a solution obtained by adding 80 μL of an indomethacin solution (concentration: 200 μg/mL) to 80 mL of a solution of methanol/acetonitrile = 1/1] were added to obtain a calibration curve sample. The calibration curve sample was pretreated by the same method as that for the sample from sampling. Since the series of dilutions (1) to (8) were each adjusted to a concentration 2 times that of an actual sample, the concentrations of the calibration curve samples were determined with a correction made in consistency with 1/2 times the concentration adjusted by the series of dilutions.

Concentrations of calibration curve samples: (8) 1 nmol/L, (7) 3 nmol/L, (6) 10 nmol/L, (5) 30 nmol/L, (4) 100 nmol/L, (3) 300 nmol/L, (2) 1000 nmol/L and (1) 3000 nmol/L

MS conditions (set values): ESI positive, Capillary (kV) 2.00, Cone (V) 25.00, Source Offset (V) 50.0, Source Temperature (°C) 150, Desolvation Temperature (°C) 600, Desolvation Gas Flow (L/Hr) 1000, Cone Gas Flow (L/Hr) 150, Collision Gas Flow (mL/Min) 0.20, Nebuliser Gas Flow (Bar) 7.00

[Table 4]

| Compound | Q1 (m/z) | Q3 (m/z) | Dwell (sec) | Cone. Volt (V) | Col. Energy (eV) |
|----------|----------|----------|-------------|----------------|------------------|
| Indomethacin (internal standard) | 359.24 | 139.17 | 0.221 | 30 | 20 |
| Nifedipine (analyte) | 347.02 | 314.92 | 0.1 | 10 | 5 |

[Acquisition of $f_u$ ratio by dynamic analysis]

Software for performing dynamic analysis ("SimBiology (R)/MATLAB, version information: 9.7.0.1190202 (R2019b)" manufactured by The MathWorks, Inc.) was used, where the obtained data of a change in concentration was input, and the parameters and the $f_u$ ratio were calculated under the following conditions and by the non-linear least-squares method (lsqnonlin). The set concentration of the analyte in the donor solution in preparation of the donor solution was used as a concentration of the analyte contained in the donor solution at the time point of 0 hour after the start of dialysis reaction.

(Expression 1):

$$V1\frac{dC1}{dt} = fu2 * C2 * PS - fu1 * C1 * PS - fu1 * C1 * CLad + koff * Xad$$

(Expression 2):

$$V2\frac{dC2}{dt} = -fu2 * C2 * PS + fu1 * C1 * PS$$

(Expression 3):

$$\frac{dXad}{dt} = fu1 * C1 * CLad - koff * Xad$$

Here, the parameters have the following meanings.

V1: Volume of donor solution (fixed value, $3.5 \times 10^{-4}$ L)
V2: Volume of acceptor solution (fixed value, $2.0 \times 10^{-4}$ L)
fu1: fu value of analyte with respect to biological sample contained in donor solution (calculated value)
fu2: fu value of analyte with respect to biological sample contained in acceptor solution (calculated value)
C1: Concentration of analyte in donor solution (total concentration: total of bound form and unbound form analytes, except for analyte adsorbed to equilibrium dialysis device) (measured value, nmol/L)
C2: Concentration of analyte in acceptor solution (total concentration: total of bound form and unbound form analytes) (measured value, nmol/L)
PS: Dialysis membrane permeation clearance of unbound form analyte (fixed value, $1.0 \times 10^{-4}$ L/h)
CLad: Adsorption clearance of analyte in donor-side chamber (variable value, L/h)
koff: Dissociation rate constant of analyte in donor-side chamber (variable value, $h^{-1}$)
Xad: Amount of adsorption of analyte in donor-side chamber (variable value, nmol)

Of the numbers written in the symbols, "1" means being obtained on the donor side, and "2" means being obtained on the acceptor side.

Convergence calculation (fitting)

In the dynamic analysis, the relative $f_u$ ratio was calculated using a set of measurement results obtained while the biological samples contained in the donor solution and the acceptor solution were reversed. The convergence calculation was performed with the following conditions (model, reaction, rules, definition, estimated parameters) described in default settings for Fit Data (SimBiology Model Analyzer, Program). The model in performing analysis is shown in Figure 3.

Reaction:

Eq1.Donor1<-> Eq1.Acceptor1: +fu1*PS*Eq1.Donor1/Vd1-fu2*PS*Eq1.Acceptor1/Vd2 Eq1.Donor1-> Eq1.Ad: +CL1*fu1*Eq1.Donor1/Vd1-(Koff1*Eq1.Ad)
Eq2.Donor2<-> Eq2.Acceptor2: +fu2*PS*Eq2.Donor2/Vd1-fu1*PS*Eq2.Acceptor2/Vd2 Eq2.Donor2-> Eq2.Ad: +CL2*fu2*Eq2.Donor2/Vd1-(Koff2*Eq2.Ad)

Rules:

rule_1 Eq1.Ca1 = Eq1.Acceptor1/Vd2 repeatedAssignment
rule_2 Eq1.Cd1 = Eq1.Donor1/Vd1 repeatedAssignment
rule_3 Eq2.Ca2 = Eq2.Acceptor2/Vd2 repeatedAssignment

rule_4 Eq2.Cd2 = Eq2.Donor2/Vd1 repeatedAssignment
rule_5 Eq1.Donor1 = Dose1 initialAssignment
rule_6 Eq2.Donor2 = Dose2 initialAssignment

Definition:

When the first biological sample was from a mouse, and the second biological sample was from a human, a set of the following four pieces of data was used for analysis.

Data 1 (Eq1.Ca1): Concentration of analyte in acceptor solution in equilibrium dialysis system with donor solution containing mouse serum and acceptor solution containing human serum
Data 2 (Eq1.Cd1): Concentration of analyte in donor solution in equilibrium dialysis system with donor solution containing mouse serum and acceptor solution containing human serum
Data 3 (Eq2.Ca2): Concentration of analyte in acceptor solution in equilibrium dialysis system with donor solution containing human serum and acceptor solution containing mouse serum
Data 4 (Eq2.Cd2): Concentration of analyte in donor solution in equilibrium dialysis system with donor solution containing human serum and acceptor solution containing mouse serum

Estimated Parameters:

$\exp(fu1)$, InitialValue = 1, Bounds = [0.01 100];
$\exp(fu2)$, InitialValue = 1, Bounds = [0.01 100];
$\exp(Koff1)$, InitialValue = 1, Bounds = [0.01 1000000];
$\exp(Koff2)$, InitialValue = 1, Bounds = [0.01 1000000];
$\exp(CL1)$, InitialValue = 0.001, Bounds = [1e-07 0.1];
$\exp(CL2)$, InitialValue = 0.001, Bounds = [1e-07 0.1];
$\exp(Dose1)$, InitialValue = 0.2115, Bounds = [0.1 0.4];
$\exp(Dose2)$, InitialValue = 0.2114, Bounds = [0.1 0.4];

Pooled fit, true

Table 5 shows the $f_u$ ratios of the analyte (nifedipine) with respect to various species (/human, serum concentration: 1/10, human/human is defined as 1.00) which were calculated by the above-described dynamic analysis method, and Figure 4 ((1) to (4)) shows plotted concentrations of the analyte in the solution added to each chamber at the time points of 0.5 hours, 2 hours, 4 hours, 16 hours and 24 hours after the start of dialysis reaction and fitting curves by dynamic analysis on those plots.

[Table 5]

|  | Relative $f_u$ ratio (/human) |
|---|---|
| Mouse | 1.37 |
| Rat | 0.06 |
| Dog | 1.40 |
| Monkey | 1.10 |
| Human | 1.00 |

[Comparative Example 1]

Acquisition of relative $f_u$ ratio between different species by static analysis (analyte: nifedipine)

A donor solution (10% mouse, rat, dog or monkey serum) and an acceptor solution (10% human serum) were prepared in the same manner as in Example 1, dialysis reaction was started, and the concentrations of an analyte in solutions added to the chambers at the time point of 24 hours after the start of the dialysis reaction were measured. The obtained data was applied to the following expression 4 to acquire $f_u$ ratios of nifedipine in serums of various species (mouse, rat, dog or monkey) (versus human, human/human was defined as 1.00). When the concentration of the analyte was measured in the same serum in a plurality of wells, an average value of concentrations measured in the wells for the same serum group was used for calculation of the $f_u$ ratio.

(Expression 4):

fu ratio (versus human)

$$= \frac{\text{nifedipine concentration in acceptor solution at time point of 24 hours (nM)}}{\text{nifedipine concentration in donor solution at time point of 24 hours (nM)}}$$

The obtained results are shown below.

[Table 6]

|  | $f_u$ ratio (/human) |
| --- | --- |
| Mouse | 1.25 |
| Rat | 0.12 |
| Dog | 1.29 |
| Monkey | 1.01 |
| Human | 1.00 |

[Reference Example 1]

Acquisition of $f_u$ ratio between different species by static analysis (versus buffer) (analyte: nifedipine)

[Preparation of donor solution]

A donor solution having a serum concentration of 10% (volume ratio), a nifedipine concentration of 1 μmol/L and a DMSO concentration of 0.1% (volume ratio) were prepared by the same method as in Example 1 except that the amounts of serums of various species (five species: mouse, rat, dog, monkey and human (A to E)), phosphate buffer physiological saline and solution of nifedipine at 1 mmol/L were set to the amounts shown in Table 7.

[Table 7]

| Species from which serum is derived | Serum (mL) | Phosphate buffer physiological saline (mL) | Solution of nifedipine at 1 mmol/L (μL) |
| --- | --- | --- | --- |
| Mouse, rat, dog, monkey, human | 0.2 | 1.8 | 2 |

[Preparation of acceptor solution]

To the phosphate buffer physiological saline (PBS) (20 mL) was added 1/1000 times its volume of a DMSO solution (20 μL) to prepare an acceptor solution (buffer) having a DMSO concentration of 0.1% (volume ratio).

[Dialysis reaction]

The dialysis reaction was carried out by the same method as in Example 1 except that the solutions injected into the chambers were set as shown in Table 8.

[Table 8]

|  | 1 | 2 | 3 | 4 | 5 | 6 |
|---|---|---|---|---|---|---|
| Elapsed time | 24h | | 24h | | 24h | |
| Chamber | **Donor** | **I Acceptor** | **Donor** | **I Acceptor** | **Donor** | **I Acceptor** |
| A | Mouse | PBS | Mouse | PBS | Mouse | PBS |
| | 1 | 1 | 2 | 2 | 3 | 3 |
| B | Rat | PBS | Rat | PBS | Rat | PBS |
| | 1 | 1 | 2 | 2 | 3 | 3 |
| C | Dog | PBS | Dog | PBS | Dog | PBS |
| | 1 | 1 | 2 | 2 | 3 | 3 |
| D | Monkey | PBS | Monkey | PBS | Monkey | PBS |
| | 1 | 1 | 2 | 2 | 3 | 3 |
| E | Human | PBS | Human | PBS | Human | PBS |
| | 1 | 1 | 2 | 2 | 3 | 3 |

[Sampling after dialysis reaction]

Sampling was performed by the same method as in Example 1 except that the time for sampling was set to only the time point of 24 hours after the start of dialysis reaction.

[Pretreatment of sample from sampling and measurement of concentration of analyte by LC-MS/MS]

Pretreatment of the sample after sampling and measurement of the concentration of the analyte in the sample were performed by the same method as in Example 1. When the concentration of the analyte was measured in the same serum in a plurality of wells, an average value of concentrations measured in the wells for the same serum group was used for calculation of the $f_u$ ratio.

[Calculation of relative $f_u$ ratio between different species using $f_u$ values in various species]

First, the obtained concentration data was applied to the following expression 5 to acquire $f_u$ values of nifedipine in serums of various species (mouse, rat, dog, monkey or human).

(Expression 5):

$$fu = \frac{\text{nifedipine concentration in acceptor solution at time point of 24 hours (nM)}}{\text{nifedipine concentration in donor solution at time point of 24 hours (nM)}}$$

Next, the $f_u$ value was applied to the following expression 6 to calculate relative $f_u$ ratios of various species with respect to the human.

(Formula 6):

$$f_u \text{ ratio} = \alpha/\beta$$

wherein $\alpha$ represents a $f_u$ value for any of various species (mouse, rat, dog or monkey), and $\beta$ represents a $f_u$ value for the human.

The obtained results are shown below.

[Table 9]

| | $f_u$ ratio (/human) |
|---|---|
| Mouse | 1.48 |
| Rat | 0.12 |
| Dog | 1.37 |
| Monkey | 1.17 |
| Human | 1.00 |

[Example 2]

Acquisition of relative $f_u$ ratio between different species by dynamic analysis (analyte: cyclosporine)

[Preparation of donor solution]

First, solutions of cyclosporine as an analyte at 10 mmol/L and 1 mmol/L were prepared. Specifically, 0.566 mL of dimethylsulfoxide (DMSO) was added to 6.81 mg of powdered cyclosporine ("Cyclosporine" manufactured by FUJIFILM Wako Pure Chemical Corporation, molecular weight: 1202.61, CLogP: 14.36, number of amino acid residues: 11) to obtain a solution of cyclosporine at 10 mmol/L. Subsequently, 180 uL of DMSO was added to 20 $\mu$L of a solution of cyclosporine at 10 mmol/L to obtain a solution of cyclosporine at 1 mmol/L.

Next, serums of various species (mouse serum, rat serum, dog serum, monkey serum and human serum) were fused. The serums of various species which had been fused were collected, and to each of the serums were added 9 times its volume of phosphate buffer physiological saline and 1/1000 times its volume of the solution of cyclosporine at 1 mmol/L to prepare a donor solution having a serum concentration of 10% (volume ratio), a cyclosporine concentration of 1 $\mu$mol/L and a DMSO concentration of 0.1% (volume ratio). Table 10 below shows the specific amounts of the samples used.

The samples used were the same as in Example 1 except for cyclosporine.

[Table 10]

| Species from which serum is derived | Serum (mL) | Phosphate buffer physiological saline (mL) | Solution of cyclosporine at 1 mmol/L ($\mu$L) |
|---|---|---|---|
| Mouse, rat, dog, monkey | 1 | 9 | 10 |
| Human | 4 | 36 | 40 |

[Preparation of acceptor solution]

The same method as described in Example 1 was carried out.

[Dialysis reaction]

The same method as described in Example 1 was carried out.

[Sampling after dialysis reaction]

The same method as described in Example 1 was carried out.

[Pretreatment of sample from sampling]

The same method as described in Example 1 was carried out.

[Measurement of concentration of analyte by LC-MS/MS]

For each of the LC-MS/MS measurement samples, a cyclosporine concentration in the solution added to each chamber was measured using the following measuring equipment and measurement conditions. When the concentration of the analyte was measured in the same serum in a plurality of wells, an average value of concentrations measured in the wells for the same serum group was used for calculation of the $f_u$ ratio.

(Measuring equipment)

LC: Waters Acquity UPLC
MS: Xevo TQ-S (Waters Corporation, Milford, Massachusetts)
Column: YMC-Triart Bio C4, column size: 2.1 x 30 mm, particle size: 1.9 $\mu$m, pore size: 30 nm

(Measurement conditions)

Column temperature: 40°C (set value)
Solvent:

A) 0.1% aqueous formic acid solution
B) 0.1% acetonitrile formate solution

Gradient conditions:

[Table 11]

| Time (min) | A% | B % |
|---|---|---|
| 0.0 | 60 | 40 |
| 0.2 | 60 | 40 |
| 0.4 | 15 | 85 |
| 0.6 | 5 | 95 |
| 0.8 | 1 | 99 |
| 1.4 | 1 | 99 |
| 1.41 | 60 | 40 |
| 2.00 | 60 | 40 |

Flow rate: 0.6 mL/min
Autosampler temperature: 10°C (set value)
Injection volume: 1$\mu$L
Preparation of calibration curve sample: the same method as described in Example 1 was carried out except that cyclosporine was used instead of nifedipine.
Calibration curve: 1 nmol/L, 3 nmol/L, 10 nmol/L, 30 nmol/L, 100 nmol/L, 300 nmol/L, 1000 nmol/L and 3000 nmol/L
MS conditions (set values): ESI positive, Capillary (kV) 2.00, Cone (V) 25.00, Source Offset (V) 50.0, Source Temperature (°C) 150, Desolvation Temperature (°C) 600, Desolvation Gas Flow (L/Hr) 1000, Cone Gas Flow (L/Hr) 150, Collision Gas Flow (mL/Min) 0.20, Nebuliser Gas Flow (Bar) 7.00

[Table 12]

| Compound | Q1 (m/z) | Q3 (m/z) | Dwell (sec) | Cone. Volt (V) | Col. Energy (ev) |
|---|---|---|---|---|---|
| Indomethacin (internal standard) | 359.24 | 139.17 | 0.221 | 30 | 20 |
| cyclosporine (analyte) | 1203.79 | 425.32 | 0.1 | 40 | 40 |

[Acquisition of $f_u$ ratio by dynamic analysis]

As in Example 1, software for performing dynamic analysis ("SimBiology (R)/MATLAB, version information: 9.7.0.1190202 (R2019b)" manufactured by The MathWorks, Inc.) was used, where the obtained data of a change in concentration was input, and the parameters and the $f_u$ ratio were calculated under the following conditions.

The relational expressions involved in the dynamic analysis are the same as expressions 1 to 3 described in Example 1. The parameters in the expressions have the following meanings.

V1: Volume of donor solution (fixed value, $3.5 \times 10^{-4}$ L)
V2: Volume of acceptor solution (fixed value, $2.0 \times 10^{-4}$ L)
fu1: fu value of analyte with respect to biological sample contained in donor solution (calculated value)
fu2: fu value of analyte with respect to biological sample contained in acceptor solution (calculated value)
C1: Concentration of analyte in donor solution (total concentration: total of bound form + unbound form analytes, except for analyte adsorbed to equilibrium dialysis device) (measured value, nmol/L)
C2: Concentration of analyte in acceptor solution (total concentration: total of bound form + unbound form analytes) (measured value, nmol/L)
PS: Dialysis membrane permeation clearance of analyte (fixed value, $1.0 \times 10^{-5}$ L/h)
CLad: Adsorption clearance of analyte in donor-side chamber (variable value, L/h)
Koff: Dissociation rate constant of analyte in donor-side chamber (variable value, $h^{-1}$)
Xad: Amount of adsorption of analyte in donor-side chamber (variable value, nmol)
Fitting: Performed with the same parameters as described in Example 1 except that the following parameters were used.

Estimated Parameters:

exp(fu1), InitialValue = 1, Bounds = [0.01 100];
exp(fu2), InitialValue = 1, Bounds = [0.01 100];
exp(Koff1), InitialValue = 1, Bounds = [0.01 1000000];
exp(Koff2), InitialValue = 1, Bounds = [0.01 1000000];
exp(CL1), InitialValue = 0.001, Bounds = [1e-07 0.1];
exp(CL2), InitialValue = 0.001, Bounds = [1e-07 0.1];
exp(Dose1), InitialValue = 0.2115, Bounds = [0.1 0.5];
exp(Dose2), InitialValue = 0.2114, Bounds = [0.1 0.5];

Table 13 shows the $f_u$ ratios of the analyte (cyclosporine) with respect to various species (/human, serum concentration: 1/10, human/human is defined as 1.00) which were calculated by the above-described dynamic analysis method, and Figure 5 ((1) to (4)) shows plotted concentrations of the analyte in the solution added to each chamber at the time points of 0.5 hours, 2 hours, 4 hours, 16 hours and 24 hours after the start of dialysis reaction and fitting curves by dynamic analysis on those plots.

[Table 13]

|  | Relative $f_u$ ratio (versus human) |
|---|---|
| Mouse | 0.63 |
| Rat | 0.99 |
| Dog | 0.82 |
| Monkey | 2.01 |
| Human | 1.00 |

[Comparative Example 2]

Acquisition of relative fu ratio between different species by static analysis (analyte: cyclosporine)

A donor solution (10% mouse, rat, dog or monkey serum) and an acceptor solution (10% human serum) were prepared in the same manner as in Example 2, dialysis reaction was started, and the concentrations of an analyte in solutions added to the chambers at the time point of 24 hours after the start of the dialysis reaction were measured. When the concentration of the analyte was measured in the same serum in a plurality of wells, an average value of concentrations measured in the wells for the same serum group was used for calculation of the $f_u$ ratio. The obtained data was applied to the following expression 7 to acquire $f_u$ ratios of cyclosporine in serums of various species (mouse, rat, dog or monkey) (versus human, human/human was defined as 1.00).

(Formula 7):

fu ratio (versus human)

$$= \frac{\text{cyclosporine concentration in acceptor solution at time point of 24 hours (nM)}}{\text{cyclosporine concentration in donor solution at time point of 24 hours (nM)}}$$

The obtained results are shown below.

[Table 14]

|  | $f_u$ ratio (/human) |
|---|---|
| Mouse | 0.23 |
| Rat | 0.45 |
| Dog | 0.27 |
| Monkey | 0.78 |
| Human | 1.00 |

[Reference Example 2]

Acquisition of $f_u$ ratio between different species by static analysis (versus buffer) (analyte: cyclosporine)

[Preparation of donor solution]

A donor solution having a serum concentration of 10% (volume ratio), a cyclosporine concentration of 1 $\mu$mol/L and a DMSO concentration of 0.1% (volume ratio) was prepared by the same method as in Example 2 except that the amounts of serums of various species (mouse, rat, dog, monkey and human), phosphate buffer physiological saline and solution of cyclosporine at 1 mmol/L were set to the amounts shown in Table 15.

[Table 15]

| Species from which serum is derived | Serum (mL) | Phosphate buffer physiological saline (mL) | Solution of cyclosporine at 1 mmol/L ($\mu$L) |
|---|---|---|---|
| Mouse, rat, dog, monkey, human | 0.2 | 1.8 | 2 |

[Preparation of acceptor solution]

The same method as described in Reference Example 1 was carried out.

[Dialysis reaction]

The same method as described in Reference Example 1 was carried out.

[Sampling after dialysis reaction]

The same method as described in Reference Example 1 was carried out.

[Pretreatment of sample from sampling and measurement of concentration of analyte by LC-MS/MS]

The same method as described in Example 2 was carried out.

[Calculation of relative $f_u$ ratio between different species using $f_u$ values in various species]

The same method as described in Reference Example 1 was carried out.
The obtained results are shown below.

[Table 16]

|  | $f_u$ ratio (versus human) |
|---|---|
| Mouse | 1.14 |
| Rat | 1.79 |
| Dog | 1.31 |
| Monkey | 2.55 |
| Human | 1.00 |

[Example 3]

Acquisition of relative $f_u$ ratio between different species by dynamic analysis (analyte: ibuprofen)

[Preparation of donor solution]

First, solutions of ibuprofen as an analyte at 10 mmol/L and 1 mmol/L were prepared. Specifically, 0.926 mL of dimethylsulfoxide (DMSO) was added to 1.91 mg of powdered ibuprofen ("Ibuprofen" manufactured by FUJIFILM Wako Pure Chemical Corporation, molecular weight: 206.28, CLogP: 3.68) to obtain a solution of ibuprofen at 10 mmol/L. Subsequently, 180 uL of DMSO was added to 20 uL of a solution of ibuprofen at 10 mmol/L to obtain a solution of ibuprofen at 1 mmol/L.
Next, serums of various species (mouse serum, rat serum, dog serum, monkey serum and human serum) were fused. The serums of various species which had been fused were collected, and to each of the serums were added 9 times its volume of phosphate buffer physiological saline and 1/1000 times its volume of the solution of ibuprofen at 1 mmol/L to prepare a donor solution having a serum concentration of 10% (volume ratio), an ibuprofen concentration of 1 $\mu$mol/L and a DMSO concentration of 0.1% (volume ratio). Table 17 below shows the specific amounts of the samples used.
The samples used were the same as in Example 1 except for ibuprofen.

[Table 17]

| Species from which serum is derived | Serum (mL) | Phosphate buffer physiological saline (mL) | Solution of ibuprofen at 1 mmol/L ($\mu$L) |
|---|---|---|---|
| Mouse, rat, dog, monkey | 1 | 9 | 10 |
| Human | 4 | 36 | 40 |

[Preparation of acceptor solution]

The same method as described in Example 1 was carried out.

[Dialysis reaction]

The same method as described in Example 1 was carried out.

[Sampling after dialysis reaction]

The same method as described in Example 1 was carried out.

[Pretreatment of sample from sampling]

The same method as described in Example 1 was carried out.

[Measurement of concentration of analyte by LC-MS/MS]

For each of the LC-MS/MS measurement samples, an ibuprofen concentration in the solution added to each chamber was measured using the following measuring equipment and measurement conditions. When the concentration of the analyte was measured in the same serum in a plurality of wells, an average value of concentrations measured in the wells for the same serum group was used for calculation of the $f_u$ ratio, as in Example 1.

(Measuring equipment)

LC: Waters Acquity UPLC
MS: Xevo TQ-S (Waters Corporation, Milford, Massachusetts)
Column: YMC-Triart C18 column, column size: 2.0 x 30 mm, particle size: 1.9 $\mu$m, pore size: 12 nm

(Measurement conditions)

Column temperature: 50°C (set value)
Solvent:

A) 10 mmol/L aqueous solution of ammonium formate (water : solution of ammonium formate at 1 mol/L, 1000 ml : 10 ml)
B) 10 mmol/L MeOH solution of ammonium formate (MeOH : solution of ammonium formate at 1 mol/L, 1000 ml : 10 ml)

Gradient conditions:

[Table 18]

| Time (min) | A% | B % |
| --- | --- | --- |
| 0.0 | 99 | 1 |
| 0.8 | 1 | 99 |
| 1.6 | 1 | 99 |
| 1.61 | 99 | 1 |
| 2.0 | 99 | 1 |

Flow rate: 0.6 mL/min
Autosampler temperature: 10°C (set value)
Injection Volume: 10 $\mu$L
Preparation of calibration curve sample: 90 $\mu$L of DMSO was added to 10 $\mu$L of a solution of ibuprofen at 1 mmol/L dissolved in DMSO to obtain a solution of ibuprofen at 100 $\mu$mol/L, and the following series of dilutions (1) to (9) and BLANK solution were prepared from the solution of ibuprofen at 100 $\mu$mol/L.
[(1) 376 $\mu$L of DMSO was added to 24 $\mu$L of a solution of ibuprofen at 100 $\mu$mol/L to obtain a solution of ibuprofen at 6000 nmol/L.
(2) 240 $\mu$L of DMSO was added to 240 $\mu$L of a solution of ibuprofen at 6000 nmol/L to obtain a solution of ibuprofen at 3000 nmol/L.
(3) 120 $\mu$L of DMSO was added to 240 $\mu$L of a solution of ibuprofen at 3000 nmol/L to obtain a solution of ibuprofen at 2000 nmol/L.
(4) 280 $\mu$L of DMSO was added to 120 $\mu$L of a solution of ibuprofen at 2000 nmol/L to obtain a solution of ibuprofen at 600 nmol/L.
(5) 240 $\mu$L of DMSO was added to 240 $\mu$L of a solution of ibuprofen at 600 nmol/L to obtain a solution of ibuprofen at 300 nmol/L.
(6) 120 $\mu$L of DMSO was added to 240 $\mu$L of a solution of ibuprofen at 300 nmol/L to obtain a solution of ibuprofen at 200 nmol/L.

(7) 280 μL of DMSO was added to 120 μL of a solution of ibuprofen at 200 nmol/L to obtain a solution of ibuprofen at 60 nmol/L.

(8) 240 μL of DMSO was added to 240 μL of a solution of ibuprofen at 60 nmol/L to obtain a solution of ibuprofen at 30 nmol/L.

(9) 120 μL of DMSO was added to 240 μL of a solution of ibuprofen at 30 nmol/L to obtain a solution of ibuprofen at 20 nmol/L. 200 μL of DMSO was used as a BLANK solution.]

To 10 μL of each of the series of dilutions (1) to (9) and the BLANK solution, 20 μL of a matrix solution (animal or human serum/PBS/DMSO = 10/90/0.1, volume ratio) and 170 μL of a quench solution 2 [a solution obtained by adding 80 μL of an indomethacin solution (concentration: 200 μg/mL) to 80 mL of a solution of methanol/acetonitrile = 1/1] were added to obtain a calibration curve sample. The calibration curve sample was pretreated by the same method as that for the sample from sampling. Since the series of dilutions (1) to (9) were each adjusted to a concentration 2 times that of an actual sample, the concentrations of the calibration curve samples were determined with a correction made by halving the concentrations adjusted by the series of dilutions.

Concentrations of calibration curve samples: (9) 10 nmol/L, (8) 15 nmol/L, (7) 30 nmol/L, (6) 100 nmol/L, (5) 150 nmol/L, (4) 300 nmol/L, (3) 1000 nmol/L, (2) 1500 nmol/L and (1) 3000 nmol/L

Calibration curve: 10 nmol/L, 15 nmol/L, 30 nmol/L, 100 nmol/L, 150 nmol/L, 300 nmol/L, 1000 nmol/L, 1500 nmol/L and 3000 nmol/L

MS conditions (set values): (ESI negative), Capillary (kV) 2.00, Cone (V) 25.00, Source Offset (V) 50.0, Source Temperature (°C) 150, Desolvation Temperature (°C) 600, Desolvation Gas Flow (L/Hr) 1000, Cone Gas Flow (L/Hr) 150, Collision Gas Flow (mL/Min) 0.20, Nebuliser Gas Flow (Bar) 7.00

[Table 19]

| Compound | Q1 (m/z) | Q3 (m/z) | Dwell (sec) | Cone. volt (V) | Col. Energy (eV) |
|---|---|---|---|---|---|
| Indomethacin (internal standard) | 355.92 | 297 | 0.221 | 30 | 20 |
| Ibuprofen (analyte) | 205.12 | 161.30 | 0.2 | 10 | 5 |

[Acquisition of $f_u$ ratio by dynamic analysis]

The $f_u$ ratio of ibuprofen was determined by the method described in Example 1. For mouse versus human, the result of measurement on human donor → mouse acceptor was not used for analysis and the $f_u$ ratio was determined by the result of measurement on mouse donor → human acceptor, i.e. analysis only in one direction because the concentration of the mouse acceptor was below LLOQ and thus undetectable.

Fitting (human versus mouse):

For human versus mouse, fitting was performed with the following conditions described in default settings for Fit Data (SimBiology Model Analyzer, Program).

Definition:

When the first biological sample was from a mouse, and the second biological sample was from a human, a set of the following two pieces of data was used for analysis.

Data 1 (Eq1.Ca1): Concentration of analyte in acceptor solution in equilibrium dialysis system with donor solution containing mouse serum and acceptor solution containing human serum

Data 2 (Eq1.Cd1): Concentration of analyte in donor solution in equilibrium dialysis system with donor solution containing mouse serum and acceptor solution containing human serum

Estimated Parameters:

exp(fu1), InitialValue = 1, Bounds = [0.01 100];
exp(fu2), InitialValue = 1, Bounds = [0.01 100];
exp(Koff1), InitialValue = 1, Bounds = [0.01 1000000];
exp(CL1), InitialValue = 0.001, Bounds = [1e-07 0.1];
exp(Dose1), InitialValue = 0.2115, Bounds = [0.1 0.5];

Pooled fit, true

Other set values were the same as in Example 1.

Fitting (human versus rat, human versus dog and human versus monkey):

For human versus rat, human versus dog and human versus monkey, fitting was performed with the same parameters as described in Example 1 except that the following parameters were used.

Estimated Parameters:

exp(fu1), InitialValue = 1, Bounds = [0.01 100];
exp(fu2), InitialValue = 1, Bounds = [0.01 100];
exp(Koff1), InitialValue = 1, Bounds = [0.01 1000000];
exp(Koff2), InitialValue = 1, Bounds = [0.01 1000000];
exp(CL1), InitialValue = 0.001, Bounds = [1e-07 0.1];
exp(CL2), InitialValue = 0.001, Bounds = [1e-07 0.1];
exp(Dose1), InitialValue = 0.2115, Bounds = [0.1 0.5];
exp(Dose2), InitialValue = 0.2114, Bounds = [0.1 0.5];

Table 20 shows the $f_u$ ratios of the analyte (ibuprofen) with respect to various species (/human, serum concentration: 1/10, human/human is defined as 1.00) which were calculated by the above-described dynamic analysis method, and Figure 6 ((1) to (4)) shows plotted concentrations of the analyte in the solution added to each chamber at the time points of 0.5 hours, 2 hours, 4 hours, 16 hours and 24 hours after the start of dialysis reaction and fitting curves by dynamic analysis on those plots.

[Table 20]

|  | Relative $f_u$ ratio (versus human) |
|---|---|
| Mouse | 17.02 |
| Rat | 4.58 |
| Dog | 1.06 |
| Monkey | 2.41 |
| Human | 1.00 |

[Comparative Example 3]

Acquisition of relative fu ratio between different species by static analysis (analyte: ibuprofen)

A donor solution (10% mouse, rat, dog or monkey serum) and an acceptor solution (10% human serum) were prepared in the same manner as in Example 3, dialysis reaction was started, and the concentrations of an analyte in solutions added to the chambers at the time point of 24 hours after the start of the dialysis reaction were measured. When the concentration of the analyte was measured in the same serum in a plurality of wells, an average value of concentrations measured in the wells for the same serum group was used for calculation of the $f_u$ ratio. The obtained data was applied to the following expression 8 to acquire $f_u$ ratios of ibuprofen in serums of various species (mouse, rat, dog or monkey) (versus human, human/human was defined as 1.00).

(Formula 8):

fu ratio (versus human)

$$= \frac{\text{ibuprofen concentration in acceptor solution at time point of 24 hours (nM)}}{\text{ibuprofen concentration in donor solution at time point of 24 hours (nM)}}$$

The obtained results are shown below.

[Table 21]

|  | $f_u$ ratio (/human) |
|---|---|
| Mouse | 7.58 |
| Rat | 3.37 |
| Dog | 0.59 |
| Monkey | 1.26 |
| Human | 1.00 |

[Reference Example 3]

Acquisition of $f_u$ ratio between different species by static analysis (versus buffer) (analyte: ibuprofen)

[Preparation of donor solution]

A donor solution having a serum concentration of 10% (volume ratio), an ibuprofen concentration of 1 μmol/L and a DMSO concentration of 0.1% (volume ratio) was prepared by the same method as in Example 2 except that the amounts of serums of various species (mouse, rat, dog, monkey and human), phosphate buffer physiological saline and solution of ibuprofen at 1 mmol/L were set to the amounts shown in Table 22.

[Table 22]

| Species from which serum is derived | Serum (mL) | Phosphate buffer physiological saline (mL) | Solution of ibuprofen at 1 mmol/L (μL) |
|---|---|---|---|
| Mouse, rat, dog, monkey human | 0.2 | 1.8 | 2 |

[Preparation of acceptor solution]

The same method as described in Reference Example 1 was carried out.

[Dialysis reaction]

The same method as described in Reference Example 1 was carried out.

[Sampling after dialysis reaction]

The same method as described in Reference Example 1 was carried out.

[Pretreatment of sample from sampling and measurement of concentration of analyte by LC-MS/MS]

The same method as described in Example 3 was carried out.

[Calculation of relative $f_u$ ratio between different species using $f_u$ values in various species]

The same method as described in Reference Example 1 was carried out.

The obtained results are shown below.

[Table 23]

|  | $f_u$ ratio (versus human) |
|---|---|
| Mouse | 16.08 |
| Rat | 5.01 |
| Dog | 1.76 |
| Monkey | 3.34 |
| Human | 1.00 |

[Comparison of relative $f_u$ ratios between different species acquired in Examples 1 to 3, Comparative Examples 1 to 3 and Reference Examples 1 to 3]

The relative $f_u$ ratios between different species, which had been acquired in Example 1, Comparative Example 1 and Reference Example 1, Example 2, Comparative Example 2 and Reference Example 2 and Example 3, Comparative Example 3 and Reference Example 3, were compared, and the results shown in Figures 7 to 12 were obtained.

This showed that for the relative $f_u$ ratio by the method of the present invention (a $f_u$ ratio between different species which is acquired by dynamic analysis from a change in concentration until the time point of 24 hours of dialysis reaction after the start of the dialysis reaction where serum derived from an arbitrary species is added to each of a donor solution and an acceptor solution) and the $f_u$ ratio determined by a conventional known method and shown in each of Reference Examples (a value obtained by calculating a $f_u$ ratio between an animal and a human using a $f_u$ value determined from a concentration ratio at the time point of 24 hours of dialysis reaction after the start of the dialysis reaction where serum derived from an arbitrary species is added to a donor solution and phosphate buffer physiological saline is used as an acceptor solution), all of the plots for each compound fell within 2-fold error. The absolute average fold error (AAFE) was confirmed to be 1.16-fold error for nifedipine, 1.46-fold error for cyclosporine, and 1.22-fold error for ibuprofen. (Figures 7, 8 and 9).

On the other hand, for the correlation between the $f_u$ ratio by the method of each of Comparative Examples (a $f_u$ ratio between an animal and a human which is determined from a concentration ratio at the time point of 24 hours of dialysis reaction after the start of the dialysis reaction where serum derived from an arbitrary species is added to each of a donor solution and an acceptor solution) and the $f_u$ ratio determined by a conventional known method and shown in each of Reference Examples, all of the five plots for nifedipine, one of the five plots for cyclosporine and three of the five plots for ibuprofen fell within 2-fold error. The absolute average fold errors were confirmed to be 1.08 times for nifedipine, 3.16 times for cyclosporine, and 1.90 times for ibuprofen. (Figures 10, 11 and 12). That is, for nifedipine which reaches an equilibrium state in a relatively short time, the relative $f_u$ ratios by the conventional method and the present invention were equivalent to each other, whereas for cyclosporine and ibuprofen which hardly reach an equilibrium state, the $f_u$ ratio determined by the conventional method (static analysis) was inferior in accuracy to the relative $f_u$ ratio by the method of the present invention (dynamic analysis) and the $f_u$ ratio determined by the conventional known method and shown in each of Reference Examples.

Therefore, it can be seen that by using the method of the present invention, a relative $f_u$ ratio having a good correlation with the $f_u$ ratio by the conventional known method can be acquired before an equilibrium state is reached (i.e. in a shorter time) even for an analyte which hardly reaches an equilibrium state.

[Examples 4 to 13]

Acquisition of relative $f_u$ ratio between different species by dynamic analysis (analytes: Asunaprevir, Apalutamide, Elbasvir, Dutasteride, Deferasirox, Aripiprazole, Beclabuvir, Lomitapide, Ibrutinib and Montelukast)

[Preparation of donor solution]

Asunaprevir, Apalutamide, Elbasvir, Dutasteride, Deferasirox, Aripiprazole, Beclabuvir, Lomitapide, Ibrutinib and Montelukast were used as analytes. For each analyte, a solution at 10 mmol/L was prepared. Table 24 below shows the specific samples used and the amounts thereof.

EP 4 220 152 B1

[Table 24]

|  | Analyte | Manufacturer | MW | clog P | Weight (mg) | Amount of DMSO added (μL) |
|---|---|---|---|---|---|---|
| Example 4 | Asunaprevir | Chemscene Lcc (Cem) | 748.29 | 3.37 | 1.52 | 203.1 |
| Example 5 | Apalutamide | Chemscene Lcc (Cem) | 477.43 | 3.46 | 1.28 | 268.1 |
| Example 6 | Elbasvir | Chemscene Lcc (Cem) | 882.02 | 6.14 | 1.39 | 157.6 |
| Example 7 | Dutasteride | Tronto Research Chemicals Inc. | 528.53 | 5.79 | 2.62 | 495.7 |
| Example 8 | Deferasirox | Tronto Research Chemicals Inc. | 373.36 | 4.74 | 2.23 | 597.3 |
| Example 9 | Aripiprazole | Chemscene Lcc (Cem) | 448.39 | 4.90 | 2.24 | 499.6 |
| Example 10 | Beclabuvir | Chemscene Lcc (Cem) | 659.84 | 2.64 | 1.72 | 260.7 |
| Example 11 | Lomitapide | Tronto Research Chemicals Inc. | 693.72 | 7.70 | 1.48 | 213.3 |
| Example 12 | Ibrutinib | Chemscene Lcc (Cem) | 440.50 | 3.63 | 1.22 | 277.0 |
| Example 13 | Montelukast | Selleck Biotech Co., Ltd. | 586.18 | 8.49 | 1.42 | 242.2 |

Next, a solution of each analyte at 1 mmol/L was prepared. Specifically, the solution was prepared by adding 90 μL of DMSO to 10 μL of the solution containing each analyte at 10 mmol/L.

Next, serums of various species (rat serum and human serum) were fused. The serums of various species which had been fused were collected, and to each of the serums was added 9 times its volume of phosphate buffer physiological saline. To the resulting solutions were added 1/5000 times its volume of the solution of Asunaprevir at 1 mmol/L, 1/5000 times its volume of the solution of Apalutamide at 1 mmol/L, 1/5000 times its volume of solution of Elbasvir at 1 mmol/L, 1/5000 times its volume of the solution of Dutasteride at 1 mmol/L and 1/5000 times its volume of the solution of Deferasirox at 1 mmol/L, respectively. The serums of various species were additionally collected, and to each of the serums was added 9 times its volume of phosphate buffer physiological saline. To the resulting solutions were added 1/5000 times its volume of the solution of Aripiprazole at 1 mmol/L, 1/5000 times its volume of the solution of Beclabuvir at 1 mmol/L, 1/5000 times its volume of the solution of Lomitapide at 1 mmol/L, 1/5000 times its volume of the solution of Ibrutinib at 1 mmol/L and 1/5000 times its volume of the solution of Montelukast at 1 mmol/L, respectively. In this way, donor solutions were prepared in which the serum concentration was 10% (volume ratio), the DMSO concentration was 0.1% (volume ratio), and the concentration of each of Asunaprevir, Apalutamide, Elbasvir, Dutasteride and Deferasirox was 0.2 μmol/L, or the concentration of each of Aripiprazole, Beclabuvir, Lomitapide, Ibrutinib and Montelukast was 0.2 μmol/L. Tables 25 and 26 below show the specific amounts of samples used.

The samples used were the same as in Example 1 except for nifedipine.

[Table 25]

| Species from which serum is derived | Serum (mL) | Phosphate buffer physiological saline (mL) | Solution of each of Asunaprevir, Apalutamide, Elbasvir, Dutasteride and Deferasirox at 1 mmol/L (μL) |
|---|---|---|---|
| Human | 1 | 9 | 2 for each solution |
| Rat | 2.5 | 22.5 | 5 for each solution |

[Table 26]

| Species from which serum is derived | Serum (mL) | Phosphate buffer physiological saline (mL) | Solution of each of Aripiprazole, Beclabuvir, Lomitapide, Ibrutinib and Montelukast at 1 mmol/L (μL) |
|---|---|---|---|
| Human | 1 | 9 | 2 for each solution |
| Rat | 2.5 | 22.5 | 5 for each solution |

43

[Preparation of acceptor solution]

Except that a DMSO solution was added instead of the solution containing each analyte at 1 mmol/L, the same procedure as that for the donor solution was carried out to prepare an acceptor solution having a serum concentration of 10% (volume ratio) and a DMSO concentration of 0.1% (volume ratio).

[Dialysis reaction]

The same method as in Example 1 was carried out except that the analyte was changed from nifedipine to the analytes of Examples 4 to 13. Table 27 below shows the donor solutions or acceptor solutions injected into the chambers.

Table 27 shows the measurement performed at the time points of 0.5 hours, 2 hours, 4 hours, 20 hours and 24 hours for each of a total of six patterns where rat serum was set on the donor side and human serum was set on the acceptor side (tests A to C: the same conditions) and where the human serum was set on the donor side and the rat serum was set on the acceptor side (tests D to F: the same conditions).

(Table 27) Chamber pattern using rat/human serum

| Red device | 1 | 2 | 3 | 4 | 5 | 6 | 7 | 8 | 9 | 10 |
|---|---|---|---|---|---|---|---|---|---|---|
| Elapsed time | 0.5h | | 2h | | 4h | | 20h | | 24h | |
| Chamber | Donor | Acceptor | Donor | Acceptor | Donor | Acceptor | Donor | Acceptor | Donor | Acceptor |
| A | Rat | Human | Rat | Human | Rat | Human | Rat | Human | Rat | Human |
| | 1 | 1 | 1 | 1 | 1 | 1 | 1 | 1 | 1 | 1 |
| B | Rat | Human | Rat | Human | Rat | Human | Rat | Human | Rat | Human |
| | 2 | 2 | 2 | 2 | 2 | 2 | 2 | 2 | 2 | 2 |
| C | Rat | Human | Rat | Human | Rat | Human | Rat | Human | Rat | Human |
| | 3 | 3 | 3 | 3 | 3 | 3 | 3 | 3 | 3 | 3 |
| D | Human | Rat | Human | Rat | Human | Rat | Human | Rat | Human | Rat |
| | 1 | 1 | 1 | 1 | 1 | 1 | 1 | 1 | 1 | 1 |
| E | Human | Rat | Human | Rat | Human | Rat | Human | Rat | Human | Rat |
| | 2 | 2 | 2 | 2 | 2 | 2 | 2 | 2 | 2 | 2 |
| F | Human | Rat | Human | Rat | Human | Rat | Human | Rat | Human | Rat |
| | 3 | 3 | 3 | 3 | 3 | 3 | 3 | 3 | 3 | 3 |

[Sampling after dialysis reaction]

The same method as in Example 1 was carried out except that the collected solution and acceptor solution were hermetically sealed without using a quench solution, and each sample was stored at -80°C before being subjected to pretreatment.

[Pretreatment of sample from sampling]

5 μL of DMSO and 100 μL of an IS solution were added to 20 μL of the sample after sampling, and the mixture was transferred to a filter plate ("Multi Screen Filter Plates 0.45um, Low Binding" manufactured by Millipore Corporation), and filtered. 50 μL of water was added to the sample after the filtration to obtain a LC-MS/MS measurement sample. The composition of the IS solution is as shown in Table 28 below.

[Table 28]

| IS solution | Acetonitrile (mL) | Solution of verapamil at 100 μg/mL in DMSO(μL) | Solution of indomethacin at 1 mg/mL in DMSO (μL) |
|---|---|---|---|
|  | 50 | 0.5 | 0.5 |

[Measurement of concentration of analyte by LC-MS/MS]

For each of the LC-MS/MS measurement samples, the concentration of each analyte in each chamber was measured using the following measuring equipment and measurement conditions. When the concentration of the analyte was measured in the same serum in a plurality of wells, an average value of concentrations measured in the wells for the same serum group was used for calculation of the $f_u$ ratio.

(Measuring equipment)

LC: Nexera (SHIMADZU)
MS: QTRAP 6500 (Sciex)
Column: InertSustain C18 HP 3um, 2.1 x 30 mm (UP)

(Measurement conditions)

Column temperature: 40°C (set value)
Solvent:

A) 0.1% aqueous formic acid solution
B) 0.1% acetonitrile formate solution

Gradient conditions:

[Table 29]

| Time (min) | A% | B % |
|---|---|---|
| 0.0 | 95 | 5 |
| 0.5 | 70 | 30 |
| 0.9 | 5 | 95 |
| 1.2 | 5 | 95 |
| 1.25 | 95 | 5 |
| 1.8 | 95 | 5 |

Flow rate: 0.6 mL/min
Autosampler temperature: 4°C (set value)
Injection volume: 2 μL in Examples 4 to 8, 1 μL in Examples 9 to 13

[Preparation and pretreatment of calibration curve sample]

450 μL of PBS was added to 50 μL of rat serum or human serum to obtain 10% serum (blank matrix), 5 μL of a solution of the calibration curve sample dissolved in DMSO and 100 μL of the IS solution were added to 20 μL of the blank matrix, and the mixture was transferred to a filter plate ("Multi Screen Filter Plates 0.45um, Low Binding" manufactured by Millipore Corporation), and filtered. 50 μL of water was added to the sample after the filtration to obtain a LC-MS/MS measurement sample. A calibration curve working solution was prepared as follows. A sample obtained by adding 5 μL of DMSO instead of 5 μL of the solution of calibration curve sample was used as a BLANK sample.

Preparation of solution of calibration curve sample: The following series of dilutions (1) to (8) were prepared such that solutions of Asunaprevir, Apalutamide, Elbasvir, Dutasteride and Deferasirox at 10 mmol/L dissolved in DMSO were each added in an amount of 2 μL to 190 μL of DMSO to a total amount of 200 μL to obtain a solution of the analytes each at a concentration of 100 μmol/L.

[(1) 90 μL of DMSO was added to 60 μL of a solution of the analytes each at 100 μmol/L to obtain a compound solution at 40000 nmol/L.

(2) 140 μL of DMSO was added to 60 μL of a solution of the analytes each at 40000 nmol/L to obtain a compound solution at 12000 nmol/L.

(3) 120 μL of DMSO was added to 60 μL of a solution of the analytes each at 12000 nmol/L to obtain a compound solution at 4000 nmol/L.

(4) 140 μL of DMSO was added to 60 μL of a solution of the analytes each at 4000 nmol/L to obtain a compound solution at 1200 nmol/L.

(5) 120 μL of DMSO was added to 60 μL of a solution of the analytes each at 1200 nmol/L to obtain a compound solution at 400 nmol/L.

(6) 140 μL of DMSO was added to 60 μL of a solution of the analytes each at 400 nmol/L to obtain a compound solution at 120 nmol/L.

(7) 120 μL of DMSO was added to 60 μL of a solution of the analytes each at 120 nmol/L to obtain a compound solution at 40 nmol/L.

(8) 140 μL of DMSO was added to 60 μL of a solution of the analytes each at 40 nmol/L to obtain a compound solution at 12 nmol/L.] Since the series of dilutions (1) to (8) were each adjusted to a concentration 4 times that of an actual sample, the concentrations of the calibration curve samples were determined with a correction made in consistency with 1/4 times the concentration adjusted by the series of dilutions.

Concentrations of calibration curve samples: (8) 3 nM, (7) 10 nM, (6) 30 nM, (5) 100 nM, (4) 300 nM, (3) 1000 nM and (2) 3000 nM

Solutions of Aripiprazole, Beclabuvir, Lomitapide, Ibrutinib and Montelukast at 10 mmol/L dissolved in DMSO were each added in an amount of 2 μL to 190 μL of DMSO to a total amount of 200 μL to obtain a solution of the analytes each at a concentration of 100 μmol/L. The following series of dilutions (1) to (8) were prepared.

[(1) 144 μL of DMSO was added to 6 μL of a solution of the analytes each at 100 μmol/L to obtain a compound solution at 4000 nmol/L.

(2) 140 μL of DMSO was added to 60 μL of a solution of the analytes each at 4000 nmol/L to obtain a compound solution at 1200 nmol/L.

(3) 120 μL of DMSO was added to 60 μL of a solution of the analytes each at 1200 nmol/L to obtain a compound solution at 400 nmol/L.

(4) 140 μL of DMSO was added to 60 μL of a solution of the analytes each at 400 nmol/L to obtain a compound solution at 120 nmol/L.

(5) 120 μL of DMSO was added to 60 μL of a solution of the analytes each at 120 nmol/L to obtain a compound solution at 40 nmol/L.

(6) 140 μL of DMSO was added to 60 μL of a solution of the analytes each at 40 nmol/L to obtain a compound solution at 12 nmol/L.

(7) 120 μL of DMSO was added to 60 μL of a solution of the analytes each at 12 nmol/L to obtain a compound solution at 4 nmol/L.

(8) 140 μL of DMSO was added to 60 μL of a solution of the analytes each at 4 nmol/L to obtain a compound solution at 1.2 nmol/L.]

Since the series of dilutions (1) to (8) were each adjusted to a concentration 4 times that of an actual sample, the concentrations of the calibration curve samples were determined with a correction made in consistency with 1/4 times the concentration adjusted by the series of dilutions.

Concentrations of calibration curve samples: (8) 0.3 nM, (7) 1 nM, (6) 3 nM, (5) 10 nM, (4) 30 nM, (3) 100 nM and (2) 300 nM

[Table 30]

| Compound | Q1 (m/z) | Q3 (m/z) | DP | CE | CXP |
|---|---|---|---|---|---|
| Verapamil (internal standard) | 455.200 | 165.100 | 96.00 | 37.00 | 12.00 |
| Indomethacin (internal standard) | 357.917 | 138.900 | 81.00 | 23.00 | 10.00 |
| Asunaprevir | 748.286 | 647.900 | 66.00 | 27.00 | 18.00 |
| Apalutamide | 478.020 | 449.900 | 216.00 | 33.00 | 14.00 |
| Elbasvir | 882.165 | 708.200 | 236.00 | 71.00 | 14.00 |
| Dutasteride | 529.064 | 461.000 | 211.00 | 49.00 | 14.00 |
| Deferasirox | 373.943 | 346.000 | 116.00 | 45.00 | 12.00 |
| Aripiprazole | 448.072 | 285.200 | 166.00 | 39.00 | 16.00 |
| Beclabuvir | 660.128 | 534.900 | 151.00 | 51.00 | 28.00 |
| Lomitapide | 694.042 | 248.900 | 196.00 | 57.00 | 14.00 |
| Ibrutinib | 441.225 | 138.000 | 176.00 | 35.00 | 10.00 |
| Montelukast | 586.079 | 568.100 | 126.00 | 23.00 | 18.00 |

In Table 30, DP is declustering potential, CE is collision energy, and CEP is collision entrance potential.

MS conditions: (ESI positive) Curtain Gas TM flow (CUR) 30, IonSpray TM Voltage (IS) 5500, Temperature (TEM) 650, Ion Source Gas 1 (GS1) 20, Ion Source Gas 2 (GS2) 20

[Acquisition of $f_u$ ratio by dynamic analysis]

The rat-to-human $f_u$ ratio was calculated by the same method as in Example 1 except that the conditions were changed as shown below.

Definition

When the first biological sample was from a rat, and the second biological sample was from a human, a set of the following four pieces of data was used for analysis.

Data 1 (Eq1.Cd1): Concentration of analyte in donor solution in equilibrium dialysis system with donor solution containing rat serum and acceptor solution containing human serum

Data 2 (Eq1.Ca1): Concentration of analyte in acceptor solution in equilibrium dialysis system with donor solution containing rat serum and acceptor solution containing human serum

Data 3 (Eq2.Cd2): Concentration of analyte in donor solution in equilibrium dialysis system with donor solution containing human serum and acceptor solution containing rat serum

Data 4 (Eq2.Ca2): Concentration of analyte in acceptor solution in equilibrium dialysis system with donor solution containing human serum and acceptor solution containing rat serum

Estimated Parameters:

exp(fu1).InitialValue = 0.01; Bounds = [1e-05 1];

exp(fu2).InitialValue = 0.01; Bounds = [1e-05 1];
exp(Koff1).InitialValue = 1; Bounds = [1e-05 10000];
exp(Koff2).InitialValue = 1; Bounds = [1e-05 10000];
exp(CL1). InitialValue = 0.001; Bounds = [1e-07 100];
exp(CL2), InitialValue = 0.001; Bounds = [1e-07 100];
exp(Dose1).InitialValue = 0.2115; Bounds = [0.01 0.3];
exp(Dose2).InitialValue = 0.2114; Bounds = [0.01 0.3];

The obtained results are shown below.

[Table 31]

|  | $f_u$ ratio (rat/human) |
| --- | --- |
| Asunaprevir | 3.34 |
| Apalutamide | 2.50 |
| Elbasvir | 2.90 |
| Dutasteride | 1.94 |
| Deferasirox | 3.12 |
| Aripiprazole | 1.74 |
| Beclabuvir | 1.34 |
| Lomitapide | 6.52 |
| Ibrutinib | 0.26 |
| Montelukast | - |

[Comparative Examples 4 to 13]

Acquisition of relative fu ratio between different species by static analysis (analyte: the same as in Examples 4 to 13)

A donor solution and an acceptor solution were prepared in the same manner as in Examples 4 to 13, dialysis reaction was started, and the concentrations of an analyte in solutions added to the chambers at the time point of 24 hours after the start of the dialysis reaction were measured. When the concentration of the analyte was measured in the same serum in a plurality of wells, an average value of concentrations measured in the wells for the same serum group was used for calculation of the $f_u$ ratio. The obtained data was applied to expression 4 in Comparative Example 1 to acquire $f_u$ ratios of the analyte with respect to various species (rat/human serum concentration: 1/10).

The obtained results are shown below.

[Table 32]

|  | $f_u$ ratio (rat/human) |
| --- | --- |
| Asunaprevir | 0.69 |
| Apalutamide | 3.36 |
| Elbasvir | 0.18 |
| Dutasteride | 1.70 |
| Deferasirox | 1.39 |
| Aripiprazole | 0.98 |
| Beclabuvir | 0.84 |
| Lomitapide | 0.57 |
| Ibrutinib | 0.23 |
| Montelukast | - |

[Reference Examples 4 to 13]

Acquisition of $f_u$ ratio between different species by static analysis (versus buffer) (analyte: the same as in Examples 4 to 13)

[Preparation of donor solution]

Asunaprevir, Apalutamide, Elbasvir, Dutasteride, Deferasirox, Aripiprazole, Beclabuvir, Lomitapide, Ibrutinib and Montelukast were used as analytes. A solution of each analyte at 10 mmol/L was prepared. Table 33 below shows the specific samples used and the amounts thereof.

[Table 33]

|  | Analyte | Manufacturer | Weight (mg) | Amount of DMSO added (μL) |
|---|---|---|---|---|
| Reference Example 4 | Asunaprevir | Chemscene Lcc (Cem) | 1.29 | 172.4 |
| Reference Example 5 | Apalutamide | Chemscene Lcc (Cem) | 0.625 | 130.9 |
| Reference Example 6 | Elbasvir | Chemscene Lcc(Cem) | 0.715 | 81.1 |
| Reference Example 7 | Dutasteride | Tronto Research Chemicals Inc. | 1.288 | 243.7 |
| Reference Example 8 | Deferasirox | ASTATECH | 2.409 | 645 |
| Reference Example 9 | Aripiprazole | Chemscene Lcc (Cem) | 2.222 | 496 |
| Reference Example 10 | Beclabuvir | Chemscene Lcc (Cem) | 0.976 | 147.9 |
| Reference Example 11 | Lomitapide | Tronto Research Chemicals Inc. | 0.674 | 97.2 |
| Reference Example 12 | Ibrutinib | Adipogen Life Sciences | 1.15 | 261.1 |
| Reference Example 13 | Montelukast | Selleck Biotech Co., Ltd. | 1.244 | 212.2 |

Next, a cocktail solution of analytes was prepared. Specifically, 2 μL of the solution of Asunaprevir at 10 mmol/L, 2 μL of the solution of Apalutamide at 10 mmol/L, 2 μL of the solution of Elbasvir at 10 mmol/L, 2 μL of the solution of Dutasteride at 10 mmol/L and 2 μL of the solution of Deferasirox at 10 mmol/L were mixed with 90 μL of DMSO to prepare 100 μL in total of a cocktail solution. 2 μL of the solution of Aripiprazole at 10 mmol/L, 2 μL of the solution of Beclabuvir at 10 mmol/L, 2 μL of the solution of Lomitapide at 10 mmol/L, 2 μL of the solution of Ibrutinib at 10 mmol/L and 2 μL of the solution of Montelukast at 10 mmol/L were mixed with 90 μL of DMSO to prepare 100 μL in total of a cocktail solution.
Next, serums of various species (rat serum and human serum) were fused. The serums of various species which had been fused were collected, and to each of the serums was added 9 times its volume of phosphate buffer physiological saline. To the resulting solution was added 1/1000 times its volume of the cocktail solution containing Asunaprevir, Apalutamide, Elbasvir, Dutasteride and Deferasirox each at a concentration of 0.2 μmol/L, or 1/1000 times its volume of the cocktail solution containing Aripiprazole, Beclabuvir, Lomitapide, Ibrutinib and Montelukast each at a concentration of 0.2 μmol/L. Tables 34 and 35 below show the specific amounts of samples used. The samples used were the same as in Example 1 except for nifedipine.

[Table 34]

| Species from which serum is derived | Serum (mL) | Phosphate buffer physiological saline (mL) | Mixed solution of Asunaprevir, Apalutamide, Elbasvir, Dutasteride and Deferasirox (0.2 mmol/L for each analyte) (μL) |
|---|---|---|---|
| Human | 0.12 | 1.08 | 1.2 |
| Rat | 0.12 | 1.08 | 1.2 |

[Table 35]

| Species from which serum is derived | Serum (mL) | Phosphate buffer physiological saline (mL) | Mixed solution of Aripiprazole, Beclabuvir, Lomitapide, Ibrutinib and Montelukast (0.2 mmol/L for each analyte) ($\mu$L) |
|---|---|---|---|
| Human | 0.12 | 1.08 | 1.2 |
| Rat | 0.12 | 1.08 | 1.2 |

[Preparation of acceptor solution]

The same procedure as in Examples 4 to 13 was carried out to prepare an acceptor solution having a DMSO concentration of 0.1% (volume ratio).

[Dialysis reaction]

A rat/human fu value in each analyte was acquired by the same method as in Reference Example 1 except that the donor solution was changed to the donor solution prepared in Reference Examples 4 to 13 and the following conditions were adopted.

Table 36 shows the measurement performed at the time point of 24 hours for each of a total of six patterns where rat serum or human serum was set on the donor side and phosphate buffer physiological saline was set on the acceptor side (tests A to C: the same conditions).

[Table 36]

| Red Device | 1 | 2 | 3 | 4 |
|---|---|---|---|---|
| Elapsed time | 24h | | 24h | |
| Chamber | Donor | Acceptor | Donor | Acceptor |
| A | Rat | PBS | Human | PBS |
| | 1 | 1 | 1 | 1 |
| B | Rat | PBS | Human | PBS |
| | 2 | 2 | 2 | 2 |
| C | Rat | PBS | Human | PBS |
| | 3 | 3 | 3 | 3 |

[Sampling after dialysis reaction]

The same procedure as in Examples 4 to 13 was carried out.

[Pretreatment of sample from sampling]

10 $\mu$L of DMSO and 100 $\mu$L of an IS solution were added to 20 $\mu$L of the sample after sampling, and the mixture was transferred to a filter plate ("Multi Screen Filter Plates 0.45um, Low Binding" manufactured by Millipore Corporation), and filtered. 70 $\mu$L of water was added to the sample after the filtration to obtain a LC-MS/MS measurement sample. The composition of the IS solution is as shown in Table 37 below.

[Table 37]

| IS solution | Acetonitrile/methanol (1:1) solution (mL) | Solution of verapamil at 1 mg/mL in DMSO ($\mu$L) | Solution of indomethacin at 2 mg/mL in DMSO ($\mu$L) |
|---|---|---|---|
| | 20 | 0.1 | 0.5 |

Preparation of calibration curve sample: 99 $\mu$L of DMSO was added to 1 $\mu$L of the mixed solution of Asunaprevir, Apalutamide, Elbasvir, Dutasteride and Deferasirox (0.2 mmol/L for each analyte) dissolved in DMSO to obtain a 2 $\mu$mol/L

compound mixed solution. 99 μL of DMSO was added to 1 μL of the mixed solution of Aripiprazole, Beclabuvir, Lomitapide, Ibrutinib and Montelukast (0.2 mmol/L for each analyte) dissolved in DMSO to obtain a 2 μmol/L compound mixed solution. The following series of dilutions (1) to (8) and BLANK solution were prepared.

[(1) 40 μL of DMSO was added to 60 μL of a solution of the analytes each at 2 μmol/L to obtain a compound solution at 1200 nmol/L.

(2) 120 μL of DMSO was added to 60 μL of a solution of the analytes each at 1200 nmol/L to obtain a compound solution at 400 nmol/L.

(3) 140 μL of DMSO was added to 60 μL of a solution of the analytes each at 400 nmol/L to obtain a compound solution at 120 nmol/L.

(4) 120 μL of DMSO was added to 60 μL of a solution of the analytes each at 120 nmol/L to obtain a compound solution at 40 nmol/L.

(5) 140 μL of DMSO was added to 60 μL of a solution of the analytes each at 40 nmol/L to obtain a compound solution at 12 nmol/L.

(6) 120 μL of DMSO was added to 60 μL of a solution of the analytes each at 12 nmol/L to obtain a compound solution at 4 nmol/L.

(7) 140 μL of DMSO was added to 60 μL of a solution of the analytes each at 4 nmol/L to obtain a compound solution at 1.2 nmol/L.

(8) 120 μL of DMSO was added to 60 μL of a solution of the analytes each at 1.2 nmol/L to obtain a compound solution at 0.4 nmol/L.]

Since the series of dilutions (1) to (8) were each adjusted to a concentration 2 times that of an actual sample, the concentrations of the calibration curve samples were determined with a correction made in consistency with 1/2 times the concentrations adjusted by the series of dilutions.

Concentrations of calibration curve samples: (8) 0.2 nM, (8) 0.6 nM, (7) 2 nM, (6) 6 nM, (5) 20 nM, (4) 60 nM, (3) 200 nM and (2) 600 nM

[Measurement of concentration of analyte by LC-MS/MS]

For each of the LC-MS/MS measurement samples, the concentrations of Apalutamide, Elbasvir, Dutasteride and Deferasirox and the concentrations of Aripiprazole, Beclabuvir, Lomitapide, Ibrutinib and Montelukast in each chamber were measured using the following measuring equipment and measurement conditions. When the concentration of the analyte was measured in the same serum in a plurality of wells, an average value of concentrations measured in the wells for the same serum group was used for calculation of the $f_u$ ratio.

(Measuring equipment)

LC: Waters Acquity UPLC
MS: XEVO-TQS#WAA696 (Waters Corporation, Milford, Massachusetts)
Column: YMC-Triart C18 column, 30 x 2.0 mm ID, S-1.9 um, 12 nm

(Measurement conditions)

Column temperature: 40°C (set value)
Solvent:

A) 0.1% aqueous formic acid solution
B) 0.1% acetonitrile formate solution

Gradient conditions:

[Table 38]

| Time (min) | A % | B % |
|---|---|---|
| 0.0 | 80 | 20 |
| 0.2 | 80 | 20 |
| 0.4 | 35 | 65 |
| 0.6 | 15 | 85 |
| 0.8 | 5 | 95 |
| 1.2 | 5 | 95 |
| 1.3 | 80 | 20 |
| 1.6 | 80 | 20 |

Flow rate: 0.6 mL/min
Autosampler temperature: 10°C (set value)
Injection volume: 1µL (Reference Examples 5 and 7 to 13), 1.5 µL (Reference Example 6)

[Measurement of concentration of analyte by LC-MS/MS]

For each of the LC-MS/MS measurement samples, the concentration of Asunaprevir in each chamber was measured using the following measuring equipment and measurement conditions.

(Measuring equipment)

LC: Waters Acquity UPLC
MS: XEVO-TQS#WAA696 (Waters Corporation, Milford, Massachusetts)
Column: YMC-Triart Bio C4, 30 x 2.1 mm ID, S-1.9 um, 30 nm

(Measurement conditions)

Column temperature: 40°C (set value)
Solvent:

A) 0.1% aqueous formic acid solution
B) 0.1% acetonitrile formate solution

Gradient conditions:

[Table 39]

| Time (min) | A % | B % |
|---|---|---|
| 0.0 | 60 | 40 |
| 0.2 | 60 | 40 |
| 0.4 | 15 | 85 |
| 0.6 | 5 | 95 |
| 0.8 | 1 | 99 |
| 1.2 | 1 | 99 |
| 1.4 | 1 | 99 |
| 1.41 | 60 | 40 |

(continued)

| Time (min) | A % | B % |
|------------|-----|-----|
| 2 | 60 | 40 |

Flow rate: 0.6 mL/min
Autosampler temperature: 10°C (set value)
Injection volume: 1μL

[Table 40]

| Compound | Q1 (m/z) | Q3 (m/z) | Dwell (sec) | Cone. Volt (V) | Col. Energy (eV) |
|----------|----------|----------|-------------|----------------|------------------|
| Verapamil (internal standard) | 455.0866 | 164 .965 | 0.043 | 20 | 25 |
| Indomethacin (internal standard) | 359.2383 | 139.1685 | 0.043 | 30 | 20 |
| Asunaprevir | 749.51 | 535.79 | 0.043 | 30 | 30 |
| Apalutamide | 477.92 | 161.91 | 0.043 | 45 | 40 |
| Elbasvir | 882.24 | 655.94 | 0.043 | 40 | 40 |
| Dutasteride | 529.08 | 460.86 | 0.043 | 25 | 35 |
| Deferasirox | 373.98 | 107.84 | 0.043 | 40 | 40 |
| Aripiprazole | 449.62 | 286.91 | 0.043 | 100 | 30 |
| Beclabuvir | 660.2 | 534.94 | 0.043 | 35 | 30 |
| Lomitapide | 694.15 | 248.89 | 0.043 | 25 | 40 |
| Ibrutinib | 441.06 | 137.91 | 0.043 | 45 | 25 |
| Montelukast | 586.08 | 421.86 | 0.043 | 10 | 25 |

MS conditions:

ESI positive
Capillary 2 kV, Cone 25 V, Source Offset 50 V, Source Temperature 150°C, Desolvation Temperature 600°C, Cone Gas Flow 150 L/Hr, Collision Gas Flow 0.2 mL/min, Nebuliser Gas Flow 7 Bar

The obtained $f_u$ ratios (rat/human) for the analytes are shown below.

[Table 41]

| | $f_u$ ratio (rat/human) |
|--|-------------------------|
| Asunaprevir | 1.52 |
| Apalutamide | 2.30 |
| Elbasvir | 1.46 |
| Dutasteride | 1.47 |
| Deferasirox | 3.53 |
| Aripiprazole | 1.45 |
| Beclabuvir | 1.21 |
| Lomitapide | 2.77 |
| Ibrutinib | 0.40 |
| Montelukast | - |

[Evaluation Example 1]

Method for predicting distribution volume of drug in human

Distribution volume in a human was predicted using the $f_u$ ratios determined in Examples 1 to 13, Comparative Examples 1 to 13 and Reference Examples 1 to 13 above.

The "distribution volume" (V) means a theoretical amount of liquid required for the total amount of an administered drug to be contained in the body at a concentration equal to that in the blood or plasma, and is expressed by distribution volume = amount of drug in the body/blood concentration (e.g. L).

In accordance with a method for predicting distribution volume in a human (Non Patent Literature 7: Berry, L. M., et al. (2011). "Species differences in distribution and prediction of human V(ss) from preclinical data." Drug Metab Dispos 39(11): 2103-2116), steady state distribution volume per body weight (rat) was divided by the $f_u$ ratio (rat/human) for the drug, which had been obtained by the above-described method, to determine the predicted value of distribution volume in a human per body weight. The obtained predicted value was compared with the observed value of the distribution volume in a human to validate the prediction accuracy.

Predicted expression of distribution volume

$$Vss_{human} \text{ (predicted value)} = Vss_{rat} / Rfu$$

$V_{SSrat}$: steady state distribution volume per volume in rat (Vss [L/kg])
Rfu: Relative $f_u$ ratio (rat/human)

The dilution $f_u$ values (rat, serum concentration: 1/10) and the $f_u$ values (human, serum concentration: 1/10), which had been obtained in Reference Examples 4 to 13 using the following dilution expression, were corrected to a serum concentration of 1/1, and used for distribution volume prediction for compounds exhibiting a $f_u$ value of 0.01 or less in the rat or human (i.e. compounds that hardly reach an equilibrium state).

$$\text{Dilution expression: fu (after correction)} = \frac{1/D}{\left(\dfrac{1}{\text{fu (before correction)}} - 1\right) + 1/D}$$

wherein D represents a dilution ratio. D is 10 because serum diluted by a factor of 10 is used in this case.

The above dilution expression was obtained by referring to the following documents.

Non Patent Literature 8: Riccardi K, Cawley S, Yates PD, et al. Plasma protein binding of challenging compounds. J Pharm Sci. 2015;104:2627-2636.
Non Patent Literature 9: Kalvass JC, Maurer TS. Influence of nonspecific brain and plasma binding on CNS exposure: implications for rational drug discovery. Biopharm Drug Dispos. 2002;23:327-338.

The results of $f_u$ values after correction are shown below.

[Table 42]

| Analyte | Rat ($f_u$ value) | Human ($f_u$ value) |
|---|---|---|
| Asunaprevir | 0.008 | 0.005 |
| Apalutamide | 0.104 | 0.030 |
| Elbasvir | 0.001 | 0.001 |
| Dutasteride | 0.009 | 0.006 |
| Deferasirox | 0.020 | 0.005 |
| Aripiprazole | 0.003 | 0.002 |
| Beclabuvir | 0.008 | 0.007 |

(continued)

| Analyte | Rat ($f_u$ value) | Human ($f_u$ value) |
|---|---|---|
| Lomitapide | 0.006 | 0.002 |
| Ibrutinib | 0.007 | 0.019 |
| Montelukast | - | - |

From the results of Table 42, Asunaprevir, Elbasvir, Dutasteride, Deferasirox, Aripiprazole, Beclabuvir, Lomitapide and Ibrutinib were determined to be compounds exhibiting a $f_u$ value of 0.01 or less in the rat or human, and among these, compounds for which the observed values of distribution volume in the rat and human had been acquired were applied to distribution volume prediction using relative $f_u$ ratios. In addition thereto, the relative $f_u$ ratios of nifedipine and ibuprofen used in Examples 1 and 3, Comparative Examples 1 and 3 and Reference Examples 1 and 3, which exhibit a $f_u$ value of 0.01 or less in the rat or human, were also used. The results of steady state distribution volume (Vdss, Vss) obtained by prediction are shown in Tables 43 and 44 and Figures 13-1 to 13-3.

[Table 43]

| Vdss prediction | Example (dynamic analysis) | Comparative Example (static analysis) | Reference Example (known method, versus buffer) |
|---|---|---|---|
| Within 0.8-1.25 times (%) | 67 | 22 | 11 |
| Within 0.67-1.5 times (%) | 67 | 33 | 56 |

[Table 44]

| Vdss prediction | Example (dynamic analysis) | Comparative Example (static analysis) | Reference Example (known method, versus buffer) |
|---|---|---|---|
| AAFE | 1.61 | 2.38 | 1.70 |

For the relative $f_u$ ratio by the method of the present invention (a $f_u$ ratio between different species which is acquired by dynamic analysis from a change in concentration until the time point of 24 hours of dialysis reaction after the start of the dialysis reaction where serum derived from an arbitrary species is added to each of a donor-side chamber and an acceptor-side chamber), 67% of the values fell within 1.25-fold error in distribution volume prediction, and 67% of the values fell within 1.5-fold error in distribution volume prediction. The absolute average fold error (AAFE) was 1.61-fold error in distribution volume prediction. (Figure 13-1).

For the fu ratio by the method of Comparative Example (the numerical value of a fu ratio between an animal and a human which is calculated using a fu value determined from a concentration ratio at the time point of 24 hours of dialysis reaction after the start of the dialysis reaction where serum derived from an arbitrary species is added to each of a donor-side chamber and an acceptor-side chamber), 22% of the values fell within 1.25-fold error in distribution volume prediction, and 33% of the values fell within 1.5-fold error in distribution volume prediction. AAFE was 2.38-fold error. (Figure 13-2).

For the fu ratio determined by the conventional known method shown in Reference Examples, 11% of the values fell within 1.25-fold error in distribution volume prediction, 56% of the values fell within 1.5-fold error in distribution volume prediction, and AAFE was 1.68-fold error. (Figure 13-3)

The accuracy of the distribution volume prediction using a relative $f_u$ ratio determined by the method of the present invention (dynamic analysis) was superior in prediction accuracy in each of 1.25-fold error, 1.5-fold error and AAFE to the accuracy of the distribution volume prediction shown in Comparative Examples. That is, it can be seen that by using the method of the present invention, a relative $f_u$ ratio can be acquired with good accuracy and used for distribution volume prediction even for an analyte that hardly reaches an equilibrium state. In addition, the method of Reference Examples (versus buffer) requires preliminary examination to search for a time point at which compound concentrations in serum and the buffer chamber are close to an equilibrium state for each compound. The method of the present invention (dynamic analysis) does not require such a process, and may be practical.

[Examples 14 to 20]

Acquisition of relative $f_u$ ratio between different biotic samples (serum and hepatic microsome) of the same species (human) by dynamic analysis (analytes: Aripiprazole, Deferasirox, Lapatinib, Tikaglera, Ketoconazole, Itraconazole and RG12525)

The method of the present invention can be used not only for comparison between serums of different species, but also for acquisition of a $f_u$ ratio between different biotic samples in the same species, for example, a fraction unbound-to-protein ratio between serum and hepatic microsome ($f_u$ serum vs $f_{u\,mic}$ ratio). Substances to be measured and analyzed were Aripiprazole, Deferasirox, Lapatinib, Tikaglera, Ketoconazole, Itraconazole and RG12525. These substances may competitively inhibit CYP3A4 as a liver drug-metabolizing enzyme, and have a high protein binding ratio in human serum (a serum $f_u$ value of 0.01 or less). (Non Patent Literature 10: (Vieira, M. L., et al. (2014). "Evaluation of various static in vitro-in vivo extrapolation models for risk assessment of the CYP3A inhibition potential of an investigational drug." Clin Pharmacol Ther 95(2): 189-198.). Non Patent Literature 11: Hachad, H., et al. (2010). "A useful tool for drug interaction evaluation: the University of Washington Metabolism and Transport Drug Interaction Database." Hum Genomics 5(1): 61-72. Non Patent Literature 12: Chu, Q. S., et al. (2008). "A phase I and pharmacokinetic study of lapatinib in combination with letrozole in patients with advanced cancer." Clin Cancer Res 14(14): 4484-4490. Non Patent Literature 13: Koch, K. M., et al. (2017). "The effects of lapatinib on CYP3A metabolism of midazolam in patients with advanced cancer." Cancer Chemother Pharmacol 80(6): 1141-1146.

DDI prediction for these compounds may be difficult as long as a human serum $f_u$ value of 0.01 is applied in accordance with the current FDA guideline, but improvement of accuracy of DDI prediction can be expected if a $f_u$ ratio ($f_u$ serum vs $f_{u\,mic}$ ratio) can be determined with high accuracy by the method of the present invention (dynamic analysis).

[Table 45]

| | Analyte | Manufacturer | MW | clog P | Weight (mg) | Amount of DMSO added (μL) |
|---|---|---|---|---|---|---|
| Example 14 | Aripiprazole | Chemscene Lcc (Cem) | 448.39 | 4.9 | 2.222 | 496 |
| Example 15 | Deferasirox | ASTATECH | 373.36 | 4.74 | 2.409 | 645 |
| Example 16 | Lapatinib | Sigma-Aldrich | 581.06 | 4.64 | 0.936 | 161 |
| Example 17 | Tikaglera | Cayman Chemical | 522.60 | 2.28 | 0.715 | 137 |
| Example 18 | Ketoconazole | Wako Purechemical | 531.43 | 4.19 | 2.695 | 507 |
| Example 19 | Itraconazole | Sigma-Aldrich | 705.63 | 7.31 | 1.679 | 476 |
| Example 20 | RG12525 | MedChem | 423.47 | - | 1.27 | 300 |

For each analyte, a solution at 1 mmol/L was prepared. Specifically, the solution was prepared by adding 8 μL of DMSO to 2 μL of a solution containing Itraconazole at 5 mmol/L, or adding 18 μL of DMSO to 2 μL of a solution containing Aripiprazole, Deferasirox, Lapatinib, Tikaglera, Ketoconazole or RG12525 at 10 mmol/L.

Further, for RG12525, 5 μL of DMSO was added to 5 μL of the solution at 1 mmol/L to prepare a solution at 0.5 mmol/L. For Itraconazole and Ketoconazole, 8 μL of DMSO was added to 2 μL of the solution at 1 mmol/L to prepare a solution at 0.2 mmol/L.

Next, fused human serum was collected, and to each serum was added 9 times its volume of phosphate buffer physiological saline. To the resulting solution was added 1/1000 times its volume of the solution containing the analyte (Aripiprazole, Deferasirox, Lapatinib, Tikaglera, Itraconazole, Ketoconazole or RG12525) at 1 mmol/L, respectively. In this way, a donor solution having a serum concentration of 10% (volume ratio) and a DMSO concentration (volume ratio) of 0.1% was prepared. Fused human hepatic microsome (20 mg/mL) was collected, and to each microsome was added 199 times its volume of phosphate buffer physiological saline. To the resulting solution was added 1/1000 times its volume of the solution containing the analyte (Aripiprazole, Deferasirox, Lapatinib or Tikaglera) at 1 mmol/L, 1/1000 times its volume of the solution containing RG12525 at 0.5 mmol/L, or 1/1000 times its volume of the solution containing the analyte (Itraconazole or Ketoconazole) at 0.2 mmol/L. In this way, a donor solution having a microsome concentration of 0.1 mg/mL and a DMSO concentration (volume ratio) of 0.1% was prepared.

The samples used were the same as in Example 1 except for nifedipine.

[Preparation of acceptor solution]

Except that a DMSO solution was added instead of the solution containing each analyte at 1 mmol/L, the same procedure as that for the donor solution was carried out to prepare an acceptor solution having a human serum concentration of 10% (volume ratio) and a DMSO concentration of 0.1% (volume ratio).

[Dialysis reaction]

The same method as in Example 1 was carried out except that the analyte was changed from nifedipine to the analytes of Examples 14 to 20, and all acceptor solutions were human serum solutions. Table 46 below shows the donor solutions or acceptor solutions injected into the chambers.

Table 46 shows the measurement was performed at the time points of 0.5 hours, 2 hours, 4 hours and 20 hours for each of a total of eight patterns where human serum was set on the donor side and human serum was set on the acceptor side (tests A to H: the same conditions) and where human hepatic microsome was set on the donor side and the human serum was set on the acceptor side (tests A to H: the same conditions).

[Table 46]

| Red device | 1 | 2 | 3 | 4 |
|---|---|---|---|---|
| Chamber | Donor | Acceptor | Donor | Acceptor |
| Elapsed time | 0.5 h | | | |
| A | Human serum | Human serum | Human hepatic microsome | Human serum |
| | 1 | 1 | 1 | 1 |
| B | Human serum | Human serum | Human hepatic microsome | Human serum |
| | 2 | 2 | 2 | 2 |
| Elapsed time | 2 h | | | |
| C | Human serum | Human serum | Human hepatic microsome | Human serum |
| | 1 | 1 | 1 | 1 |
| D | Human serum | Human serum | Human hepatic microsome | Human serum |
| | 2 | 2 | 2 | 2 |
| Elapsed time | 4 h | | | |
| E | Human serum | Human serum | Human hepatic microsome | Human serum |
| | 1 | 1 | 1 | 1 |
| F | Human serum | Human serum | Human hepatic microsome | Human serum |
| | 2 | 2 | 2 | 2 |
| Elapsed time | 20 h | | | |
| G | Human serum | Human serum | Human hepatic microsome | Human serum |
| | 1 | 1 | 1 | 1 |
| H | Human serum | Human serum | Human hepatic microsome | Human serum |
| | 2 | 2 | 2 | 2 |

[Sampling after dialysis reaction]

The same method as in Example 1 is was carried out except that the collected donor solution and acceptor solution were hermetically sealed without using a quench solution, and each sample was stored at -80°C before being subjected to pretreatment.

[Pretreatment of sample from sampling]

10 $\mu$L of DMSO and 100 $\mu$L of an IS solution were added to 20 $\mu$L of the sample after sampling, and the mixture was transferred to a filter plate ("Multi Screen Filter Plates 0.45um, Low Binding" manufactured by Millipore Corporation), and filtered. 70 $\mu$L of water was added to the sample after the filtration to obtain a LC-MS/MS measurement sample. The composition of the IS solution is as shown in Table 47 below.

[Table 47]

| IS solution | Acetonitrile (mL) | Solution of Verapamil at 1 mg/mL in DMSO ($\mu$L) | Solution of Indomethacin at 2 mg/mL in DMSO ($\mu$L) |
|---|---|---|---|
| | 30 | 1.5 | 0.75 |
| | 70 | 3.5 | 1.75 |

[Measurement of concentration of analyte by LC-MS/MS]

Preparation of calibration curve sample: 99 $\mu$L of DMSO was added to 1 $\mu$L of a solution of each analyte (1 mmol/L) dissolved in DMSO to obtain a compound solution at 10 $\mu$mol/L. The following series of dilutions (1) to (8) and BLANK solution were prepared.

[(1) 40 $\mu$L of DMSO was added to 60 $\mu$L of a solution of each analyte at 10 $\mu$mol/L to obtain a compound solution at 6000 nmol/L.

(2) 120 $\mu$L of DMSO was added to 60 $\mu$L of a solution of each analyte at 6000 nmol/L to obtain a compound solution at 2000 nmol/L.

(3) 140 $\mu$L of DMSO was added to 60 $\mu$L of a solution of each analyte at 2000 nmol/L to obtain a compound solution at 600 nmol/L.

(4) 120 $\mu$L of DMSO was added to 60 $\mu$L of a solution of each analyte at 600 nmol/L to obtain a compound solution at 200 nmol/L.

(5) 140 $\mu$L of DMSO was added to 60 $\mu$L of a solution of each analyte at 200 nmol/L to obtain a compound solution at 60 nmol/L.

(6) 120 $\mu$L of DMSO was added to 60 $\mu$L of a solution of each analyte at 60 nmol/L to obtain a compound solution at 20 nmol/L.

(7) 140 $\mu$L of DMSO was added to 60 $\mu$L of a solution of each analyte at 20 nmol/L to obtain a compound solution at 6 nmol/L.

(8) 120 $\mu$L of DMSO was added to 60 $\mu$L of a solution of each analyte at 6 nmol/L to obtain a compound solution at 2 nmol/L.

Since the series of dilutions (1) to (8) were each adjusted to a concentration 2 times that of an actual sample, the concentrations of the calibration curve samples were determined with a correction made in consistency with 1/2 times the concentrations adjusted by the series of dilutions.

Concentrations of calibration curve samples: (8) 1 nM, (7) 3 nM, (6) 10 nM, (5) 30 nM, (4) 100 nM, (3) 300 nM, (2) 1000 nM and (1) 3000 nM

10 $\mu$L of the calibration curve sample was added to 20 $\mu$L of human hepatic microsome (0.1 mg/mL) or the human serum blank sample, 100 $\mu$L of the IS solution was added, and the mixture was transferred to a filter plate ("Multi Screen Filter Plates 0.45um, Low Binding" manufactured by Millipore Corporation), and filtered. 70 $\mu$L of water was added to the sample after the filtration to obtain a LC-MS/MS measurement sample.

[Measurement of concentration of analyte by LC-MS/MS]

For each of the LC-MS/MS measurement samples, the concentrations of Aripiprazole, Deferasirox, Lapatinib, Tikaglera, Ketoconazole, Itraconazole or RG12525 were measured using the following measuring equipment and measurement conditions. When the concentration of the analyte was measured in a biotic sample of the same type (serum or human hepatic microsome) in a plurality of wells, an average value of concentrations measured in the wells for a group of biotic samples of the same type was used for calculation of the $f_u$ ratio.

(Measuring equipment)

LC: Waters Acquity UPLC
MS: XEVO-TQS#WAA696 (Waters Corporation, Milford, Massachusetts)

Column: YMC-Triart C18 column, 30 x 2.0 mm ID, S-1.9 um, 12 nm

(Measurement conditions)

Column temperature: 50°C (set value)
Solvent:

A) 0.1% aqueous formic acid solution
B) 0.1% acetonitrile formate solution

Gradient conditions:

[Table 48]

| Time (min) | A% | B % |
|---|---|---|
| 0.0 | 99 | 1 |
| 0.8 | 1 | 99 |
| 1.6 | 1 | 99 |
| 1.61 | 99 | 1 |

Flow rate: 0.6 mL/min
Autosampler temperature: 10°C (set value)
Injection volume: $1\mu L$
MS conditions: (ESI positive)

[Table 49]

| Compound | Q1 (m/z) | Q3 (m/z) | Dwell (sec) | Cone. Volt (V) | Col. Energy (eV) |
|---|---|---|---|---|---|
| Indomethacin (internal standard) | 359.24 | 139.17 | 0.1 | 30 | 20 |
| Verapamil (internal standard) | 455.09 | 164.97 | 0.1 | 20 | 25 |
| Aripiprazole (analyte) | 449.62 | 286.91 | 0.1 | 100 | 30 |
| Deferasirox (analyte) | 373.98 | 107.84 | 0.1 | 40 | 40 |
| Lapatinib (analyte) | 580.74 | 364.74 | 0.1 | 10 | 35 |
| Tikaglera (analyte) | 522.89 | 152.81 | 0.1 | 15 | 35 |
| Ketoconazole (analyte) | 530.84 | 81.66 | 0.1 | 35 | 35 |
| Itraconazole (analyte) | 705.02 | 391.81 | 0.1 | 10 | 35 |
| RG12525 (analyte) | 423.92 | 142.87 | 0.1 | 20 | 35 |

The $f_u$ ratio was acquired by the same method as in Example 1 except that each parameter was changed as described below.

fu1: fu value of analyte with respect to biological sample contained in donor solution (calculated value)
fu2: fu value of analyte with respect to biological sample contained in acceptor solution (calculated value), where the value of fu2 was equal to that of fu1 in the case of human serum in donor solution and human serum in acceptor solution.
PS: Dialysis membrane permeation clearance of analyte (fixed value, $1.5 \times 10^{-4}$ L/h) (Model, reaction, rules, definition, estimated parameters) were described and performed.

Reaction:

Eq1.Donor1<-> Eq1.Acceptor1: +fu1*PS*Eq1.Donor1/Vd1-fu1*PS*Eq1.Acceptor1/Vd2
Eq2.Donor2<-> Eq2.Acceptor2: +fu2*PS*Eq2.Donor2/Vd1-fu1*PS*Eq2.Acceptor2/Vd2 Eq2.Donor2-> Eq2.Ad:
+CL2*fu2*Eq2.Donor2/Vd1-(Koff2*Eq2.Ad)

Rules:

The rules were the same as in Example 1.

Definition:

When the first biological sample was human serum, and the second biological sample was human hepatic microsome, a set of the following four pieces of data was used for analysis.

Data 1 (Eq1.Cd1): Concentration of analyte in donor solution in equilibrium dialysis system with donor solution containing human serum and acceptor solution containing human serum
Data 2 (Eq1.Ca1): Concentration of analyte in acceptor solution in equilibrium dialysis system with donor solution containing human serum and acceptor solution containing human serum
Data 3 (Eq2.Cd2): Concentration of analyte in donor solution in equilibrium dialysis system with donor solution containing human hepatic microsome and acceptor solution containing human serum
Data 4 (Eq2.Ca2): Concentration of analyte in acceptor solution in equilibrium dialysis system with donor solution containing human hepatic microsome and acceptor solution containing human serum

Estimated Parameters:

exp(fu1).InitialValue = 0.01; Bounds = [1e-05 11;
exp(fu2). Initial Value = 0.01; Bounds = [1e-05 1];
exp(Koff2).InitialValue = 1; Bounds = [1e-05 10000];
exp(CL2), InitialValue = 0.001; Bounds = [1e-07 100];
exp(Dose1).InitialValue = 0.2115; Bounds = [0.01 0.51;
exp(Dose2).InitialValue = 0.2114; Bounds = [0.01 0.5];

The $f_u$ (human, serum concentration: 1/10) calculated by the above-described dynamic analysis method was corrected to a serum concentration of 1/1 using the following dilution expression, and the $f_u$ ratio (serum/microsome) for each analyte was determined.

$$\text{Dilution expression: fu (after correction)} = \frac{1/D}{\left(\dfrac{1}{\text{fu (before correction)}} - 1\right) + 1/D}$$

wherein D represents a dilution ratio. D is 10 because serum diluted by a factor of 10 is used in this case.

The obtained results are shown below.

[Table 50]

|  | $f_u$ ratio (serum/microsome) |
|---|---|
| Aripiprazole | 0.0076 |
| Deferasirox | 0.0030 |
| Lapatinib | 0.0060 |
| Tikaglera | 0.0103 |
| Ketoconazole | 0.0116 |
| Itraconazole | 0.0074 |

(continued)

|  | $f_u$ ratio (serum/microsome) |
|---|---|
| RG12525 | 0.0002 |

[Comparative Examples 14 to 20]

Acquisition of relative fu ratio between different biotic samples of the same species (human) (human serum and microsome) by static analysis (analyte: the same as in Examples 14 to 20)

When the $f_u$ ratio is determined by the same method as in Examples 4 to 13, the dialysis reaction requires preparation of a donor solution having a microsome concentration of 0.1 mg/mL and a DMSO concentration of 0.1% (volume ratio) and an acceptor solution having a human serum concentration of 100% and a DMSO concentration of 0.1% (volume ratio), but the compounds of Examples 14 to 20 are compounds having a high protein binding, which are likely to exhibit a $f_u$ value of 0.01 or less, and may hardly reach an equilibrium state when the human serum concentration used is 100% (i.e. serum is not diluted with a buffer). Thus, for these compounds, calculation of the $f_u$ ratio by the same method as in Comparative Examples 4 to 13 was not performed.

[Reference Examples 14 to 20]

Acquisition of $f_u$ value in different biotic samples of the same species (human) (human serum and microsome) by static analysis (versus buffer) (analyte: the same as in Examples 14 to 20)

Fused human serum was collected, and to each serum was added 9 times its volume of phosphate buffer physiological saline. To the resulting solution was added 1/1000 times its volume of the compound solution at 1 mmol/L. In this way, a donor solution having a serum concentration of 10% (volume ratio) and a DMSO concentration (volume ratio) of 0.1% was prepared. Fused human hepatic microsome (20 mg/mL) was collected, and to each serum was added 199 times its volume of phosphate buffer physiological saline. To the resulting solution was added 1/1000 times its volume of the compound solution at 1 mmol/L. In this way, a donor solution having a microsome concentration of 0.1 mg/mL and a DMSO concentration (volume ratio) of 0.1% was prepared.

[Table 51]

| Red device | 1 | 2 | 3 | 4 |
|---|---|---|---|---|
| Elapsed time | 24 h | | 24 h | |
| Chamber | Donor | Acceptor | Donor | Acceptor |
| A | Human serum | PBS | Human hepatic microsome | PBS |
| | 1 | 1 | 1 | 1 |
| B | Human serum | PBS | Human hepatic microsome | PBS |
| | 2 | 2 | 2 | 2 |

A donor solution and an acceptor solution were prepared in the same manner as in Examples 14 to 20, dialysis reaction was started, and the concentrations of an analyte in solutions added to the chambers at the time point of 24 hours after the start of the dialysis reaction were measured. The obtained data was applied to expression 5 in Reference Example 1 to acquire fu values (microsome). As in Examples 14 to 20, the dilution fu value (human, serum concentration: 1/10) was corrected to a serum concentration of 1/1 using the dilution expression, and the fu value was determined (human, serum concentration: 1/1).

[Table 52]

| Analyte | Human serum (serum concentration: 1/1) (fu value) | Human hepatic microsome (fu value) |
|---|---|---|
| Aripiprazole | 0.0021 | 0.62 |
| Deferasirox | 0.0047 | 1.01 |
| Lapatinib | 0.0004 | 0.05 |

(continued)

| Analyte | Human serum (serum concentration: 1/1) (fu value) | Human hepatic microsome (fu value) |
|---|---|---|
| Tikaglera | 0.0032 | 0.48 |
| Ketoconazole | 0.0080 | 1.10 |
| Itraconazole | 0.0032 | 0.77 |
| RG12525 | 0.0004 | 0.77 |

The $f_u$ ratio (serum/microsome) of each analyte was determined from the obtained fu value.

[Table 53]

| | $f_u$ ratio (serum/microsome) |
|---|---|
| Aripiprazole | 0.0033 |
| Deferasirox | 0.0046 |
| Lapatinib | 0.0087 |
| Tikaglera | 0.0065 |
| Ketoconazole | 0.0073 |
| Itraconazole | 0.0041 |
| RG12525 | 0.0005 |

[Reference Examples 21 to 27]

Calculation of $f_u$ ratio based on results of Reference Examples 14 to 20 and FDA guidance (analyte: the same as in Examples 14 to 20)

The fu ratio (serum/microsome) using the fu value (microsome) acquired in each of Reference Examples 14 to 20 and the fu value (serum) = 0.01 for each analyte based on the FDA guidance are shown below.

[Table 54]

| | fu ratio (serum/microsome) |
|---|---|
| Aripiprazole | 0.0160 |
| Deferasirox | 0.0099 |
| Lapatinib | 0.1972 |
| Tikaglera | 0.0207 |
| Ketoconazole | 0.0091 |
| Itraconazole | 0.0129 |
| RG12525 | 0.0129 |

[Evaluation Example 2]

For predicting the DDI risk, midazolam that is a CYP3A4 substrate was used as a probe compound to determine an inhibition constant Ki value of each compound in human hepatic microsome.

To 24 $\mu$L of fused commercially available human hepatic microsome (20 mg/mL, manufactured by XenoTech, LLC) was added 3976 $\mu$L of a phosphate buffer at 0.1 mol/L to prepare 4000 $\mu$L in total of a human hepatic microsome solution (concentration: 0.12 mg/mL). Alternatively, to 37 $\mu$L of commercially available human hepatic microsome (20 mg/mL, manufactured by XenoTech, LLC) was added 5963 $\mu$L of a phosphate buffer at 0.1 mol/L to prepare 6000 $\mu$L in total of a human hepatic microsome solution (concentration: 0.12 mg/mL). 3 $\mu$L of an undiluted solution of an analyte compound in DMSO at a concentration 1000 times the final addition concentration was provided, and 87 $\mu$L of a 60%

acetonitrile solution was added to prepare 90 μL in total of an analyte compound solution. To 5 μL of a solution of midazolam dissolved therein at 50 mmol/L was added 70 μL of a 50% acetonitrile solution to obtain 75 μL in total of a solution. To 12 μL of the solution were added 63 μL of an acetonitrile solution and 5925 μL of water to obtain 6000 μL in total of a midazolam solution (concentration: 6.6 μmol/L). To 25 mg of commercially available β-NADPH (Wako Pure Chemical Industries, Ltd.) was added 3000 μL of a phosphate buffer at 0.1 mol/L to obtain a NADPH solution (10 mmol/L). The prepared solutions were mixed in the following order to obtain an inhibition reaction solution (100 μL in total), and the solution was reacted for 5 minutes with stirring at 37°C where the time immediately after addition of the NADPH solution was defined as 0 minute after the start of the reaction.

[Table 55]

| Metabolic reaction solution | Human hepatic microsome solution(concentration; 0.12 mg/mL) (μL) | Undiluted solution of analyte compound (μL) | Midazolam solution (concentration: 6.6 μmol/L) (μL) | NADPH solution (10 mmol/L) (μL) |
|---|---|---|---|---|
| | 82 | 3 | 5 | 10 |

[Table 56]

| | Addition concentration of inhibition reaction solution and analyte compound (μmol/L) |
|---|---|
| Aripiprazole | 0 (control), 0.94, 1.88, 3.75, 7.5, 15, 30, 60 |
| Deferasirox | 0 (control), 1.56, 3.13, 6.25, 12.5, 25, 50, 100 |
| Lapatinib | 0 (control), 1.25, 2.5, 5, 10 20, 40, 80 |
| Tikaglera | 0 (control), 1.56, 3.13, 6.25, 12.5, 25, 50, 100 |
| Ketoconazole | 0 (control), 0.0012, 0.004, 0.011, 0.03, 0.1, 0.3, 0.9 |
| Itraconazole | 0 (control), 0.0014, 0.004, 0.012, 0.04, 0.11, 0.33, 1 |
| RG12525 | 0 (control), 0.0823, 0.247, 0.741, 2.22, 6.67, 20, 60 |

After the reaction in the metabolic reaction solution, 35 μL of the reaction product was recovered, and mixed with 140 μL of a solution of acetonitrile/isopropanol = 1/1 (containing 1-hydroxymidazolam [13C3] at 2.5 nmol/L and 10 nM warfarin as IS), and the mixture was subjected to centrifugal treatment with a protein removal treatment filter. The thus-obtained sample was used as a LCMS measurement solution. Two sets of the LCMS measurement solution were prepared, with one intended for quantitative determination of 1-hydroxymidazolam generated by metabolic reaction and the other intended for measurement of the actual concentration of a compound to be measured (inhibitor). Each set was used for LCMS quantitative analysis.

For each measurement compound (inhibitor), the amount of generation of 1'OH-midazolam at 0 (control) was defined as 100%, and the 50% inhibition concentration ($IC_{50}$) was determined using the following expression.

$$IC_{50} = 10^{(Log(b/a) \times (50-A)/(B-A) + Log(a))}$$

A: Latest percentage (%) of control which is larger than 50% (low concentration side)
B: Latest percentage (%) of control which is smaller than 50% (high concentration side)
a: Compound concentration at A (low concentration)
b: Compound concentration at B (high concentration)

The addition concentration of the midazolam used as a CYP3A4 substrate in this case is 0.33 μmol/L, and this value is sufficiently lower than the Km value of 2.06 μmol/L which is suggested in the literature. Therefore, the determined $IC_{50}$ was directly used as Ki value. (Non Patent Literature 14: Takano, J., et al. (2016). "The Prediction of the Relative Importance of CYP3A/P-glycoprotein to the Nonlinear Intestinal Absorption of Drugs by Advanced Compartmental Absorption and Transit Model." Drug Metab Dispos 44(11): 1808-1818.). The Ki value of each compound is shown below.

EP 4 220 152 B1

[Table 57]

|  | Ki value μmol/L |
|---|---|
| Aripiprazole | 17.8 |
| Deferasirox | 49 (maximum addition concentration) or higher |
| Lapatinib | 8.94 |
| Tikaglera | 87 (maximum addition concentration) or higher |
| Ketoconazole | 0.034 |
| Itraconazole | 0.023 |
| RG12525 | 1.49 |

For Deferasirox and Tikaglera, a concentration equal to or higher than the maximum addition concentration was considered to be a Ki value because a concentration representing a 50% inhibition concentration was not obtained. As a Ki value to be used for prediction, the maximum addition concentration was used for the sake of convenience.

Using the $f_u$ ratio and $f_u$ value determined in each of Examples 14 to 20, Reference Examples 14 to 20 and Reference Examples 21 to 27 above and the Ki value used in the inhibitor experiments, the predicted value of an area under the blood concentration-time curve (AUC) ratio (AUCR) of midazolam that is a CYP3A4 substrate was determined, and compared with the observed value of AUCR to predict the DDI risk.

AUCR (observed value) of midazolam used in combination with the inhibitor is cited from Patent Literatures 10 to 13, and shown in the table below. When a plurality of AUCR values is described at the same dose, the geometric average of the AUCR values is calculated, and taken as an observed value. For Aripiprazole and Tikaglera, AUCR is not described as a numerical value, or is shown to have no effect on oral administration of midazolam, and therefore AUCR (observed value) is defined as 1.

[Table 58]

|  | Dose (mg) | Cmax ng/ml | AUCR (observed value) |
|---|---|---|---|
| Aripiprazole | 15 | 242 | 1.00 |
| Deferasirox | 210 | 40327 | 0.90 |
| Lapatinib | 1500 | 2470 | 1.45 |
| Tikaglera | 90 | 403 | 1.00 |
| Ketoconazole 1 | 200 | 4681.36 | 7.13 |
| Ketoconazole 2 | 400 | 5877.28 | 10.96 |
| Itraconazole 1 | 50 | 14.11 | 1.91 |
| Itraconazole 2 | 200 | 163.93 | 5.05 |
| Itraconazole 3 | 400 | 317.52 | 5.07 |
| RG12525_1 | 100 | 1482 | 1.09 |
| RG12525_2 | 600 | 8186 | 1.01 |

For DDI prediction, a model was used in which the following Net Effect is applied.

$$\text{AUCR (predicted value)} = \left( \frac{1}{(Ag \times Bg \times Cg) \times (1 - Fg) + Fg} \right) \times \left( \frac{1}{(Ah \times Bh \times Ch) \times fm + (1 - fm)} \right)$$

(Non Patent Literature 15: FDA DDI guidance (2020))

The meanings of the symbols in the above expression are as follows.

$$A\,g = \frac{1}{1 - {}^{[1]}g \big/ Ki,_{u}}$$

$$[I]g, \ [\mu mol/L] \ \text{(inhibitor concentration in small intestine cells)} = \frac{ka \times fa \times Dose}{Qent}$$

$K_{i, u}[\mu mol/L] = Ki \ \text{value} \times fu, mic$
$k_a$ is 6[h$^{-1}$], and $f_a$ is 1.

Dose is a dosage amount [$\mu$mol] of each inhibitor.

Cmax, total [$\mu$mol/L]
$Q_{ent}$ is 18[L/h/70 kg] (Non Patent Literature 16: Yang, Jamei et al., 2007).

The input value of Fg is 1 in this case for validating the prediction accuracy for the fu ratio obtained by dynamic analysis.

(Non Patent Literature 16: Yang, J., et al. (2007). "Prediction of intestinal first-pass drug metabolism." Curr Drug Metab 8(7): 676-684.)

$$A h = \frac{1}{1 - \frac{[I]h}{Ki, \ u}}$$

[I] h[$\mu$mol/L] (inhibitor concentration in hepatic cells)

$$(Cmax, total + (\frac{ka \times fa \times Dose}{Qh})/Rb)$$

$$K_{i, u}[\mu mol/L] = Ki \ \text{value} \times fu,$$

mic
$k_a$[h$^{-1}$] is 6, and $f_a$ and $R_b$ is 1.
Dose is a dosage amount [$\mu$mol] of each inhibitor.
Cmax, total [$\mu$mol/L]
Fm is 0.93.
$Q_h$ is 97[L/h/70 kg] (Non Patent Literature 17: Yang, Jamei et al., 2007).

(Non Patent Literature 17: Yang, J., et al. (2007). "Misuse of the well-stirred model of hepatic drug clearance." Drug Metab Dispos 35(3): 501-502.)
(Non Patent Literature 18: Obach, R. S., et al. (2006). "The utility of in vitro cytochrome P450 inhibition data in the prediction of drug-drug interactions." J Pharmacol Exp Ther 316(1): 336-348.)

$$\frac{[I]h}{Ki, \ u}$$

can be expressed as $\{f_{userum}/(f_{umic} \times K_i)\} \times \{ Cmax, total + (k_a \times f_a \times Dose/Q_h) \div R_b\}$, and the fu ratio (serum/microsome] is used for the part of $f_{userum}/f_{umic}$.

The input values of Bg and Bh representing irreversible inhibition and Cg and Ch representing induction in the intestinal tract and the liver, respectively, are set to 1 because the inhibitor has considered to have no effect in this case.
The results obtained from the prediction are shown in Figures 14-1 to 14-3 and the table below.

[Table 59]

| AUCR prediction | Examples 14 to 20 | Reference Examples 14 to 20 | Reference Examples 21 to 27 |
|---|---|---|---|
| AAFE | 1.12 | 1.17 | 1.37 |

**EP 4 220 152 B1**

The DDI prediction using a relative $f_u$ ratio determined by the method of the present invention (dynamic analysis) was superior in accuracy to the prediction accuracy shown in the conventional method. That is, it can be seen that by using the method of the present invention, a relative $f_u$ ratio can be acquired with good accuracy and used for DDI prediction even for an analyte that hardly reaches an equilibrium state.

[Industrial Applicability]

In prediction of the drug kinetics in a human based on nonclinical studies (analysis using mice, rats, dogs, monkeys or the like), plasma fraction unbound ($f_u$) in each animal species is considered to be an important parameter.

The present invention provides a novel assay technique which enables direct detection of a relative fraction unbound-to-protein ratio ($f_u$ ratio) of a drug between biotic samples of different types. A $f_u$ ratio with high accuracy can be acquired by the assay system in which rapid equilibrium dialysis is combined with dynamic analysis, and the accuracy of estimation of the drug kinetics in a human is expected to be improved by using drug kinetics parameters for each animal species on the basis of the ratio of a free drug concentration in plasma or the like. The technique may be usable for wide range of compounds including not only compounds having a high protein binding ratio but also compounds having moderate to very low protein binding ratios, and can be expected as an absolutely imperative technique in future studies for development of low-molecular-weight and middle-molecular-weight drugs in general.

For example, the FCIM method which is considered as having the highest accuracy among human clearance prediction methods (Non Patent Literature 6) is a method in which a human clearance value is predicted by correction of a clearance value and a $f_u$ value for each animal species. In this method, the $f_u$ value for a compound having a high protein binding ratio is difficult to acquire, and therefore is not sufficiently validated. It can be expected that human clearance prediction accuracy can be further improved when a relative $f_u$ ratio obtained according to the present invention is applied to the FCIM method.

The present invention can be widely used for not only comparison between serums of different species but also $f_u$ ratios between biotic samples of different types. For example, it may be possible to determine a ratio of fraction unbound-to-protein for hepatic microsome vs fraction unbound-to-protein for serum (ratio of $f_{u\,mic}$ vs $f_u$ serum). This may enable the precision of clinical drug-drug interaction (DDI) prediction to be enhanced.

The guidance on drug-drug interaction from FDA in U.S. requires that in drugs having an inhibitory action on drug metabolizing enzymes, the degree of drug-drug interaction be predicted with the following expression using a maximum unbound form drug concentration in plasma (Imax, u) and an inhibition constant (Ki, u) on the unbound form.

$$R1 = 1 + (Imax, u/Ki, u)$$

This expression can be converted as follows using a maximum total concentration in plasma ($I_{max}$), an inhibition constant based on the total concentration ($K_i$), plasma fraction unbound ($f_u$) and fraction unbound in microsome ($f_{u,\,mic}$).

$$R1 = 1 + (Imax/Ki)*(fu/fumic)$$

In this guidance, it is required that due to inaccuracy of measurement of the protein binding ratio, calculation be performed on the assumption that $f_u$ equals 0.01 in the case where $f_u$ is less than 0.01, and there may be a risk that the degree of interaction is accordingly overestimated, and a normally unnecessary clinical test on interaction between drugs must be conducted.

If the precision of clinical DDI prediction can be enhanced by precisely determining the $f_u/f_{u,\,mic}$ ratio directly by the method according to the present invention, unnecessary clinical DDI tests are skipped to deliver a necessary medicinal agent to a patient more quickly, and there is high usefulness in reduction of cost for clinical development.

[Reference Signs List]

I    Dialysis chamber system I
II    Dialysis chamber system II
M    Semipermeable membrane
A    First biological sample (A)
B    Second biological sample (B)
1    Donor-side chamber
2    Acceptor-side chamber

3      Analyte
4      Non-specific protein derived from first biological sample (A)
5      Non-specific protein derived from second biological sample (B)
10     Donor-side chamber
20     Acceptor-side chamber

**Claims**

1. A method for determining a relative fraction unbound ratio (relative $f_u$ ratio) of an analyte between different biological samples, the method comprising the following steps of:

   (1) providing a chamber system (I) in which adjacent chambers are separated by a semipermeable membrane permeable to the analyte;
   (2) adding a donor solution containing a first biological sample (A) and the analyte to one chamber (donor-side chamber) in the chamber system (I);
   (3) adding an acceptor solution containing a second biological sample (B) to a chamber different from the donor-side chamber (acceptor-side chamber) in the chamber system (I);
   (4) measuring concentrations of the analyte in the donor solution in the step (2) and the acceptor solution in the step (3) over time; and
   (5) calculating the relative $f_u$ ratio using data associated with the concentrations of the analyte which are measured in the step (4),

   wherein the step (5) is dynamic analysis.

2. The method according to claim 1, further comprising the following steps of:

   (6) providing a chamber system (II) different from the chamber system (I), in which adjacent chambers are separated by a semipermeable membrane permeable to the analyte;
   (7) adding an acceptor solution containing the biological sample (A) to one chamber (acceptor-side chamber) in the chamber system (II);
   (8) adding a donor solution containing the biological sample (B) and the analyte to a chamber different from the acceptor-side chamber (donor-side chamber) in the chamber system (II);
   (9) measuring concentrations of the analyte in the acceptor solution in the step (7) and the donor solution in the step (8) over time; and
   (10) calculating the relative $f_u$ ratio using data associated with the concentrations of the analyte which are measured in the step (9),

   wherein the step (10) is dynamic analysis..

3. The method according to claim 1 or 2, wherein the biological samples (A) and/or (B) are diluted with a buffer solution.

4. The method according to any one of claims 1 to 3, wherein a molecular weight cutoff of the semipermeable membrane is 50 kDa or less.

5. The method according to any one of claims 1 to 4, wherein a protein binding ratio of the analyte in the biological sample (A) is 95% or more.

6. The method according to any one of claims 1 to 5, wherein a protein binding ratio of the analyte in the biological sample (B) is 95% or more.

7. The method according to any one of claims 1 to 6, wherein the biological sample (A) is one selected from the group consisting of a microsomal fraction, blood, plasma and serum.

8. The method according to any one of claims 1 to 7, wherein the biological sample (B) is one selected from the group consisting of a microsomal fraction, blood, plasma and serum.

9. The method according to any one of claims 1 to 8, wherein the biological sample is serum.

10. The method according to any one of claims 1 to 9, wherein the biological sample (A) is derived from a mammal.

11. The method according to any one of claims 1 to 10, wherein the biological sample (B) is derived from a mammal.

12. The method according to any one of claims 1 to 11, wherein CLogP of the analyte is 25 or less.

13. The method according to any one of claims 1 to 12, wherein a molecular weight of the analyte is 5000 or less.

14. A method comprising using the $f_u$ ratio determined by the method according to any one of claims 1 to 13 and the data of clearance of a drug in a species from which one of the biological sample (A) or the biological sample (B) is derived, to predict clearance of a drug in a species from which the other biological sample is derived.

15. A method comprising using the relative fu ratio determined by the method according to any one of claims 1 to 13 and the data of distribution volume of a drug in a species from which one of the biological sample (A) or the biological sample (B) is derived, to predict distribution volume of a drug in a species from which the other biological sample is derived.

**Patentansprüche**

1. Verfahren zum Bestimmen eines relativen Verhältnisses einer ungebundenen Fraktion (relatives $f_u$-Verhältnis) eines Analyten zwischen verschiedenen biologischen Proben, wobei das Verfahren die folgenden Schritte umfasst:

   (1) Bereitstellen eines Kammersystems (I), in dem benachbarte Kammern durch eine für den Analyten durchlässige semipermeable Membran getrennt sind;
   (2) Zugeben einer Donorlösung, die eine erste biologische Probe (A) und den Analyten enthält, zu einer Kammer (donorseitige Kammer) im Kammersystem (I);
   (3) Zugeben einer Akzeptorlösung, die eine zweite biologische Probe (B) enthält, zu einer Kammer, die sich von der donorseitigen Kammer unterscheidet (akzeptorseitige Kammer) im Kammersystem (I);
   (4) Messen von Konzentrationen des Analyten in der Donorlösung in Schritt (2) und der Akzeptorlösung in Schritt (3) im Zeitverlauf; und
   (5) Berechnen des relativen $f_u$-Verhältnisses unter Verwendung von Daten, die mit den Konzentrationen des Analyten, die in Schritt (4) gemessen werden, assoziiert sind,

   wobei Schritt (5) eine dynamische Analyse ist.

2. Verfahren nach Anspruch 1, das des Weiteren die folgenden Schritte umfasst:

   (6) Bereitstellen eines Kammersystems (II), das sich vom Kammersystem (I) unterscheidet, in dem benachbarte Kammern durch eine für den Analyten durchlässige semipermeable Membran getrennt sind;
   (7) Zugeben einer Akzeptorlösung, die die biologische Probe (A) enthält, zu einer Kammer (akzeptorseitige Kammer) im Kammersystem (II);
   (8) Zugeben einer Donorlösung, die die biologische Probe (B) und den Analyten enthält, zu einer Kammer, die sich von der akzeptorseitigen Kammer unterscheidet (donorseitige Kammer) im Kammersystem (II);
   (9) Messen von Konzentrationen des Analyten in der Akzeptorlösung in Schritt (7) und der Donorlösung in Schritt (8) im Zeitverlauf; und
   (10) Berechnen des relativen $f_u$-Verhältnisses unter Verwendung von Daten, die mit den Konzentrationen des Analyten, die in Schritt (9) gemessen werden, assoziiert sind,

   wobei Schritt (10) eine dynamische Analyse ist.

3. Verfahren nach Anspruch 1 oder 2, wobei die biologischen Proben (A) und/oder (B) mit einer Pufferlösung verdünnt sind.

4. Verfahren nach einem der Ansprüche 1 bis 3, wobei ein Molekulargewicht-Cut-Off-Wert der semipermeablen Membran 50 kDa oder weniger ist.

5. Verfahren nach einem der Ansprüche 1 bis 4, wobei ein Proteinbindungsverhältnis des Analyten in der biologischen Probe (A) 95% oder mehr ist.

6. Verfahren nach einem der Ansprüche 1 bis 5, wobei ein Proteinbindungsverhältnis des Analyten in der biologischen Probe (B) 95% oder mehr ist.

7. Verfahren nach einem der Ansprüche 1 bis 6, wobei die biologische Probe (A) eine Probe ist, die ausgewählt ist aus der Gruppe bestehend aus einer mikrosomalen Fraktion, Blut, Plasma und Serum.

8. Verfahren nach einem der Ansprüche 1 bis 7, wobei die biologische Probe (B) eine Probe ist, die ausgewählt ist aus der Gruppe bestehend aus einer mikrosomalen Fraktion, Blut, Plasma und Serum.

9. Verfahren nach einem der Ansprüche 1 bis 8, wobei die biologische Probe Serum ist.

10. Verfahren nach einem der Ansprüche 1 bis 9, wobei die biologische Probe (A) von einem Säuger stammt.

11. Verfahren nach einem der Ansprüche 1 bis 10, wobei die biologische Probe (B) von einem Säuger stammt.

12. Verfahren nach einem der Ansprüche 1 bis 11, wobei der CLogP des Analyten 25 oder weniger ist.

13. Verfahren nach einem der Ansprüche 1 bis 12, wobei ein Molekulargewicht des Analyten 5000 oder weniger ist.

14. Verfahren, das die Verwendung des $f_u$-Verhältnisses, das durch das Verfahren nach einem der Ansprüche 1 bis 13 bestimmt wurde, und der Daten der Clearance eines Arzneistoffes in einer Spezies umfasst, von der eine der biologischen Probe (A) oder der biologischen Probe (B) stammt, um eine Clearance eines Arzneistoffes in einer Spezies, von der die andere biologische Probe stammt, vorauszusagen.

15. Verfahren, das die Verwendung des relativen $f_u$-Verhältnisses, das durch das Verfahren nach einem der Ansprüche 1 bis 13 bestimmt wurde, und der Daten des Verteilungsvolumens eines Arzneistoffs in einer Spezies umfasst, von der eine der biologischen Probe (A) oder der biologischen Probe (B) stammt, um ein Verteilungsvolumen eines Arzneistoffs in einer Spezies, von der die andere biologische Probe stammt, vorauszusagen.

**Revendications**

1. Méthode pour déterminer le rapport de fraction non liée relatif (rapport $f_u$ relatif) d'un analyte entre différents échantillons biologiques, la méthode comprenant les étapes suivantes de :

   (1) fourniture d'un système de chambres (I) dans lequel des chambres adjacentes sont séparées par une membrane semi-perméable qui est perméable à l'analyte ;
   (2) addition d'une solution de donneur contenant un premier échantillon biologique (A) et l'analyte dans une chambre (chambre du côté donneur) dans le système de chambres (1) ;
   (3) addition d'une solution d'accepteur contenant un deuxième échantillon biologique (B) dans une chambre différente de la chambre du côté donneur (chambre du côté accepteur) dans le système de chambres (I) ;
   (4) mesure des concentrations de l'analyte dans la solution de donneur dans l'étape (2) et la solution d'accepteur dans l'étape (3) au cours du temps ; et
   (5) calcul du rapport $f_u$ relatif par utilisation de données associées aux concentrations de l'analyte qui sont mesurées dans l'étape (4),

   dans laquelle l'étape (5) est une analyse dynamique.

2. Méthode selon la revendication 1, comprenant en outre les étapes suivantes de :

   (6) fourniture d'un système de chambres (II) différent du système de chambres (I) et dans lequel des chambres adjacentes sont séparées par une membrane semi-perméable qui est perméable à l'analyte ;
   (7) addition d'une solution d'accepteur contenant l'échantillon biologique (A) dans une chambre (chambre du côté accepteur) dans le système de chambres (II) ;
   (8) addition d'une solution de donneur contenant l'échantillon biologique (B) et l'analyte dans une chambre différente de la chambre du côté accepteur (chambre du côté donneur) dans le système de chambres (II) ;

(9) mesure des concentrations de l'analyte dans la solution d'accepteur dans l'étape (7) et la solution de donneur dans l'étape (8) au cours du temps ; et
(10) calcul du rapport f$_u$ relatif par utilisation de données associées aux concentrations de l'analyte qui sont mesurées dans l'étape (9),

dans laquelle l'étape (10) est une analyse dynamique.

3. Méthode selon la revendication 1 ou 2, dans laquelle les échantillons biologiques (A) et/ou (B) sont dilués avec une solution de tampon.

4. Méthode selon l'une quelconque des revendications 1 à 3, dans laquelle la séparation des masses moléculaires de la membrane semi-perméable est à 50 kDa ou moins.

5. Méthode selon l'une quelconque des revendications 1 à 4, dans laquelle le rapport de liaison aux protéines de l'analyte dans l'échantillon biologique (A) est de 95 % ou plus.

6. Méthode selon l'une quelconque des revendications 1 à 5, dans laquelle le rapport de liaison aux protéines de l'analyte dans l'échantillon biologique (B) est de 95 % ou plus.

7. Méthode selon l'une quelconque des revendications 1 à 6, dans laquelle l'échantillon biologique (A) est l'un choisi dans le groupe constitué par une fraction microsomale, du sang, du plasma, et du sérum.

8. Méthode selon l'une quelconque des revendications 1 à 7, dans laquelle l'échantillon biologique (B) est l'un choisi dans le groupe constitué par une fraction microsomale, du sang, du plasma, et du sérum.

9. Méthode selon l'une quelconque des revendications 1 à 8, dans laquelle l'échantillon biologique est du sérum.

10. Méthode selon l'une quelconque des revendications 1 à 9, dans laquelle l'échantillon biologique (A) dérive d'un mammifère.

11. Méthode selon l'une quelconque des revendications 1 à 10, dans laquelle l'échantillon biologique (B) dérive d'un mammifère.

12. Méthode selon l'une quelconque des revendications 1 à 11, dans laquelle CLogP de l'analyte est de 25 ou moins.

13. Méthode selon l'une quelconque des revendications 1 à 12, dans laquelle une masse moléculaire de l'analyte est de 5 000 ou moins.

14. Méthode comprenant l'utilisation du rapport f$_u$ déterminé par la méthode de l'une quelconque des revendications 1 à 13 et des données de clairance d'un médicament dans une espèce dont dérive l'un parmi l'échantillon biologique (A) et l'échantillon biologique (B), pour prédire la clairance d'un médicament dans une espèce dont dérive l'autre échantillon biologique.

15. Méthode comprenant l'utilisation du rapport f$_u$ relatif déterminé par la méthode de l'une quelconque des revendications 1 à 13 et des données de volume de distribution d'un médicament dans une espèce dont dérive l'un parmi l'échantillon biologique (A) et l'échantillon biologique (B), pour prédire le volume de distribution d'un médicament dans une espèce dont dérive l'autre échantillon biologique.

## Fig.1

(Figure 1a)

(Figure 1b)

(Figure 1c)

(Figure 1d)

(Figure 1e)

(Figure 1f)

(Figure 1g)

(Figure 1h)

# *Fig.2*

Fig.3

Fig.4

*Fig.5*

(1) (2) (3) (4) — Concentration vs Time (hr)

*Fig.6*

EP 4 220 152 B1

## Fig.7

## Fig.8

# Fig.9

## Fig.10

## Fig.11

EP 4 220 152 B1

Fig.12

Ibuprofen

83

## Fig.13-1

## Fig.13-2

Fig.13-3

## Fig.14-1

# Fig.14-2

**Static analysis (versus buffer)**

# Fig.14-3

Conventional method (fu serum = 0.01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ERIKSSON, M. A et al.** Studies of drug binding to plasma proteins using a variant of equilibrium dialysis. *J Pharm Biomed Anal*, 2005, vol. 38 (3), 381-389
- **KALVASS, J. C. et al.** Mathematical and Experimental Validation of Flux Dialysis Method: An Improved Approach to Measure Unbound Fraction for Compounds with High Protein. *Drug Metabolism and Disposition*, April 2018, vol. 46, 458-469
- **CHEN, Y. C. et al.** Improving Confidence in the Determination of Free Fraction for Highly Bound Drugs Using Bidirectional Equilibrium Dialysis. *J Pharm Sci*, 2019, vol. 108 (3), 1296-1302
- **DESHMUKH, S. V.** ; **A. HARSCH**. Direct determination of the ratio of unbound fraction in plasma to unbound fraction in microsomal system (fu p/fu mic) for refined prediction of phase I mediated metabolic hepatic clearance. *J Pharmacol Toxicol Methods*, 2011, vol. 63 (1), 35-39
- **JONES, R. S. et al.** Evaluation of a competitive equilibrium dialysis approach for assessing the impact of protein binding on clearance predictions. *J Pharm Sci.*, 15 September 2020
- **TANG, H.** ; **M. MAYERSOHN**. A novel model for prediction of human drug clearance by allometric scaling. *Drug Metab Dispos*, 2005, vol. 33 (9), 1297-1303
- **BERRY, L. M. et al.** Species differences in distribution and prediction of human V(ss) from preclinical data.. *Drug Metab Dispos*, 2011, vol. 39 (11), 2103-2116
- **RICCARDI K** ; **CAWLEY S** ; **YATES PD et al.** Plasma protein binding of challenging compounds. *J Pharm Sci.*, 2015, vol. 104, 2627-2636
- **KALVASS JC** ; **MAURER TS**. Influence of nonspecific brain and plasma binding on CNS exposure: implications for rational drug discovery. *Biopharm Drug Dispos.*, 2002, vol. 23, 327-338
- **VIEIRA, M. L. et al.** Evaluation of various static in vitro-in vivo extrapolation models for risk assessment of the CYP3A inhibition potential of an investigational drug.. *Clin Pharmacol Ther*, 2014, vol. 95 (2), 189-198
- **HACHAD, H. et al.** A useful tool for drug interaction evaluation: the University of Washington Metabolism and Transport Drug Interaction Database. *Hum Genomics*, vol. 5 (1), 61-72

- **CHU, Q. S. et al.** A phase I and pharmacokinetic study of lapatinib in combination with letrozole in patients with advanced cancer.. *Clin Cancer Res*, 2008, vol. 14 (14), 4484-4490
- **KOCH, K. M. et al.** The effects of lapatinib on CYP3A metabolism of midazolam in patients with advanced cancer.. *Cancer Chemother Pharmacol*, 2017, vol. 80 (6), 1141-1146
- **TAKANO, J. et al.** The Prediction of the Relative Importance of CYP3A/P-glycoprotein to the Nonlinear Intestinal Absorption of Drugs by Advanced Compartmental Absorption and Transit Model.. *Drug Metab Dispos*, 2016, vol. 44 (11), 1808-1818
- **YANG, J. et al.** Prediction of intestinal first-pass drug metabolism.. *Curr Drug Metab*, 2007, vol. 8 (7), 676-684
- **YANG, J. et al.** Misuse of the well-stirred model of hepatic drug clearance.. *Drug Metab Dispos*, 2007, vol. 35 (3), 501-502
- **OBACH, R. S. et al.** The utility of in vitro cytochrome P450 inhibition data in the prediction of drug-drug interactions. *J Pharmacol Exp Ther*, 2006, vol. 316 (1), 336-348
- **BERRY, L. M et al.** Species differences in distribution and prediction of human V(ss) from preclinical data.. *Drug Metab Dispos*, 2011, vol. 39 (11), 2103-2116
- **BERRY, L. M. et al.** Species differences in distribution and prediction of human V(ss) from preclinical data. *Drug Metab Dispos*, 2011, vol. 39 (11), 2103-2116
- **VIEIRA, M. L et al.** Evaluation of various static in vitro-in vivo extrapolation models for risk assessment of the CYP3A inhibition potential of an investigational drug. *Clin Pharmacol Ther*, 2014, vol. 95 (2), 189-198
- **HACHAD, H. et al.** A useful tool for drug interaction evaluation: the University of Washington Metabolism and Transport Drug Interaction Database. *Hum Genomics*, 2010, vol. 5 (1), 61-72
- **CHU, Q. S et al.** A phase I and pharmacokinetic study of lapatinib in combination with letrozole in patients with advanced cancer. *Clin Cancer Res*, 2008, vol. 14 (14), 4484-4490
- **YANG, J et al.** Prediction of intestinal first-pass drug metabolism. *Curr Drug Metab*, 2007, vol. 8 (7), 676-684

- **YANG, J et al.** Misuse of the well-stirred model of hepatic drug clearance. *Drug Metab Dispos*, 2007, vol. 35 (3), 501-502

- **OBACH, R. S. et al.** The utility of in vitro cytochrome P450 inhibition data in the prediction of drug-drug interactions.. *J Pharmacol Exp Ther*, 2006, vol. 316 (1), 336-348